# EUROPEAN PATENT APPLICATION

(11) **EP 4 327 785 A2**
(43) Date of publication of application: **28.02.2024**
(21) Application number: 23199592.9
(22) Date of filing: 28.10.2020
(51) Int. Cl.: A61F 2/844

(54) **PROSTHETIC HEART VALVE HAVING LOCKING FEATURE**

(30) Priority: 30.10.2019 US 201962928291 P; 18.12.2019 US 201962950005 P; 26.02.2020 US 202062981666 P; 22.04.2020 US 202063013912 P; 18.05.2020 US 202063026267 P
(62) Divisional of application: 20811817.4
(71) Applicant: Edwards Lifesciences Corporation, Irvine, CA 92614 (US)
(72) Inventor: Neumann, Yair A., IRVINE, CA, 92614 (US); Cohen, Oren, IRVINE, CA, 92614 (US); Perlmutter, Khen, IRVINE, CA, 92614 (US); Atias, Eitan, IRVINE, CA, 92614 (US); Miller, Noam, IRVINE, CA, 92614 (US); Goldberg, Eran, IRVINE, CA, 92614 (US); Dvorsky, Anatoly, IRVINE, CA, 92614 (US); Levi, Tamir S., IRVINE, CA, 92614 (US); Yushtein, Haim, IRVINE, CA, 92614 (US); Garmahi, Danny M., IRVINE, CA, 92614 (US); Yohanan, Ziv, IRVINE, CA, 92614 (US); Bukin, Michael, IRVINE, CA, 92614 (US); Nir, Noam, IRVINE, CA, 92614 (US); Sherman, Elena, IRVINE, CA, 92614 (US); Sagi, Gideon, IRVINE, CA, 92614 (US)
(74) Representative: Eisenführ Speiser

(57) **Abstract**

An implantable prosthetic valve can include a frame movable between a radially compressed and a radially expanded configuration. The frame can include a first strut having a first locking feature disposed on a radially facing inner surface of the strut and a second strut having a second locking feature disposed on a radially facing outer surface of the strut. The first and second locking features can engage each other so as to allow pivoting of the first and second struts relative to one another in a first direction upon radial expansion of the frame and resist pivoting of the first and second struts relative to one another in a second direction to resist radial compression of the frame.

## Description

### CROSS REFERENCE TO RELATED APPLICATIONS

This application claims the benefit of U.S. Provisional Application 63/026,267 filed on May 18, 2020, U.S. Provisional Application 63/013,912 filed on April 22, 2020, U.S. Provisional Application 62/981,666 filed on February 26, 2020, U.S. Provisional Application 62/950,005 filed on December 18, 2019, and U.S. Provisional Application 62/928,291 filed on October 30, 2019, all of which are incorporated by reference herein in their entirety.

### FIELD

The present disclosure relates to implantable, mechanically expandable prosthetic devices, such as prosthetic heart valves, and to methods and delivery assemblies for, and including, such devices.

### BACKGROUND

The human heart can suffer from various valvular diseases. These valvular diseases can result in significant malfunctioning of the heart and ultimately require repair of the native valve or replacement of the native valve with an artificial valve. There are a number of known repair devices (e.g., stents) and artificial valves, as well as a number of known methods of implanting these devices and valves in humans. Percutaneous and minimally-invasive surgical approaches are used in various procedures to deliver prosthetic medical devices to locations inside the body that are not readily accessible by surgery or where access without surgery is desirable. In one specific example, a prosthetic heart valve can be mounted in a crimped state on the distal end of a delivery apparatus and advanced through the patient's vasculature (e.g., through a femoral artery and the aorta) until the prosthetic heart valve reaches the implantation site in the heart. The prosthetic heart valve is then expanded to its functional size, for example, by inflating a balloon on which the prosthetic valve is mounted, actuating a mechanical actuator that applies an expansion force to the prosthetic heart valve, or by deploying the prosthetic heart valve from a sheath of the delivery apparatus so that the prosthetic heart valve can self-expand to its functional size.

Prosthetic heart valves that rely on a mechanical actuator for expansion can be referred to as "mechanically expandable" prosthetic heart valves. Mechanically expandable prosthetic heart valves can provide one or more advantages over self-expandable and balloon-expandable prosthetic heart valves. For example, mechanically expandable prosthetic heart valves can be expanded to various diameters. Mechanically expandable prosthetic heart valves can also be compressed after an initial expansion (e.g., for repositioning and/or retrieval).

Despite the recent advancements in percutaneous valve technology, there remains a need for improved transcatheter heart valves and delivery devices for such valves.

### SUMMARY

Embodiments of improved prosthetic implant delivery assemblies and frames therefor are disclosed herein, as well as related methods and devices for such assemblies. In several embodiments, the disclosed assemblies are configured for delivering replacement heart valves into a heart of a patient.

In a representative embodiment, an implantable prosthetic device can comprise a frame movable between a radially compressed and a radially expanded configuration. The frame can comprise a first strut having a first locking feature disposed on a radially facing inner surface of the strut and a second strut having a second locking feature disposed on a radially facing outer surface of the strut. The first and second locking features can engage each other so as to allow pivoting of the first and second struts relative to one another in a first direction upon radial expansion of the frame and resist pivoting of the first and second struts relative to one another in a second direction to resist radial compression of the frame.

In some embodiments, the first locking feature is disposed at a first end portion of the first strut, and the second locking feature is disposed at a first end portion of the second strut.

In some embodiments, the first locking feature comprises a first toothed portion and the second locking feature comprises a second toothed portion. The first and second struts can be pivotably coupled to one another at a junction, and the first and second toothed portions can be arrayed circumferentially around at least a portion of the junction. The first toothed portion can comprise a first set of surfaces extending perpendicularly to the radially facing inner surface of the first strut and a second set of surface extending at an angle less than 90° relative to the radially facing inner surface of the strut, and the second toothed portion can comprise a third set of surfaces extending perpendicularly to the radially facing outer surface of the second strut and a fourth set of surfaces extending at an angle less than 90° relative to the radially facing outer surface of the strut.

In some embodiments, the first locking feature is formed integrally with the first strut and the second locking feature is formed integrally with the second strut.

In some embodiments, the first and second struts are pivotably coupled to one another at a junction by a fastener extending through the junction, and the fastener comprises a biasing member configured to bias the first and second struts against one another. The fastener can have a head portion and a shaft, the shaft extending through the first and second struts, and the biasing member can be disposed around the shaft at a location between the head portion and the first and second struts.

In some embodiments, the first and second locking features are movable from a disengaged position to an engaged position. When in the disengaged position the first and second struts can move relative to one another in the first and second directions. When in the engaged position the first and second locking features are rotationally aligned with one another and when in the disengaged position the first and second locking features are rotationally offset from one another.

In another representative embodiment, an implantable prosthetic device can comprise a frame movable between a radially compressed and a radially expanded configuration. The frame can comprise a first set of struts and a second set of struts. Each strut of the second set of struts can be pivotally connected at one or more junctions to at least one strut of the first set of struts. At least one strut of the first set of struts and at least one strut of the second set of struts can comprise first and second locking features, respectively, at a junction where the at least one strut of the first set of struts and the at least one strut of the second set of struts are pivotally connected to each other. The first and second locking features can engage each other so as to allow pivoting of the struts of the first set relative to the struts of the second set in a first direction upon radial expansion of the frame and resist pivoting of the struts relative to one another in a second direction to resist radial compression of the frame.

In some embodiments, the first locking feature is a first toothed portion and the second locking feature is a second, correspondingly toothed portion.

In some embodiments, the first locking feature is disposed on a radially facing inner surface of the at least one strut of the first set of struts and the second locking feature is disposed on a radially facing outer surface of the at least one strut of the second set of struts.

In some embodiments, the first locking feature is formed integrally with the at least one strut of the first set of struts and the second locking feature is formed integrally with the at least one strut of the second set of struts.

In some embodiments, each strut comprises a plurality of segments coupled to one another by a plurality of intermediate segments, and the first locking feature is disposed on an intermediate segment of the at least one strut of the first set of struts and the second locking feature is disposed on an intermediate segment of the at least one strut of the second set of struts.

In some embodiments, each junction comprises a fastener extending through respective struts of the first and second sets of struts. Such embodiments can further comprise a biasing member configured to bias the radially facing inner surface of a respective strut of the first set of struts against the radially facing outer surface of a respective strut of the second set of struts.

In a representative embodiment, a method comprises inserting a distal end of a delivery apparatus into the vasculature of a patient. The delivery apparatus can be releasably coupled to a prosthetic valve movable between a radially compressed and a radially expanded configuration. The prosthetic valve can comprise a frame having a first set of struts pivotably coupled to a second set of struts, each strut having a radially facing inner surface, and a radially facing outer surface. The method further comprises advancing the prosthetic valve to a selected implantation site and radially expanding the prosthetic valve such that a first locking feature disposed on a radially facing inner surface of at least one of the first set of struts engages a second locking feature disposed on a radially facing outer surface of at least one of the second set of struts to lock the frame in the radially expanded configuration.

In some embodiments, radially expanding the prosthetic valve comprises pivoting the first set of struts away from the second set of struts such that the first and second locking features rotate toward one another and engage one another.

In some embodiments, the first locking feature comprises a first plurality of teeth and the second locking feature comprises a second plurality of teeth and engagement of the first plurality of teeth with the second plurality of teeth prevents radially compression of the prosthetic valve.

In a representative embodiment, an implantable prosthetic device comprises a frame movable between a radially compressed and a radially expanded configuration, the frame comprising an inflow end portion and an outflow end portion and at least one expansion and locking mechanism. The expansion and locking mechanism can comprise a first member coupled to the frame at a first location, the first member comprising a pawl, and a second member coupled to the frame at a second location spaced apart from the first location, the second member extending at least partially into the first member and comprising a rack having a plurality of teeth arrayed along a length of the second member. Engagement of the pawl with the rack allows movement in a first direction to allow radial expansion of the frame and prevents movement in a second direction to prevent radial compression of the frame. The first member can comprise a sleeve and the teeth of the second member are housed in the sleeve.

In some embodiments, the first and second members have a rectangular or square cross-sectional profile in a plane perpendicular to a length of the expansion and locking mechanism.

In some embodiments, the pawl is biased toward the plurality of teeth.

In some embodiments, the prosthetic device further comprises a retaining member disposed between the pawl and the second member, the retaining member configured to selectively retain the pawl from engaging the rack.

In some embodiments, the entirety of the rack can be enclosed within the sleeve.

In a representative embodiment, an assembly can comprise a prosthetic heart valve and a delivery apparatus. The prosthetic heart valve can comprise a frame movable between a radially compressed and a radially expanded configuration, the frame comprising an inflow end portion and an outflow end portion, and at least one expansion and locking mechanism comprising a first member coupled to the frame at a first location, the first member comprising a pawl, and a second member coupled to the frame at a second location spaced apart from the first location, the second member extending at least partially into the first member and comprising a rack comprising a plurality of teeth, wherein the first member is shaped to enclose the rack. The delivery apparatus can comprise a handle, a first actuation member extending from the handle and coupled to the first member, the first actuation member configured to apply a distally directed force to the first member, and a second actuation member extending from the handle and coupled to the second member, the second actuation member configured to apply a proximally directed force to the second member. The prosthetic heart valve can be is radially expandable from the radially compressed configuration to the radially expanded configuration upon application of the distally directed force and the proximally directed force to the prosthetic heart valve via the first and second actuation members, respectively. Expansion of the prosthetic valve can cause movement of the first and second members relative to one another such that the pawl engages the teeth of the rack allowing movement of the first and second members in a first direction to allow radial expansion of the frame and preventing movement in a second direction to prevent radial compression of the frame.

In another representative embodiment, an implantable prosthetic device can comprise a frame being radially expandable and compressible between a radially compressed state and a radially expanded state, the frame comprising a first set of first struts, a second set of second struts, and a third set of third struts, the frame having a distal end and a proximal end. The first struts can be pivotably connected to each other at a plurality of distal and proximal apices at the distal and proximal ends of the frame, respectively. The second struts can be pivotably connected to each other at a plurality of distal and proximal apices at the distal and proximal ends of the frame, respectively. The third struts can be pivotably connected to each other at a plurality of distal and proximal apices at the distal and proximal ends of the frame, respectively. The first struts can be pivotably connected to the second and third struts at junctions between the distal and proximal ends of the frame. The second struts can be pivotably connected to the first and third struts at junctions between the distal and proximal ends of the frame. At least one expansion mechanism can be coupled to the frame at a pair of a distal apex and a proximal apex formed by the first struts. At least one locking mechanism can be coupled to the frame at a pair of axially spaced junctions, each of which is formed by struts of different sets. A plurality of commissure posts can be coupled to the frame at respective junctions. A leaflet assembly comprising a plurality of leaflets can be arranged to form a plurality of commissures coupled to respective commissure posts.

In another representative embodiment, an implantable prosthetic device can comprise a frame movable between a radially compressed and a radially expanded configuration, the frame comprising an inflow end portion and an outflow end portion, and at least one expansion and locking mechanism. Each expansion and locking mechanism can comprise a first member coupled to the frame at a first location, a second member coupled to the frame at a second location spaced apart from the first location, the second member extending at least partially into the first member and comprising a rack having a plurality of teeth arrayed along a length of the second member, and a locking member coupled to the first member via one or more protrusions extending from the second member into the first member, the locking member being biased toward the plurality of teeth. Engagement of the locking member with the rack can allow movement in a first direction to allow radial expansion of the frame and can prevent movement in a second direction to prevent radial compression of the frame.

In some embodiments, the locking member comprises one or more apertures extending at least partially through a thickness of the locking member, and wherein the one or more protrusions extend into the apertures.

In some embodiments, the one or more protrusions have a substantially cylindrical shape.

In some embodiments, the one or more protrusions have a hemispherical shape.

In some embodiments, the locking member comprises a locking tooth extending toward the rack and configured to engage the plurality of teeth.

In some embodiments, the locking member comprises a disengagement tooth extending axially from a proximal edge of the locking member. In some such embodiments, the prosthetic device further comprises a disengagement member configured to selectively engage the disengagement tooth to retain the locking member from engaging the rack.

In some embodiments, the locking member comprises a cutout defining a neck portion configured to bias the locking tooth radially against the rack. In some such embodiments, the neck portion is a first neck portion and wherein the locking member further comprises a second cutout defining a second neck portion configured to bias the locking tooth axially against the rack.

In some embodiments, the first member comprises a first engagement surface and the second member comprises a protrusion having a second engagement surface, and wherein the second engagement surface is configured to selectively abut the first engagement surface to prevent proximal movement of the second member past a predetermined point.

In some embodiments, the prosthetic device further comprises a stopper disposed on a distal end portion of the second member, the stopper sized to selectively abut a distal end portion of the first member to prevent proximal movement of the second member past a predetermined point. In some such embodiments, the distal end portion of the second member comprises a threaded portion and the stopper comprises a correspondingly threaded portion, and wherein rotation of the stopper in a first direction advances the stopper distally relative to the second member and rotation of the stopper in a second direction advances the stopper proximally relative to the second member.

In some embodiments, the first member has a rectangular cross-sectional profile in a plane perpendicular to a length of the expansion and locking mechanism.

In some embodiments, the first member comprises an inner bore having a first portion and a second portion, each having a first width, the first and second portions being separated by a neck portion having a second width smaller than the first width. In some such embodiments, the second member is sized to extend at least partially into the first portion of the inner bore.

In some embodiments, the first member comprises an opening in which the locking member is disposed. In some such embodiments, the first member comprises a ledge portion extending at least partially into the opening on which a portion of the locking member is disposed.

In another representative embodiment, a method of making an expansion and locking mechanism can comprise providing an outer member having an inner wall and an outer wall each comprising one or more first apertures having a first diameter, a first side wall, and a second side wall, the first side wall including an opening. The method can further comprise disposing a locking member within the opening, the locking member comprising one or more second apertures extending at least partially through a thickness of the locking member, the locking member can be disposed in the opening such that each second aperture aligns with a respective first aperture, and each second aperture can have a second diameter greater than the first diameter such that a respective lip portion is defined between each pair of first and second apertures. The method can further comprise using a punch member to apply force to a respective first aperture such that the lip portion deforms into the respective second aperture thereby securing the locking member to the outer member.

In some embodiments, the punch member is a cylindrical member having a third diameter greater than the first diameter and smaller than the second diameter.

In yet another representative embodiment, an implantable prosthetic device can comprise a frame movable between a radially compressed and a radially expanded configuration, the frame comprising an inflow end portion and an outflow end portion, and at least one expansion and locking mechanism. Each expansion and locking mechanism can comprise a first member coupled to the frame at a first location, the first member comprising a locking member, a second member coupled to the frame at a second location spaced apart from the first location, the second member extending at least partially into the first member and comprising a rack having a plurality of teeth arrayed along a length of the second member, and a stopper. The stopper can be disposed on an end portion of the second member and configured to prevent movement of the second member in a first direction past a predetermined point. Engagement of the locking member with the rack can allow movement in a first direction to allow radial expansion of the frame and prevent movement in a second direction to prevent radial compression of the frame.

In some embodiments, the stopper can comprise an annular nut sized to selectively abut a distal edge of the first member to retain the frame at a predetermined diameter.

In some embodiments, the end portion of the second member comprises a threaded portion and the stopper comprises a correspondingly threaded portion, and rotation of the stopper in a first direction advances the stopper in the first direction relative to the second member and rotation of the stopper in a second direction advances the stopper in the second direction relative to the second member.

In another representative embodiment, an implantable prosthetic device can comprise a frame movable between a radially compressed and a radially expanded configuration, the frame comprising an inflow end portion and an outflow end portion and at least one expansion and locking mechanism. Each expansion and locking mechanism can comprise a first member coupled to the frame at a first location, the first member comprising an aperture extending through a thickness of the first member, a second member coupled to the frame at a second location spaced apart from the first location, the second member extending at least partially into the first member, and a locking member coupled to the first member and being biased toward the second member. Engagement of the locking member with the second member allows movement in a first direction to allow radial expansion of the frame and prevents movement in a second direction to prevent radial compression of the frame. The aperture can be positioned such that a proximal edge of the second member can be viewed through the aperture when the prosthetic heart valve is in an assembled configuration.

In some embodiments, the first member has a rectangular cross-sectional profile in a plane perpendicular to a length of the expansion and locking mechanism, the first member comprising a first wall and a second wall, the first wall disposed radially outwardly of the second wall. In some such embodiments, the aperture extends through a thickness of the first wall.

In some embodiments, the first member comprises a commissure attachment portion and wherein the aperture is disposed distally relative to the commissure attachment portion.

In some embodiments, the aperture is positioned such that an apex of the locking tooth can be viewed through the aperture.

In some embodiments, the implantable device can further comprise a valvular structure including a plurality of leaflets, wherein each pair of adjacent leaflets is coupled to a respective expansion and locking mechanism at a respective commissure attachment portion to form a commissure. In some such embodiments, the aperture is positioned such that when the prosthetic device is in the radially expanded position, the commissure does not cover the aperture.

In some embodiments, the second member comprises a rack having a plurality of teeth arrayed along a length of the second member, and wherein the locking member comprises a locking tooth extending toward the rack and configured to engage the plurality of teeth.

In some embodiments, the locking member comprises a disengagement tooth extending axially from a proximal edge of the locking member. In some such embodiments, the implantable device can further comprise a disengagement member configured to selectively engage the disengagement tooth to retain the locking member from engaging the rack.

In some embodiments, the first member comprises a first engagement surface and the second member comprises a protrusion having a second engagement surface, and wherein the second engagement surface is configured to selectively abut the first engagement surface to prevent proximal movement of the second member past a predetermined point.

In some embodiments, the implantable device can further comprise a fastener extending radially outward from the first wall of the first member. In some such embodiments, the aperture is positioned distally adjacent to the fastener.

In some embodiments, the implantable device further comprises a fastener extending radially outward from a distal end portion of the second member.

In some embodiments, the implantable device further comprises a stopper disposed on a distal end portion of the second member, the stopper sized to selectively abut a distal end portion of the first member to prevent proximal movement of the second member past a predetermined point. In some such embodiments, the distal end portion of the second member comprises a threaded portion and the stopper comprises a correspondingly threaded portion, and wherein rotation of the stopper in a first direction advances the stopper distally relative to the second member and rotation of the stopper in a second direction advances the stopper proximally relative to the second member.

In some embodiments, the first member comprises an inner bore having a first portion and a second portion, each having a first width, the first and second portions being separated by a neck portion having a second width smaller than the first width. In some such embodiments, the second member is sized to extend at least partially into the first portion of the inner bore.

In some embodiments, the first member comprises an opening in which the locking member is disposed.

In some embodiments, the aperture is positioned such that a connection between the second member and an actuator of a delivery apparatus is visible.

In some embodiments, the frame comprises three expansion and locking mechanisms disposed circumferentially around the frame. In some such embodiments, the three expansion and locking mechanisms are spaced apart evenly from one another.

In another representative embodiment, an implantable prosthetic device can comprise a frame movable between a radially compressed and a radially expanded configuration, the frame comprising an inflow end portion and an outflow end portion, and at least one expansion and locking mechanism. Each expansion and locking mechanism can comprise a first member coupled to the frame at a first location, the first member having a first wall and a second wall and comprising an aperture extending through a thickness of the first wall, a second member coupled to the frame at a second location spaced apart from the first location, the second member extending at least partially into the first member, and a locking member coupled to the first member and being biased toward the second member. The implantable prosthetic device can further comprise a valvular structure comprising a plurality of leaflets, wherein each pair of adjacent leaflets is coupled to a respective expansion and locking mechanism at a respective commissure attachment portion to form a commissure. A respective aperture can be disposed distally adjacent each commissure.

In some embodiments, the valvular structure comprises three leaflets and three commissures.

In a representative embodiment, an assembly can comprise an implantable prosthetic device having a frame movable between a radially compressed and a radially expanded configuration, the frame comprising an inflow end portion and an outflow end portion, and at least one expansion and locking mechanism. The expansion and locking mechanism can comprise a first member coupled to the frame at a first location, the first member comprising an aperture extending through a thickness of the first member, a second member coupled to the frame at a second location spaced apart from the first location, the second member extending at least partially into the first member, and a locking member coupled to the first member and being biased toward the second member. The assembly can further comprise a delivery apparatus comprising a handle, a first actuation member extending from the handle and coupled to the first member, the first actuation member configured to apply a distally directed force to the first member, and a second actuation member extending from the handle and coupled to the second member, the second actuation member configured to apply a proximally directed force to the second member. The connection between the second member and the second actuation member can be visible through the aperture.

In some embodiments, the second actuation member extends at least partially into the first member.

In some embodiments, a distal end portion of the second actuation member comprises an engagement member. In some such embodiments, a proximal end portion of the second member comprises a bore into which the engagement member can extend. In some such embodiments, the engagement member comprises an external threaded surface configured to couple a correspondingly threaded surface of the bore. In other such embodiments, the engagement member comprises a magnet, and the inner bore comprises a correspondingly magnetic material.

In some embodiments, a distal end portion of the first actuation member is configured to abut a proximal end portion of the first member.

In some embodiments, the first member has a rectangular cross-sectional profile in a plane perpendicular to a length of the expansion and locking mechanism, the first member comprising a first wall and a second wall, the first wall disposed radially outwardly of the second wall.

In some embodiments, the aperture extends through a thickness of the first wall.

In some embodiments, the first member comprises a commissure attachment portion and wherein the aperture is disposed distally relative to the commissure attachment portion.

In some embodiments, the prosthetic device can further comprise a valvular structure including a plurality of leaflets, wherein each pair of adj acent leaflets is coupled to a respective expansion and locking mechanism at a respective commissure attachment portion to form a commissure. In some such embodiments, the aperture is positioned such that when the prosthetic device is in the radially expanded position, the commissure does not cover the aperture.

In some embodiments, the expansion and locking mechanism further comprises a fastener extending radially outward from the first wall of the first member.

In some embodiments, the aperture is positioned distally adjacent to the fastener.

In a representative embodiment, an implantable prosthetic device can comprise a frame movable between a radially compressed and a radially expanded configuration, the frame comprising an inflow end portion and an outflow end portion, and at least one expansion and locking mechanism. The expansion and locking mechanism can comprise a first member coupled to the frame at a first location, the first member comprising a commissure opening extending through a thickness of the first member, a second member coupled to the frame at a second location spaced apart from the first location, the second member extending at least partially into the first member, and a locking member coupled to the first member, the locking member configured to engage the second member to allow movement in a first direction to allow radial expansion of the frame and prevent movement in a second direction to prevent radial compression of the frame. The prosthetic device can further comprise a valvular structure comprising a plurality of leaflets each including one or more tabs, wherein tabs of adjacent leaflets are disposed within the commissure opening to couple the valvular structure to the frame.

In some embodiments, the commissure opening comprises a first aperture and a second aperture forming a channel between them, and wherein the channel has at least one angled surface corresponding to one or more angled edges of the plurality of leaflets.

In some embodiments, the first member comprises a bore extending longitudinally along the length of the first member, and wherein the bore is offset from a longitudinal axis of the first member.

In some embodiments, each expansion and locking mechanism further comprises a wedge disposed between adjacent tabs to help couple the valvular structure to the frame.

In some embodiments, the portion of the first member comprising the commissure opening extends past the outflow end portion of the frame.

In some embodiments, the first member is coupled to the frame via a fastener extending from a surface of the first member. In some such embodiments, the commissure opening is closer to the outflow end portion of the frame than the fastener.

In some embodiments, the second member is coupled to the frame via a fastener extending from a surface of the second member.

In some embodiments, the commissure opening extends to an outflow edge of the first member. In some such embodiments, the commissure opening defines a first extension and a second extension in the outflow end portion of the first member with the commissure opening between them. In some embodiments, the first member comprises a bore extending along a length of the first member and disposed in the second extension.

In some embodiments, the first member comprises an angled portion such that an outflow end portion of the expansion and locking mechanism has a first width and an inflow end portion has a second width narrower than the first width.

In some embodiments, the first member comprises one or more rounded radially inner edges.

In some embodiments, the first member comprises one or more rounded radially outer edges configured to correspond to a radially inner surface of the frame.

In some embodiments, the first member comprises one or more chamfered radially inner edges.

In some embodiments, the first member comprises one or more chamfered radially outer edges.

In a representative embodiment, a method of assembling a prosthetic valve can comprise providing a frame movable between a radially compressed and a radially expanded configuration, the frame comprising an inflow end portion and an outflow end portion, the frame including one or more expansion and locking mechanisms including an outer member comprising a commissure opening extending through a thickness of the outer member, an inner member, and a locking member. The method can further comprise inserting tabs of adjacent leaflets of a valvular structure into the commissure opening such that the tabs extend through the outer member, inserting a wedge between radially outer portions of the tabs to form a commissure assembly, and coupling the commissure assembly to the outer member.

In some embodiments, inserting the tabs into the commissure opening comprises inserting the tabs through a radially inner aperture in the outer member and then inserting the tabs at least partially through a radially outer aperture in the outer member.

In some embodiments, coupling the commissure assembly to the outer member comprises using one or more sutures.

In another representative embodiment, a method of assembling a prosthetic valve can comprise providing a frame movable between a radially compressed and a radially expanded configuration, the frame comprising an inflow end portion and an outflow end portion, the frame including one or more expansion and locking mechanisms including an outer member comprising a commissure opening extending through a thickness of the outer member and extending to an outflow edge of the outer member to form an outflow aperture, an inner member, and a locking member. The method can further comprise inserting a wedge between adjacent tabs of adjacent leaflets of a valvular structure and coupling the wedge to the tabs to form a commissure assembly, inserting the commissure assembly into the commissure opening by sliding the commissure assembly through the outflow aperture, and coupling the commissure assembly to the outer member.

In some embodiments, coupling the wedge to the tabs includes using one or more sutures.

In some embodiments, coupling the commissure assembly to the outer member includes using one or more sutures.

In a representative embodiment, an implantable prosthetic device can comprise a frame movable between a radially compressed and a radially expanded configuration, the frame comprising an inflow end portion and an outflow end portion, and at least one expansion and locking mechanism. The expansion and locking mechanism can comprise a first member coupled to the frame at a first location, the first member comprising an inner bore extending the length of the first member, the bore having a first portion and a second portion separated by a neck portion, a second member coupled to the frame at a second location spaced apart from the first location, the second member extending at least partially into the first portion of the bore, and a locking member comprising a pawl portion and a body portion, the locking member disposed within the second portion of the bore. Engagement of the locking member with the inner member allows movement in a first direction to allow radial expansion of the frame and prevents movement in a second direction to prevent radial compression of the frame.

In some embodiments, at least a portion of the second member has a semi-circular shape in cross-section comprising a curved surface and a flat surface.

In some embodiments, the body portion of the locking member has a semi-circular shape in cross-section comprising a curved surface and a flat surface.

In some embodiments, the flat surfaces of the second member and locking member are oriented toward one another.

In some embodiments, the flat surfaces of the second member and locking member are spaced apart from one another.

In some embodiments, the flat surfaces of the second member and locking member contact one another.

In some embodiments, the flat surfaces of the second member and locking member are coated with a lubricious coating.

In some embodiments, a side wall of the first member comprises an opening aligned with the pawl portion of the locking member such that the pawl portion can selectively deflect into the opening.

In some embodiments, the body portion of the locking member comprises one or more apertures and wherein the locking member is coupled to the first member via one or more protrusions extending from the first member into the apertures.

In another representative embodiment, an implantable prosthetic device can comprise a frame movable between a radially compressed and a radially expanded configuration, the frame comprising an inflow end portion and an outflow end portion, and at least one expansion and locking mechanism. The expansion and locking mechanism can comprise a first member coupled to the frame at a first location, a second member coupled to the frame at a second location spaced apart from the first location, the second member extending at least partially into the first member, and a locking member coupled to the first member via one or more lateral extensions extending from the first member into a recess of the locking member. Engagement of the locking member with the inner member allows movement in a first direction to allow radial expansion of the frame and prevents movement in a second direction to prevent radial compression of the frame.

In some embodiments, the second member comprises a rack having a plurality of teeth arrayed along a length of the second member, and wherein the locking member comprises a locking tooth extending toward the rack and configured to engage the plurality of teeth.

In some embodiments, the lateral extensions have a rectangular shape.

In some embodiments, the lateral extensions have a length along a longitudinal axis of the outer member greater than a width of the lateral extensions.

In some embodiments, the lateral extensions have a length along a longitudinal axis of the outer member less than a width of the lateral extensions.

In some embodiments, the recess has a rectangular shape corresponding to the rectangular shapes of the lateral extensions.

In some embodiments, the recess has a depth corresponding to a thickness of the lateral extensions.

In some embodiments, the lateral extensions are aligned with one another along a length of the first member.

In some embodiments, the lateral extensions are offset from one another along a length of the first member.

In a representative embodiment, a method of making an expansion and locking mechanism can comprise providing an outer member having an inner wall, an outer wall, a first side wall, and a second side wall, the first side wall including an opening and the inner wall and outer wall each comprising one or more bendable lateral extensions aligned with a first portion of the opening. The method can further comprise disposing a locking member within the opening, the locking member comprising a pawl portion and a body portion including a recess, the locking member being disposed within the opening such that the recess is aligned with the lateral extensions, and applying a force to each lateral extension such that the lateral extension deforms into the recess, thereby securing the locking member to the outer member.

In some embodiments, the lateral extensions have a rectangular shape.

In some embodiments, the lateral extensions have a length along a longitudinal axis of the outer member greater than a width of the lateral extensions.

In some embodiments, the lateral extensions have a length along a longitudinal axis of the outer member less than a width of the lateral extensions

In some embodiments, when the lateral extensions are in an undeformed position they extend perpendicular to the first side wall.

In some embodiments, when the lateral extensions are in a deformed position they extend parallel to the first side wall.

In a representative embodiment, an implantable prosthetic device can comprise a frame movable between a radially compressed and a radially expanded configuration, the frame comprising an inflow end portion and an outflow end portion, and at least one expansion and locking mechanism. The expansion and locking mechanism comprising a first member coupled to the frame at a first location, the first member comprising an opening including one or more lateral extensions, a second member coupled to the frame at a second location spaced apart from the first location, the second member extending at least partially into the first member, and a locking member comprising a pawl portion and a body portion, the body portion including first and second angled surfaces, the locking member being coupled to the first member via the one or more lateral extensions engaging the angled surfaces. The locking member can be configured to engage the second member to allow movement in a first direction to allow radial expansion of the frame and prevent movement in a second direction to prevent radial compression of the frame.

In some embodiments, the body portion of the locking member has a triangular shape with chamfered corners in cross-section.

In some embodiments, the angled surfaces are disposed at an angle relative to an inner wall of the locking member. In some embodiments, the inner wall is disposed nearer to the longitudinal axis of the outer member than the angled surfaces. In some embodiments, the angle is a 45 degree angle.

In some embodiments, the second member comprises a rack having a plurality of teeth arrayed along a length of the second member, and wherein the locking member comprises a locking tooth extending toward the rack and configured to engage the plurality of teeth.

In some embodiments, the lateral extensions have a rectangular shape.

In some embodiments, each lateral extension comprises a chamfered edge portion. In some embodiments, the chamfered edge portions are configured such that when the lateral extensions are engaged with the angled surfaces the chamfered edge portions do not extend past the angled surfaces.

In a representative embodiment, a method of making an expansion and locking mechanism can comprise providing a first member having an inner wall, an outer wall, a first side wall comprising an opening, and a second side wall, the inner and outer walls each comprising a lateral extension, and disposing a locking member within the opening, the locking member comprising a body portion including first and second angled surfaces, wherein the locking member is disposed within the opening such that each angled surface aligns with a respective lateral extension. The method can further comprise applying force to the lateral extensions such that the lateral extensions deform to engage the angled surfaces thereby securing the locking member to the first member.

In some embodiments, the force applied is directed toward a longitudinal axis of the first member.

In another representative embodiment, an implantable prosthetic device can comprise a frame movable between a radially compressed and a radially expanded configuration, the frame comprising an inflow end portion and an outflow end portion and at least one expansion and locking mechanism. The expansion and locking mechanism can comprise a first member coupled to the frame at a first location, a second member coupled to the frame at a second location spaced apart from the first location, the second member extending at least partially into the first member, and a locking member comprising a pawl portion and a body portion, the body portion including first and second elongated recesses, the locking member being coupled to the first member via first and second protrusions extending from the first member into the first and second recesses. The locking member can be configured to to engage the second member to allow movement in a first direction to allow radial expansion of the frame and prevent movement in a second direction to prevent radial compression of the frame.

In some embodiments, the first and second elongated recesses each have a V-shape in cross-section. In some embodiments, the opening of the opening of each V-shape is oriented toward the side walls of the first member. In some embodiments, each protrusion has a V-shape that corresponds to the V-shape of a respective recess.

In some embodiments, the second member comprises a rack having a plurality of teeth arrayed along a length of the second member, and wherein the locking member comprises a locking tooth extending toward the rack and configured to engage the plurality of teeth.

In a representative embodiment, a method of making an expansion and locking mechanism can comprise providing a first member having an inner wall, an outer wall, a first side wall including an opening, and a second side wall, and disposing a locking member within the opening, the locking member an outer wall and an inner wall, the outer and inner walls each comprising an elongated recess. The method can further comprise applying a force to the inner and outer walls of the first member such that the inner and outer walls deform to form protrusions that extend into respective recesses thereby securing the locking member to the first member.

In some embodiments, the force applied is directed inwardly toward a longitudinal axis of the first member.

In another representative embodiment, an implantable prosthetic device can comprise a frame movable between a radially compressed and radially expanded configuration, the frame comprising an inflow end portion and an outflow end potion and at least one expansion and locking mechanism. The expansion and locking mechanism can comprise a first member coupled to the frame at a first location via a first fastener, the first fastener comprising a body portion and a flanged end portion, a second member coupled to the frame at a second location via a second fastener, the second fastener comprising a body portion and a flanged end portion, and a locking member coupled to the first member. The body portions of the first and second fasteners can extend through one or more apertures in the frame, and the flanged end portions can be sized to retain the first and second fasteners within the apertures.

In some embodiments, each flanged end portion is formed by radial riveting.

In some embodiments, each fastener is a solid piece of material.

In some embodiments, each fastener further comprises a base portion.

In some embodiments, the first fastener is formed integrally with the first member.

In some embodiments, the body portion of the first fastener extends through an aperture in a wall of the first member.

In a representative embodiment, a method of making a prosthetic valve can comprise providing a frame movable between a radially compressed and a radially expanded configuration, the frame comprising a plurality of struts each including one or more apertures, disposing an expansion and locking mechanism comprising a first member having a first fastener such that the first fastener extends through one or more apertures at a first location, and radially riveting the first fastener to form a first flanged end portion configured to retain the first fastener within its respective apertures, thereby coupling the expansion and locking mechanism to the frame.

In some embodiments, the expansion and locking mechanism further comprises a second member having a second fastener, and wherein the method further comprises disposing the expansion and locking mechanism such that the second fastener extends through one or more apertures at a second location spaced apart from the first location along a longitudinal axis of the frame, and radially riveting the second fastener to form a second flanged end portion configured to retain the second fastener within its respective apertures.

In another a representative embodiment, an expansion and locking mechanism can comprise an outer member comprising a first wall and a second wall, the first wall comprising an opening extending through the first wall, the opening comprising a main portion, a guide portion, and an entry portion, and a fastener having a base portion and a body portion, the body portion having one or more recesses configured such that when the recesses are aligned with the guide portion the fastener can slide along the guide portion and into the main opening and when the recesses are offset from the guide portion the fastener is retained within the main portion.

In some embodiments, the recesses are configured such that the portion of the body portion on which the recesses are disposed has a non-circular shape in cross-section.

In some embodiments, the main portion of the opening has a first width greater than a second width of the guide portion.

In some embodiments, the entry portion has a width corresponding to the width of the base portion of the fastener.

In a representative embodiment, a method of making an expansion and locking mechanism can comprise deforming a tubular member including an inner bore extending along the length of the tubular member such that it forms an oval shape in cross-section having first, second, third, and fourth walls, and cutting a fastener opening and inflow end cutout in the first wall and a locking member opening in the second wall. The method can further comprise disposing a locking member including a first end portion comprising a pawl and a second end portion within the locking member opening, disposing a fastener within the fastener opening, and disposing an inner member at least partially within the inner bore of the tubular member.

In some embodiments, the method further comprises deforming the first and third walls to form elongated indentations configured to retain the locking member within the locking member opening.

In some embodiments, the fastener opening comprises a main portion having a first width and a guide portion having a second width narrower than the first width. In some embodiments, disposing the fastener within the fastener opening comprises aligning one or more recesses in the fastener with the guide portion, sliding the fastener through the guide portion into the main portion, and rotating the fastener within the main portion such that the recesses are offset from the guide portion.
In some embodiments, the method can further comprise deforming the guide portion once the fastener is disposed within the main portion.

In some embodiments, the method can further comprise cutting a commissure opening in the third wall of the tubular member to form two deflectable portions, and bending the deflectable portions toward the first wall to form first and second commissure posts.

In another representative embodiment, a method of making an expansion and locking mechanism comprises deforming a sheet of material having first and second edges to form an elongated member having a substantially rectangular shape with rounded edges in cross-section, the elongated member comprising an inner bore extending along the length of the elongated member and a slot extending along the length of the elongated member defined by the first and second edges, and cutting a fastener opening and inflow end cutout in a first wall of the elongated member and a locking member opening in a second wall. The method can further comprise disposing a locking member including a first end portion comprising a pawl and a second end portion within the locking member opening, disposing a fastener within the fastener opening, and disposing an inner member at least partially within the inner bore of the elongated member.

In some embodiments, the fastener opening and the inflow end cutout incorporate at least a portion of the slot.

In some embodiments, the method further comprises deforming the first and third walls to form elongated indentations configured to retain the locking member within the locking member opening.

In some embodiments, the fastener opening comprises a main portion having a first width and a guide portion having a second width narrower than the first width.

In some embodiments, disposing the fastener within the fastener opening comprises aligning one or more recesses in the fastener with the guide portion, sliding the fastener through the guide portion into the main portion, and rotating the fastener within the main portion such that the recesses are offset from the guide portion. In some embodiments, the method further comprises cutting a commissure opening in the third wall of the tubular member to form two deflectable portions, and bending the deflectable portions toward the first wall to form first and second commissure posts.

The foregoing and other objects, features, and advantages of the disclosure will become more apparent from the following detailed description, which proceeds with reference to the accompanying figures.

### BRIEF DESCRIPTION OF THE DRAWINGS

FIG. 1 is a perspective view of an exemplary embodiment of a prosthetic heart valve.
FIG. 2 is a perspective view of a portion of another exemplary embodiment of a prosthetic heart valve.
FIG. 3 is a side view of the frame of the prosthetic heart valve of FIG. 2 shown in a radially collapsed configuration.
FIG. 4 is a side view of the frame of the prosthetic heart valve of FIG. 2 shown in a radially expanded configuration.
FIG. 5 is a side view of an embodiment of a strut for a frame of a prosthetic valve, such as the frame of FIG. 4.
FIG. 6 is a side elevation view of an embodiment of a prosthetic valve delivery assembly.
FIG. 7 is a perspective view of a portion of an exemplary embodiment of a frame for a prosthetic heart valve.
FIG. 8 is a side elevation view of a portion of an exemplary embodiment of a locking feature.
FIG. 9 is a perspective view of a portion of the frame of FIG. 7 in a radially compressed configuration.
FIG. 10 is a perspective view of a portion of the frame of FIG. 7 in a transitional position between the radially compressed configuration and the radially expanded configuration.
FIG. 11 is a perspective view of a portion of the frame of FIG. 7 in a transitional position between the radially compressed configuration and the radially expanded configuration.
FIG. 12 is a perspective view of a portion of the frame of FIG. 7 in a transitional position between the radially compressed configuration and the radially expanded configuration.
FIG. 13 is a perspective view of a portion of the frame of FIG. 7 in the radially expanded configuration.
FIG. 14 is a perspective view of a frame for a prosthetic heart valve comprising three expansion and locking mechanisms, according to another embodiment.
FIG. 15 is a side elevation view of a portion the frame comprising an expansion and locking mechanism of FIG. 14.
FIG. 16 is a perspective view of a portion of the frame comprising an expansion and locking mechanism of FIG. 14.
FIG. 17 is a cross-sectional side elevation view of the expansion and locking mechanism of FIG. 14.
FIG. 18 is a cross-sectional side elevation view of a portion of the expansion and locking mechanism of FIG. 14.
FIG. 19 is a cross-sectional side elevation view of a portion of the expansion and locking mechanism of FIG. 14.
FIG. 20 is a perspective view of the expansion and locking mechanism of FIG. 14 coupled to a delivery apparatus.
FIG. 21 is a partial cross-sectional side elevation view of the expansion and locking mechanism of FIG. 14 coupled to a delivery apparatus.
FIG. 22 is a partial cross-sectional side elevation view of the expansion and locking mechanism of FIG. 14 coupled to a delivery apparatus.
FIG. 23 is a partial cross-sectional side elevation view of the expansion and locking mechanism of FIG. 14 uncoupled from a delivery apparatus.
FIG. 24 is a cross-sectional side elevation view of the expansion and locking mechanism of FIG. 14 further comprising a retaining member.
FIG. 25 is a top plan view of the frame and expansion and locking mechanisms of FIG. 14.
FIG. 26 is a perspective view of an exemplary embodiment of a frame for a prosthetic heart valve.
FIG. 27 is an elevational view of an exemplary embodiment of a locking mechanism mounted to a portion of the frame of FIG. 26.
FIG. 28 is an elevational view of an exemplary embodiment of a commissure attachment post mounted to a portion of the frame of FIG. 26.
FIG. 29 is a perspective view of a frame for a prosthetic heart valve comprising three expansion and locking mechanisms, according to another embodiment.
FIG. 30 is a perspective view of the frame of FIG. 29 shown in the partially compressed configuration.
FIG. 31 is an exploded perspective view of an expansion and locking mechanism of FIG. 29.
FIG. 32 is a perspective view of the expansion and locking mechanism of FIG. 31.
FIG. 33A is a perspective view of the inner member of the expansion and locking mechanism of FIG. 31.
FIG. 33B is an end-on view of the inner member of the expansion and locking mechanism of FIG. 31.
FIG. 34A is a perspective view of the outer member of the expansion and locking mechanism of FIG. 31.
FIG. 34B is an end-on view of the distal end of the outer member of the expansion and locking mechanism of FIG. 31.
FIG. 35 is a perspective view of the locking member of the expansion and locking mechanism of FIG. 31.
FIG. 36 is an end-on view of the proximal end of the expansion and locking mechanism of FIG. 31, with the locking member assembled on the outer member.
FIG. 37 is a cross-sectional elevational side view of the expansion and locking mechanism of FIG. 31.
FIG. 38 is a cross-sectional perspective view of the expansion and locking mechanism of FIG. 31.
FIG. 39 is a cross-sectional perspective view of the expansion and locking mechanism of FIG. 31.
FIG. 40 is a cross-sectional end-on view of the expansion and locking mechanism of FIG. 31 including an exemplary punch member.
FIG. 41 is a perspective view of the expansion and locking mechanism of FIG. 31 including an exemplary disengagement member.
FIGS. 42-44 are cross-sectional side views of the expansion and locking mechanism of FIG. 31 including the exemplary disengagement member of FIG. 41.
FIGS. 45-46 are side elevational views of the locking member of the expansion and locking mechanism of FIG. 31.
FIG. 47 is a perspective view of an exemplary embodiment of a frame for a prosthetic heart valve comprising an exemplary embodiment of an expansion and locking mechanism.
FIG. 48 is a perspective view of a portion of the frame and expansion and locking mechanism of FIG. 47.
FIG. 49 is a cross-sectional elevational side view of the expansion and locking mechanism of FIG. 47.
FIG. 50A is a side elevational view of an expansion and locking mechanism, according to one embodiment.
FIG. 50B is an enlarged view of a portion of the expansion and locking mechanism of FIG. 50A.
FIG. 51 is a side elevational view of the expansion and locking mechanism of FIG. 50A with the outer member transparent.
FIG. 52 is a perspective view of a portion of a prosthetic heart valve comprising the expansion and locking mechanism of FIG. 50A.
FIG. 53 is a perspective view of a frame for a prosthetic heart valve comprising three expansion and locking mechanisms, according to another embodiment.
FIG. 54 is an exploded perspective view of an expansion and locking mechanism of FIG. 53.
FIG. 55 is a perspective view of an expansion and locking mechanism of FIG. 53.
FIG. 56 is a perspective view of an expansion and locking mechanism of FIG. 53 with the outer member transparent.
FIG. 57 is a side elevational view of the outer member of an expansion and locking mechanism of FIG. 53.
FIG. 58 is a cross-sectional view of the outer member of FIG. 57 including a commissure assembly.
FIG. 59 is an end-on view of the outflow end of the outer member of FIG. 57.
FIGS. 60-62 are perspective views of a commissure assembly being inserted into the expansion and locking mechanism of FIG. 53.
FIG. 63 is a perspective view of a portion of the expansion and locking mechanism of FIG. 53 including a commissure assembly.
FIG. 64 is a perspective view of a frame for a prosthetic heart valve comprising three expansion and locking mechanisms, according to another embodiment.
FIG. 65 is a perspective view of an expansion and locking mechanism of FIG. 64.
FIG. 66 is a perspective view of the expansion and locking mechanism of FIG. 65 with the outer member transparent.
FIG. 67 is a perspective view of the outer member of the expansion and locking mechanism of FIG. 65.
FIG. 68 is a side elevational view of the outer member of FIG. 67.
FIG. 69 is an end-on view of the outflow end of the outer member of FIG. 67.
FIGS. 70-72 are perspective views of a commissure assembly being inserted into the expansion and locking mechanism of FIG. 65.
FIG. 73 is a perspective view of a portion of the expansion and locking mechanism of FIG. 65 including a commissure assembly.
FIG. 74 is an end-on view of the outflow end of the expansion and locking mechanism of FIG. 65 including a commissure assembly.
FIG. 75 is a perspective view of a frame for a prosthetic heart valve comprising three expansion and locking mechanisms, according to another embodiment.
FIG. 76 is a perspective view of an expansion and locking mechanism of FIG. 75.
FIG. 77 is a perspective view of the expansion and locking mechanism of FIG. 76 with the outer member transparent.
FIG. 78 is a perspective view of the outer member of the expansion and locking mechanism of FIG. 76.
FIG. 79 is a side elevational view of the outer member of FIG. 78.
FIG. 80 is an end-on view of the outflow end of the outer member of FIG. 78.
FIG. 81A is an end-on view of the outflow end of a prosthetic heart valve comprising three expansion and locking mechanisms.
FIGS. 81B-81C illustrate various views of an expansion and locking mechanism of FIG. 81A.
FIG. 82A is an end-on view of the outflow end of a prosthetic heart valve comprising three expansion and locking mechanisms.
FIGS. 82B-82C illustrate various views of an expansion and locking mechanism of FIG. 82A.
FIG. 83A is an end-on view of the outflow end of a prosthetic heart valve comprising three expansion and locking mechanisms.
FIGS. 83B-83C illustrate various views of an expansion and locking mechanism of FIG. 83A.
FIG. 84 is a perspective view of an exemplary expansion and locking mechanism.
FIG. 85 is a cross-sectional side elevational view of the expansion and locking mechanism of FIG. 84.
FIG. 86 is a perspective view of the locking member of the expansion and locking mechanism of FIG. 84.
FIG. 87 is a perspective view of the inner member of the expansion and locking mechanism of FIG. 84.
FIG. 88 is an end-on view of the outflow end of the expansion and locking mechanism of FIG. 84.
FIG. 89 is a perspective view of the outer member of another exemplary expansion and locking mechanism.
FIG. 90 is a perspective view of the locking member of the expansion and locking mechanism of FIG. 89.
FIG. 91 is a perspective view of the outer member of the expansion and locking mechanism of FIG. 89.
FIG. 92 is a perspective view of a portion of the expansion and locking mechanism of FIG. 89.
FIG. 93 is an end-on view of the outflow end of the expansion and locking mechanism of FIG. 92.
FIG. 94 is a perspective view of the expansion and locking mechanism of FIG. 92.
FIG. 95 is a perspective view of a portion of an outer member of an embodiment of an expansion and locking mechanism.
FIG. 96 is a perspective view of a portion of a locking member of the expansion and locking mechanism of FIG. 95.
FIG. 97 is a perspective view of a portion of an expansion and locking member including the outer member of FIG. 95 and the locking member of FIG. 96.
FIG. 98 is an end-on cross-sectional view of the expansion and locking member of FIG. 97 along the line 98-98.
FIG. 99 is a perspective view of a portion of a locking member of another embodiment of an expansion and locking mechanism.
FIG. 100 is a perspective view of an expansion and locking mechanism including the locking mechanism of FIG. 99.
FIG. 101 is an end-on cross-sectional view of a portion of the expansion and locking mechanism of FIG. 100 along the line 101-101.
FIG. 102 is a perspective view of an embodiment of a fastener shown disposed at a junction between two struts after the radial riveting process.
FIG. 103 is a perspective view of the fastener of FIG. 102 prior to the radial riveting process.
FIG. 104 is a perspective view of the fastener of FIG. 102.
FIG. 105 is a side elevational view of the fastener of FIG. 102 during the process of radial riveting using a riveting member.
FIG. 106 is a perspective view of an embodiment of an outer member for use in an expansion and locking mechanism.
FIG. 107 is a perspective view of the outer member of FIG. 106 including various exemplary cutouts.
FIG. 108 is a perspective view of the outer member of FIG. 107 including an exemplary embodiment of a commissure opening.
FIG. 109 is a perspective view of the outer member of FIG. 108.
FIGS. 110-111 are perspective views of the outer member of FIG. 107 including various embodiments of commissure openings.
FIG. 112 is an exploded perspective view of an embodiment of an expansion and locking mechanism including the outer member of FIG. 108.
FIG. 113 is a perspective view of a portion of the expansion and locking mechanism of FIG. 112.
FIG. 114 is a perspective view of the expansion and locking mechanism of FIG. 112.
FIG. 115 is a cross-sectional perspective view of the expansion and locking mechanism of FIG. 112 along line 115-115 including an inner member.
FIG. 116 is a perspective view of another embodiment of an outer member for use in an expansion and locking mechanism.
FIG. 117 is a perspective view of the outer member of FIG. 116 including various exemplary cutouts.
FIG. 118 is an exploded perspective view of an embodiment of an expansion and locking mechanism including the outer member of FIG. 117.
FIG. 119 is a perspective view of a portion of the expansion and locking mechanism of FIG. 118.
FIG. 120 is a perspective view of the expansion and locking mechanism of FIG. 118 including an inner member.

### DETAILED DESCRIPTION

Described herein are examples of prosthetic implant delivery assemblies and components thereof which can improve a physician's ability to control the size of a mechanically-expandable prosthetic implant, such as prosthetic valves (e.g., prosthetic heart valves or venous valves), stents, or grafts, as well as facilitate separation of the prosthetic implant from the delivery assembly, during the implantation procedure. The present disclosure also provides frames for use with such prosthetic implants. The frames can comprise locking mechanisms configured to hold the frame in an expanded configuration when the implant is expanded at a selected delivery site within a patient.

Prosthetic valves disclosed herein can be radially compressible and expandable between a radially compressed configuration and a radially expanded configuration. Thus, the prosthetic valves can be crimped on an implant delivery apparatus in the radially compressed configuration during delivery, and then expanded to the radially expanded configuration once the prosthetic valve reaches the implantation site.

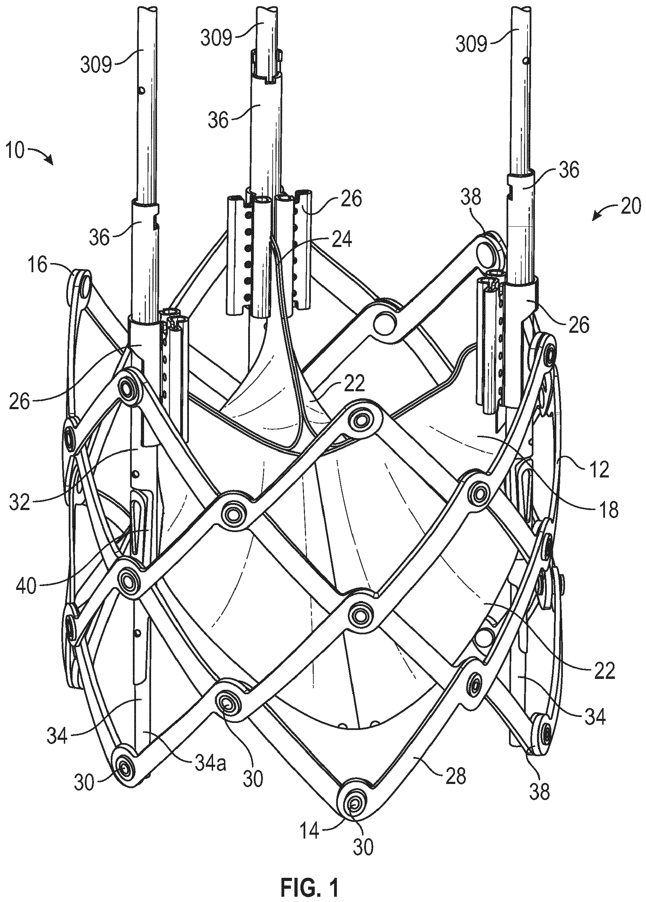

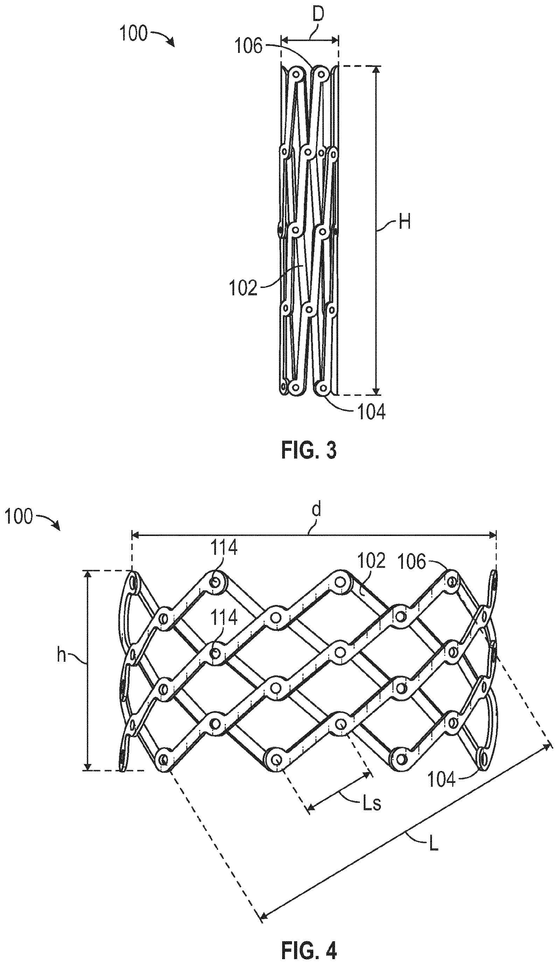

FIG. 1 shows an exemplary prosthetic valve 10, according to one embodiment. The prosthetic valve 10 can be radially compressible and expandable between a radially compressed configuration for delivery into a patient (see e.g., FIG. 3) and a radially expanded configuration (see e.g., FIGS. 1 and 4). In particular embodiments, the prosthetic valve 10 can be implanted within the native aortic annulus, although it also can be implanted at other locations in the heart, including within the native mitral valve, the native pulmonary valve, and the native tricuspid valve. The prosthetic valve 10 can include an annular stent or frame 12 having a first end 14 and a second end 16.

In the depicted embodiments, the first end 14 is an inflow end and the second end 16 is an outflow end. The outflow end 16 can be coupled to a delivery apparatus for delivering and implanting the prosthetic valve within the native aortic valve is a transfemoral, retrograde delivery approach. Thus, in the delivery configuration of the prosthetic valve, the outflow end 16 is the proximal-most end of the prosthetic valve. In other embodiments, the inflow end 14 can be coupled to the delivery apparatus, depending on the particular native valve being replaced and the delivery technique that is used (e.g., trans-septal, transapical, etc.). For example, the inflow end 14 can be coupled to the delivery apparatus (and therefore is the proximal-most end of the prosthetic valve in the delivery configuration) when delivering the prosthetic valve to the native mitral valve via a trans-septal delivery approach.

The prosthetic valve 10 can also include a valvular structure 18 which is coupled to the frame 12 and configured to regulate the flow of blood through the prosthetic valve 10 from the inflow end to the outflow end. The prosthetic valve 10 can further include a plurality of actuators 20 mounted to and equally spaced around the inner surface of the frame 12. Each of the actuators 20 can be configured to form a releasable connection with one or more respective actuators of a delivery apparatus, as further described below.

The valvular structure 18 can include, for example, a leaflet assembly comprising one or more leaflets 22 (three leaflets 22 in the illustrated embodiment) made of a flexible material. The leaflets 22 of the leaflet assembly can be made from in whole or part, biological material, bio-compatible synthetic materials, or other such materials. Suitable biological material can include, for example, bovine pericardium (or pericardium from other sources). The leaflets 22 can be arranged to form commissures 24, which can be, for example, mounted to respective actuators 20. Further details regarding transcatheter prosthetic heart valves, including the manner in which the valvular structure can be coupled to the frame 12 of the prosthetic valve 10, can be found, for example, in U.S. Patent Nos. 6,730,118, 7,393,360, 7,510,575, 7,993,394, and 8,652,202, and U.S. Patent Application Publication No. 2018/0325665, all of which are incorporated herein by reference in their entireties.

In some embodiments, the prosthetic valve 10 can include a plurality of commissure support elements configured as commissure clasps or clamps 26. In the illustrated configuration, the prosthetic valve includes a commissure clamp 26 positioned at each commissure 24 and configured to grip adjacent portions of two leaflets 22 at each commissure 24 at a location spaced radially inwardly of the frame 12. Each clamp 26 can be mounted on an actuator 20 as shown. In alternative embodiments, the commissure supports elements (such as clamps 26) can be mounted to the struts 28 of the frame, or alternatively, the commissures 24 can be mounted (e.g., sutured) directly to the struts of the frame. Further details of the commissure clamps 26 and other techniques for mounting the commissures of a valve assembly to a frame can be found in U.S. Patent Application Publication No. 2018/0325665.

Although not shown, the prosthetic valve 10 can also include one or more skirts or sealing members. For example, the prosthetic valve 10 can include an inner skirt mounted on the inner surface of the frame. The inner skirt can function as a sealing member to prevent or decrease perivalvular leakage, to anchor the leaflets 22 to the frame, and/or to protect the leaflets against damage caused by contact with the frame during crimping and during working cycles of the prosthetic valve. The prosthetic valve 10 can also include an outer skirt mounted on the outer surface of the frame 12. The outer skirt can function as a sealing member for the prosthetic valve by sealing against the tissue of the native valve annulus and helping to reduce paravalvular leakage past the prosthetic valve. The inner and outer skirts can be formed from any of various suitable biocompatible materials, including any of various synthetic materials (e.g., PET) or natural tissue (e.g., pericardial tissue). The inner and outer skirts can be mounted to the frame using sutures, an adhesive, welding, and/or other means for attaching the skirts to the frame.

The frame 12 can be made of any of various suitable materials, such as stainless steel, a cobalt chromium alloy, or a nickel titanium alloy ("NiTi"), for example Nitinol. Referring again to FIG. 1, as shown, the frame 12 can include a plurality of interconnected struts 28 arranged in a lattice-type pattern. The struts 28 are shown as positioned diagonally, or offset at an angle relative to, and radially offset from, a longitudinal axis of the prosthetic valve 10 when the prosthetic valve 10 is in the expanded configuration. In other implementations, the struts 28 can be offset by a different amount than depicted in FIG. 1, or some or all of the struts 28 can be positioned parallel to the longitudinal axis of the prosthetic valve 10.

In the illustrated embodiment, the struts 28 are pivotably coupled to one another at one or more pivot joints or junctions along the length of each strut. For example, in the illustrated configuration, each of the struts 28 can be formed with apertures (see e.g., apertures 114 in FIG. 4) at opposing ends of the strut and apertures spaced along the length of the strut. Respective hinges can be formed at the locations where struts 28 overlap each other via fasteners or pivot members, such as rivets or pins 30 that extend through the apertures. The hinges can allow the struts 28 to pivot relative to one another as the frame 12 is radially expanded or compressed, such as during assembly, preparation, or implantation of the prosthetic valve 10.

In some embodiments, the frame 12 can be constructed by forming individual components (e.g., the struts and fasteners of the frame) and then mechanically assembling and connecting the individual components together. In other embodiments, the struts 28 are not coupled to each other with respective hinges but are otherwise pivotable or bendable relative to each other to permit radial expansion and contraction of the frame 12. For example, the frame 12 can be formed (e.g., via laser cutting, electroforming or physical vapor deposition) from a single piece of material (e.g., a metal tube). Further details regarding the construction of the frame and the prosthetic valve are described in U.S. Publication Nos. 2018/0153689; 2018/0344456; 2019/0060057 all of which are incorporated herein by reference. Additional examples of expandable prosthetic valves that can be used with the delivery apparatuses disclosed herein are described in U.S. Publication No. 2015/0135506 and 2014/0296962, which are incorporated herein by reference.

Referring still to FIG. 1, in some embodiments, the prosthetic valve 10 can comprise one or more actuators 20 configured to produce radial expansion and compression of the frame. The one or more actuators in the illustrated embodiment comprise one or more push-pull mechanisms 32 coupled to the frame 12. In the illustrated embodiment, the prosthetic valve 10 has three push-pull mechanisms 32, however, in other embodiments a greater or fewer number of push-pull mechanisms 32 can be used.

Each push-pull mechanism 32 can generally comprise an inner member 34, such as an inner tubular member, and an outer member 36 disposed about the inner member 34. The inner members 34 and the outer members 36 can be movable longitudinally relative to each other in a telescoping manner to radially expand and contract the frame 12, as further described in U.S. Publication Nos. 2018/0153689, 2018/0153689 and 2018/0325665 which are incorporated herein by reference. The inner members 34 can be, for example, rods, cables, wires, or tubes. The outer members 36 can be, for example, tubes or sheaths having sufficient rigidity such that they can apply a distally directed force to the frame without bending or buckling.

The inner members 34 can have distal end portions 34a coupled to the inflow end 14 of the frame 12 (e.g., with a coupling element such as a pin member 30). In the illustrated embodiment, each of the inner members 34 are coupled to the frame at respective apices 38 at the inflow end 14 of the frame 12. For example, the distal end portion 34a of each inner member 34 can be pivotably connected to the rivet or pin 30 that connects the two struts at the adjacent apex 38. The outer members 36 can be coupled to apices 38 at the outflow end 16 of the frame 12 at, for example, a mid-portion of the outer member 36, as shown in FIG. 1, or at a proximal end portion of the outer member, as desired. The outer members 36 can be pivotably connected to the rivet or pin 30 that connects the two struts at the adjacent apex 38.

The inner member 34 and the outer member 36 can telescope relative to each other between a fully contracted state (corresponding to a fully radially expanded state of the prosthetic valve) and a fully extended state (corresponding to a fully radially compressed state of the prosthetic valve). In the fully extended state, the inner member 34 is fully extended from the outer member 36. In this manner, the push-pull mechanisms 32 allow the prosthetic valve to be fully expanded or partially expanded to different diameters and retain the prosthetic valve in the partially or fully expanded state. It should be understood that the inner members 34 and the outer members 36 can be coupled to other locations on the frame to produce radial compression and expansion of the frame, so long as the inner member and outer member of each actuator are coupled at axial spaced pivot joints of the frame.

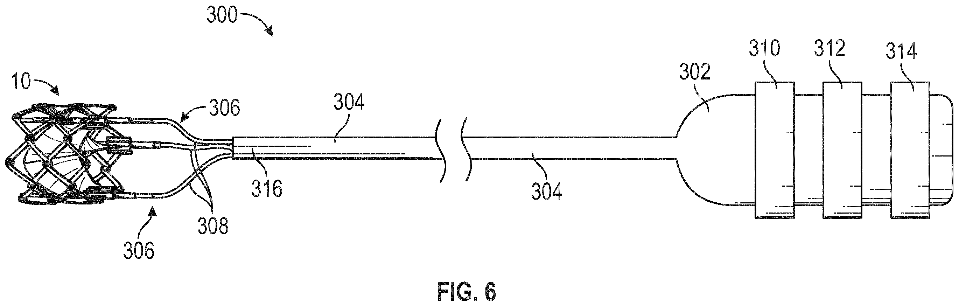

In use, a delivery apparatus (see, e.g., delivery apparatus 300 in FIG. 6) can be releasably coupled to the actuators 20 (e.g., push-pull mechanisms 32) of prosthetic valve 10. For example, the delivery apparatus can have one or more actuator assemblies (e.g., actuator assemblies 306 in FIG. 6) that are releasably coupled to respective actuators 20 (e.g., push-pull mechanisms 32) of the prosthetic valve. The actuator assemblies can be configured to transfer expansion forces (e.g., pushing and/or pulling forces) from a handle of the delivery apparatus to the push-pull mechanisms 32 of the prosthetic valve. Each of the actuator assemblies can include an inner member 309 that is releasably coupled to a respective inner member 34 of a push-pull mechanism 32. Each actuator assembly of the delivery apparatus can also include an outer member 308 (FIG. 6) that is releasably coupled to a respective outer member 36 of a push-pull mechanism 32.

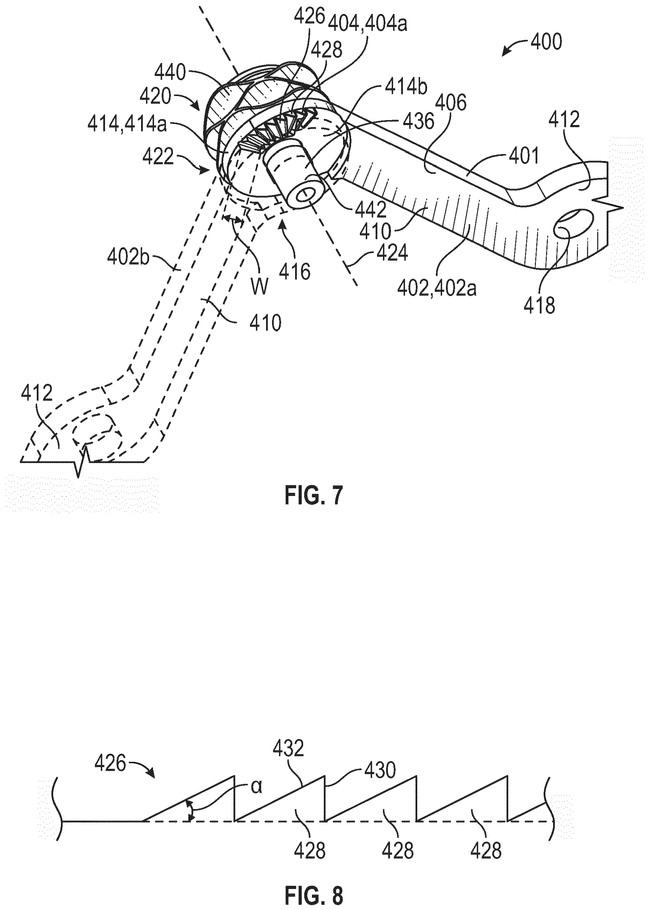

Once coupled to the delivery apparatus, the prosthetic valve 10 can then be radially collapsed (see e.g., FIG. 3) and the distal end portion of the delivery apparatus, along with the radially collapsed valve, can be inserted into a patient. Once the prosthetic valve 10 is at the desired implantation site, the prosthetic valve can be radially expanded (see e.g., FIG. 4). In some embodiments, as shown in FIG. 1, the push-pull mechanisms 32 can comprise one or more locking mechanisms 40, allowing the frame 12 to maintain an expanded diameter after the prosthetic valve is released from the delivery apparatus. Additional details of the locking mechanism 40 can be found in Patent Publication No. 2018/0153689. In other embodiments, the frame 12 can comprise additional and/or alternative locking mechanisms, for example, locking features 404 as described in more detail below with reference to FIGS. 7-13.

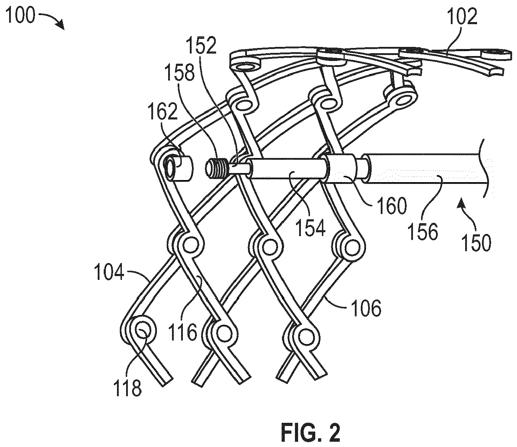

FIG. 2 illustrates a medical assembly, according to another embodiment. The assembly comprises a prosthetic valve 100 and one or more linear actuator assemblies 150 (one shown in FIG. 2) releasably coupled to the prosthetic valve. The prosthetic valve 100 comprises a frame 102. The prosthetic valve 100 can include leaflets 18 and inner and/or outer skirts as previously described, although these components are omitted for purposes of illustration. The frame 102 comprises a plurality of struts 116 formed with apertures 114 (see FIG. 4) and pivot members 118 (e.g., pins or rivets) connecting the struts to each other form a plurality of pivot joints. The frame 102 can have the same construction as the frame 12, except that the frame 102 includes struts 116 that are longer than struts 28 of frame 12. The longer struts 116 form more pivot joints along the length of each strut and more openings or cells of the frame compared to the struts 28.

The one or more actuator assemblies 150 can be components of a delivery apparatus and are configured to produce radial expansion and compression of the frame 102. FIG. 2 shows a linear actuator assembly 150 in the process of being disconnected from the frame 102 after the frame has been radially expanded. As shown, the actuator assembly 150 can include an inner actuator member 152 (which can also be referred to as an actuation member), a cover tube 154 extending co-axially over the actuator member 152, a support tube or pusher member 156 extending co-axially over the cover tube 154, a threaded screw 158, and a stopper 160 fixedly mounted on the frame 102. The actuator member 152 can be, for example, a rod, cable, or wire. The actuator member 152 can be connected at its distal end to the threaded screw 158 such that rotation of the actuator member 152 causes rotation of the threaded screw 158. The proximal end of the actuator member 152 can be connected to a handle or other control device (not shown) of the delivery apparatus that a doctor or operator of the delivery apparatus can use to rotate the actuator member 152. Similarly, the proximal ends of each cover tube 154 and each support tube 156 can be connected to the handle.

The screw 158 has an externally threaded surface that can engage an internally threaded surface of a nut or sleeve 162, which is affixed to the frame 102, such as at the distal end of the frame. When the actuator member 152 is rotated to screw the screw 158 into the sleeve 162, the actuator member 152 becomes connected to the distal end of the frame 102 such that proximal or distal motion of the actuator member 152 causes proximal or distal motion, respectively, of the distal end of the frame 102.

The cover tube 154 annularly surrounds the actuator member 152. The cover tube 154 can be connected to the actuator member 152 such that the actuator member 152 and the cover tube 154 rotate together and move axially together. The actuator member 152 and the cover tube 154 extend through the stopper 160, which can be affixed to a proximal end of the frame. The support tube 156 annularly surrounds the cover tube 154. The stopper 160 has an annular inner surface with an inner diameter larger than the outer diameter of the cover tube 154 and the screw 158 such that the cover tube 154 and the screw 158 can be retracted through the stopper 160 as the frame 102 is expanded and once the actuator is retracted proximally by the user to disconnect it from the frame. The stopper 160 is sized to abut or engage the distal end of the support tube 156 such that the support tube 156 is prevented from moving distally beyond the stopper 160.

In operation, prior to implantation in a patient, the screw 158 is threaded into the sleeve 162, thereby connecting the linear actuator assembly 150 to the frame 102. The frame 102 can then be placed in a radially collapsed state and the prosthetic valve and the distal end portion of the delivery apparatus can be inserted in a patient. Once the prosthetic valve 100 is at a desired implantation site, the frame 102 can be radially expanded as described herein.

To radially expand the frame 102, the support tube 156 is held firmly against the stopper 160. The actuator member 152 is then pulled in a proximal direction through the support tube 156, such as by pulling on the proximal end of the actuator member 152 or actuating a control knob on the handle that produces proximal movement of the actuator member 152. Because the support tube 156 is being held against the stopper 160, which is connected to the proximal end of the frame 102, the proximal end of the frame 102 is prevented from moving relative to the support tube 156 and the handle. As such, movement of the actuator member 152 in a proximal direction results in movement of the distal end of the frame 102 in a proximal direction causing the frame 102 to foreshorten axially and expand radially.

It should be understood that the frame 102 can also be radially expanded by pushing the proximal end of the frame toward the distal end of the frame by pushing the support tube 156 against the stopper 160 while keeping the actuator member 152 stationary relative to the handle, or alternatively, by simultaneously pushing the support tube 156 distally against the stopper 160 and pulling the actuator member 152 proximally.

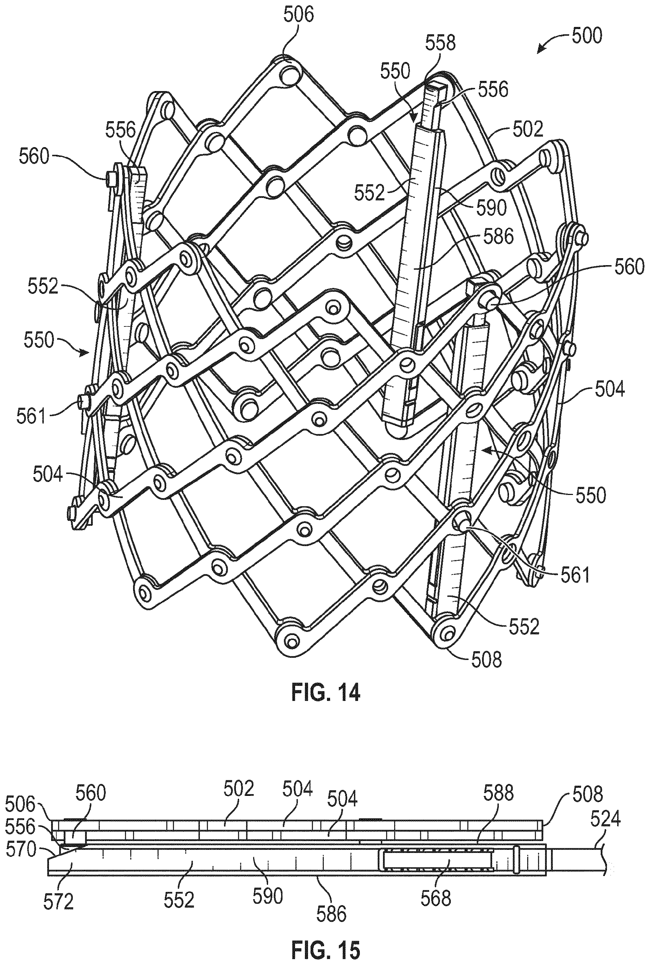

After the frame 102 is expanded to a desired radially expanded size, one or more locking mechanisms can be actuated to lock the frame 102 in the desired radially expanded size, as discussed in further detail below (see FIGS. 14-25), and the linear actuator assembly 150 can be disconnected from the frame 120. To disconnect the linear actuator assembly 150 from the frame 102, the actuator member 152 can be rotated so as to unscrew the screw 158 from the sleeve 162. The actuator member 152 and the cover tube 154 can then be retracted proximally through the stopper 160 and the linear actuator assembly 150 (including the actuator member 152, the screw 158, the cover tube 154, and the support tube 156) can be withdrawn from the patient. The cover tube 154 facilitates passage of the screw 158 through the stopper 160. In some embodiments, the cover tube 154 can be excluded. In embodiments that have more than one linear actuator assembly 150, the above procedure for expanding the frame 102 is performed for each linear actuator assembly 150.

Further details of the actuator assemblies and various exemplary locking mechanisms can be found in U.S. Publication No. 2018/0153689. In some embodiments, the locking mechanism can be formed integrally with the struts of the prosthetic valve, such as described in more detail below.

FIGS. 3-4 illustrate the bare frame 102 (without the leaflets and other components) of the prosthetic valve 100 for purposes of illustrating expansion of the prosthetic valve from the radially compressed configuration to the radially expanded configuration. FIG. 3 shows the frame 102 in the radially compressed configuration, and FIG. 4 shows the frame 102 in the fully radially expanded configuration. The prosthetic valve 100 in the illustrated configuration can be radially expanded by maintaining the first end 104 of the frame 102 at a fixed position while applying a force in the axial direction against the second end 106 toward the first end 104. Alternatively, the prosthetic valve 100 can be expanded by applying an axial force against the first end 104 while maintaining the second end 106 at a fixed position, or by applying opposing axial forces to the first and second ends 104, 106, respectively.

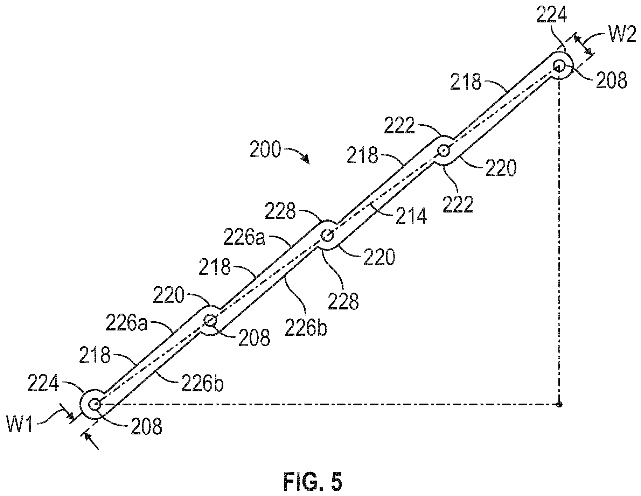

FIG. 5 illustrates a representative embodiment of a strut 200. A plurality of such struts 200 can be arranged in a lattice pattern to form a frame, such as frames 12 and 100, described above.

Each strut 200 can have an offset, or zig-zag, pattern defined by a plurality of offset linear portions or segments 218. The linear segments 218 in the illustrated embodiment are arranged end-to-end relative to each other with adjacent ends interconnected to each other by intermediate segments 220. The strut 200 can have enlarged end portions 224 that form the apices at the inflow and outflow end of the frame. Each linear segment 218 is slightly laterally offset from an adjacent linear segment 218 in a direction perpendicular to the overall length of the strut 200 to provide the zig-zag pattern to the strut. Each of the intermediate segments 220 and end portions 224 can have a respective aperture 208 at its geometric center for receiving a fastener.

The amount of offset of each linear segment 218 relative to an adjacent linear segment along the length of the strut 200 can be constant such that an imaginary line 214 can pass through the aperture 208 of each intermediate segment 220 along the entire length of the strut. In alternative embodiments, the amount of offset between two adjacent linear segments 218 can vary along the length of the strut. For example, the amount of offset between linear segments 218 adjacent the outflow end of the frame can be greater than the amount of offset between linear segments 218 adjacent the inflow end of the frame, or vice versa.

The linear segments 218 can include at least substantially flat or linear opposing longitudinal edges 226a, 226b extending between curved or rounded edges 228 of the intermediate segments 220. In alternative embodiments, the opposing edges 228 of the intermediate segments 220 can be substantially flat or linear edges that extend at an angle between respective ends of the edges 226a, 226b of the liner segments 218.

As best shown in FIG. 5, the width W1 of each liner segment 218 is defined as the distance measured between the opposing edges 226a, 226b of a segment 218. In the illustrated embodiment, the width W1 is constant along the length of the strut 200. As such, each longitudinal edge 226a is laterally offset from an adjacent longitudinal edge 226a of an adjacent linear segment 218, and each longitudinal edge 226b is laterally offset from an adjacent longitudinal edge 226b of an adjacent linear segment 218. The width W2 of each intermediate segment 220 and end portion 224 can be greater than the width W1 of the linear segments 218.

In alternative embodiments, the width W1 of each linear segment 218 can vary along the length of a strut. For example, the width W1 of a linear segment 218 adjacent the inflow end of the frame can be greater than the width W1 of a linear segment 218 adjacent the outflow end of the frame, or vice versa. Further, where the width W1 of the linear segments 218 vary along the length of a strut 200, a linear segment can have one longitudinal edge 226a or 226b that is collinear with a longitudinal edge of an adjacent linear segment on the same side of the strut, while the other longitudinal edge 226a, 226b is laterally offset from the longitudinal edge of an adjacent linear strut on the same side of the strut. In other words, the strut 200 can have an overall zig-zag or offset pattern by virtue of the varying widths W1 of the linear segments.

FIG. 6 illustrates a delivery apparatus 300, according to one embodiment, adapted to deliver a prosthetic heart valve, such as the illustrated prosthetic heart valve 10, described above. The prosthetic valve 10 can be releasably coupled to the delivery apparatus 300. It should be understood that the delivery apparatus 300 and other delivery apparatuses disclosed herein can be used to implant prosthetic devices other than prosthetic valves, such as stents or grafts.

The delivery apparatus 300 in the illustrated embodiment generally includes a handle 302, a first elongated shaft 304 (which comprises an outer shaft in the illustrated embodiment) extending distally from the handle 302, at least one actuator assembly 306 (e.g., three in the illustrated embodiment) extending distally through the outer shaft 304. The at least one actuator assembly 306 can be configured to radially expand and/or radially collapse the prosthetic valve 10 when actuated. Though the illustrated embodiment shows three actuator assemblies 306, it should be understood that more or fewer actuator assemblies can be present. In some embodiments, a distal end portion 316 of the shaft 304 can be sized to house the prosthetic valve in its radially compressed, delivery state during delivery of the prosthetic valve through the patient's vasculature. In this manner, the distal end portion 316 functions as a delivery sheath or capsule for the prosthetic valve during delivery,

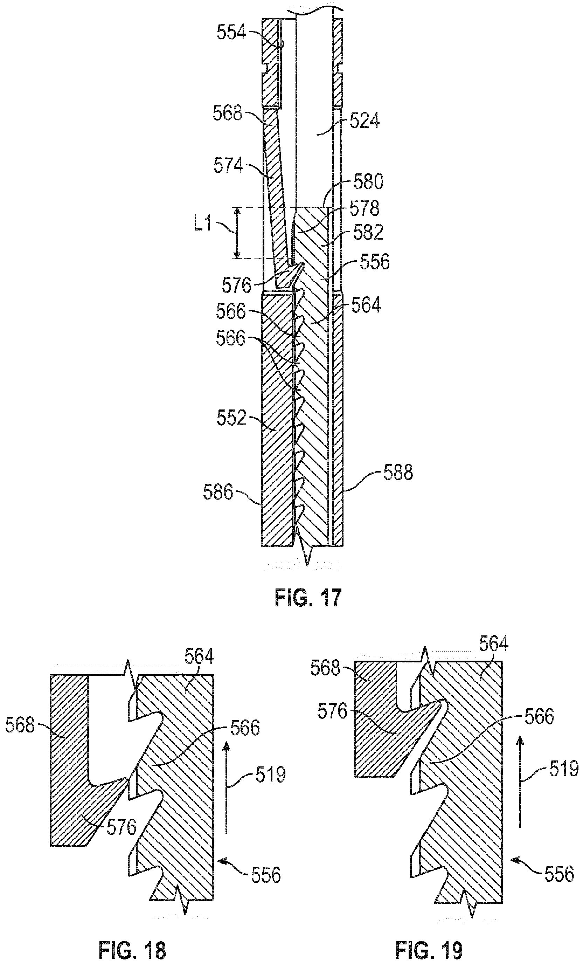

The actuator assemblies 306 can be releasably coupled to the prosthetic valve 10. For example, in the illustrated embodiment, each actuator assembly 306 can be coupled to a respective actuator 20 of the prosthetic valve 10. Each actuator assembly 306 can comprise a first actuation member 308 and a second actuation member 309 (FIGS. 1 and 17) extending through the first actuation member 308. When actuated, the first and second actuation members 308, 309 can move axially relative to one another to transmit pushing and/or pulling forces to portions of the prosthetic valve. The actuator assemblies 306 can be at least partially disposed radially within, and extend axially through, one or more lumens of the outer shaft 304. For example, the actuator assemblies 306 can extend through a central lumen of the shaft 304 or through separate respective lumens formed in the shaft 304. In the illustrated embodiment, the second actuation member (not shown) extends through the first actuation member 308. However, in other embodiments, the first and second actuation members may be spaced apart from each other circumferentially around the prosthetic valve 10.

The first actuation member 308 can be, for example, a sleeve, cylinder, shaft, tube, or other member configured to apply a distally directed forced to the prosthetic valve. The second actuation member 309 (FIGS. 1 and 17) can comprise an elongated actuator member in the form of, for example, a rod, shaft, cable, wire, suture, or other member configured to apply a proximally directed force to the prosthetic valve.

As mentioned above, each actuator 20 of the prosthetic valve 10 can generally comprise an inner member 34 and an outer member 36 disposed about the inner member 34. The inner members 34 and the outer members 36 can be movable longitudinally relative to each other in a telescoping manner to radially expand and contract the frame 12. In some embodiments, each first actuation member 308 of the delivery apparatus 300 can be releasably coupled to a respective outer member 36 of the actuator 20, and each second actuation member can be releasably coupled to a respective inner member 34 of the actuator 20. A user can actuate the actuator assemblies (e.g., using knob 312 as described below) thereby causing axial movement of the first actuation member 308 relative to the second actuation member. Movement of the actuator assemblies 306 can result in corresponding movement of the actuators 20 to radially expand and/or collapse the frame 12. Once the prosthetic valve 10 is fully expanded, it can be locked into position using one or more locking mechanisms and/or locking features, as described in more detail below.

The handle 302 of the delivery apparatus 300 can include one or more control mechanisms (e.g., knobs or other actuating mechanisms) for controlling different components of the delivery apparatus 300 in order to expand and/or deploy the prosthetic valve 10. For example, in the illustrated embodiment the handle 302 comprises first, second, and third knobs 310, 312, and 314.

The first knob 310 can be a rotatable knob configured to produce axial movement of the outer shaft 304 relative to the prosthetic valve 10 in the distal and/or proximal directions in order to deploy the prosthetic valve from the delivery sheath 316 once the prosthetic valve has been advanced to a location at or adjacent the desired implantation location with the patient's body. For example, rotation of the first knob 310 in a first direction (e.g., clockwise) can retract the outer sheath 304 proximally relative to the prosthetic valve 10 and rotation of the first knob 310 in a second direction (e.g., counter-clockwise) can advance the outer sheath 304 distally. In other embodiments, the first knob 310 can be actuated by sliding or moving the knob 310 axially, such as pulling and/or pushing the knob. In other embodiments, actuation of the first knob 310 (rotation or sliding movement of the knob 310) can produce axial movement of the actuator assemblies 306 (and therefore the prosthetic valve) relative to the delivery sheath 316.

The second knob 312 can be a rotatable knob configured to produce radial expansion and/or contraction of the prosthetic valve 10. For example, rotation of the second knob 312 can move the first and second actuation members 308, 309 axially relative to one another. Rotation of the second knob 312 in a first direction (e.g., clockwise) can radially expand the prosthetic valve 10 and rotation of the second knob 312 in a second direction (e.g., counter-clockwise) can radially collapse the prosthetic valve 10. In other embodiments, the second knob 312 can be actuated by sliding or moving the knob 312 axially, such as pulling and/or pushing the knob.

The third knob 314 can be a rotatable knob configured to release the prosthetic heart valve 10 from the delivery apparatus 300. For example, rotation of the third knob in a first direction (e.g., clockwise) can disengage the actuator assemblies 306 from the actuators 20 of the prosthetic valve 10. In other embodiments, the third knob 314 can be actuated by sliding or moving the third knob 314 axially, such as pulling and/or pushing the knob.

FIGS. 7-13 illustrate a representative embodiment of a prosthetic valve 400 comprising a frame 401. The prosthetic valve 400 can include a valvular structure (e.g., valvular structure 18), inner and/or outer skirts, and actuators (e.g., actuators 20) as previously described, although these components are omitted for purposes of illustration. The frame 401 can comprise a plurality of interconnected struts 402 which extend from the inflow end to the outflow end of the frame. Referring now to FIG. 7, the plurality of struts 402 can comprise radially disposed outer or first struts 402a and radially disposed inner or second struts 402b. Each strut 402 can comprise one or more locking features 404. Locking features 404 can be used in lieu of or in addition to locking mechanisms 40, described above.

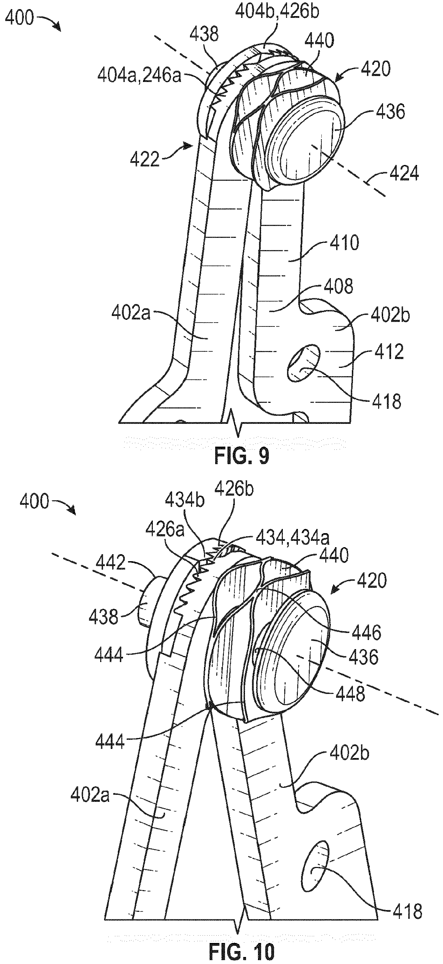

The outer struts 402a can comprise one or more locking features 404a disposed on a radially facing inner surface 406 of each respective outer strut 402a. The inner struts 402b can comprise one or more locking features 404b disposed on a radially facing outer surface 408 (see FIG. 9) of each respective inner strut 402b. Each locking feature 404a on a struts 402a is paired with and engages a locking feature 404b on a strut 402b. Each pair of locking features can be movable from an unlocked or disengaged position to an engaged position. When in the disengaged position the first and second struts 402a, 402b can pivot relative to one another in a first direction (e.g., away from one another) and/or in a second direction opposite the first direction (e.g., toward one another). When in the engaged position the first and second struts 402a, 402b can pivot relative to one another in the first direction (e.g., away from one another) but are prevented from pivoting relative to each other in the second direction (e.g., toward one another). In other words, when in the engaged position, the locking features 404a, 404b allow for radial expansion of the frame 401 and resist radially compression of the frame 401.

Similar to struts 200 described above, each strut 402 can comprise a plurality of linear portions or segments 410 joined end-to-end relative to each other with adjacent ends interconnected by intermediate segments 412. The strut 402 can also have enlarged end portions 414 at either end of the strut 402 that form apices 416 at the inflow and outflow ends of the frame. Each of the intermediate segments 412 and end portions 414 can have a respective aperture 418 desirably at its geometric center for receiving a fastener, such as fastener 420 described below.

The inner and outer struts 402a, 402b can be pivotably coupled to one another at one or more pivot joints along the length of each strut. Respective hinges or junctions 422 can be formed at the locations where struts 402 overlap each other (including at apices 416) via fasteners 420 that extend through the apertures 418. The hinges 422 can allow the struts 402 to pivot relative to one another about a pivot axis (e.g., pivot axis 424) as the frame 401 is radially expanded or compressed, such as during assembly, preparation, or implantation of the prosthetic valve 10.

The one or more locking features 404 can be disposed, for example, at one or more of the end portions 414 of each strut 402 and/or at one or more of the intermediate segments 412. For example, in the illustrated embodiment, each strut 402a, 402b comprises a single locking feature 404a and 404b, respectively, disposed at a first end portion 414a, 414b of the strut. In other embodiments, a strut 402 can comprise a respective locking feature 404 at each intermediate segment 412 and/or at each end portion 414. In still other embodiments, a strut 402 can comprise locking features 404 at alternating intermediate segments 412 including or not including the end portions 414 and/or including only one of the end portions 414.

As shown in the illustrated embodiment, each locking feature 404 can be disposed at least partially circumferentially around a portion of a respective aperture 418 such that the locking feature 404 has an arcuate shape. In other embodiments, the locking feature can be disposed around the entire circumference of an aperture, such that the locking feature has a circular shape. In the illustrated embodiment, the locking features 404 have a width W extending from the aperture 418 to the outer edge of the end portion 414 in a radial direction of the pivot joint. However, in other embodiments, the width of the locking features 404 can extend only partially from the aperture 418 to the outer edge of the end portion 414.

As the first and second struts 402a, 402b pivot about the pivot axis 424, the first and second locking features 404a, 404b rotate about the fastener 420. As mentioned previously, when the frame 401 is in the radially compressed configuration, the first and second locking features 404a, 404b can be in the disengaged position, and when the frame 401 is in the expanded or partially expanded configuration, the first and second locking features 404a, 404b can be in the engaged position. When in the disengaged position, the first and second locking features 404a, 404b are rotationally offset from one another, and when in the engaged position the first and second locking features are rotationally aligned with one another.

As used herein, the term "rotationally offset" means that the first locking feature 404a is in a rotational position relative to the second locking feature 404b such that the first and second locking features 404a, 404b do not overlap one another (see FIG. 9). The term "rotationally aligned" means that the first locking feature 404a is in a rotational position relative to the second locking feature 404b wherein at least a portion of the first locking feature 404a overlaps at least a portion of the second locking feature 404b (see FIGS. 10-13).

The locking features 404 can be formed integrally with each strut 402. That is, the strut 402 and the locking features 404 can be machined or otherwise formed from a single piece of material. For example, the locking feature 404 can be cut into a surface of the strut 402. Alternatively, the locking feature 404 can be formed separately and joined later in the fabrication process, such as by fasteners (e.g., screws), welding, or adhesives. In such embodiments, the strut 402 can comprise a recessed portion in which the locking feature can be disposed.

As shown in FIG. 7, each locking feature 404 comprises a toothed portion 426 including one or more teeth 428. Referring now to FIG. 8, the toothed portion 426 can comprise a plurality of first surfaces 430 extending perpendicular to an inner and/or outer surface 406, 408 of the strut 402 and a plurality of second, angled or curved surfaces 432 extending at an angle α relative to the inner or outer surface 406, 408 of the strut 402. Each pair of surfaces (e.g., a first surface and a second surface) can define a tooth 428. The angle α can be, for example, less than 90 degrees. In some particular embodiments, the angle can be about 30 degrees. However, in other embodiments, the angle can be greater or less than 30 degrees. Greater angles can require more force to be applied to the frame 401 to move the frame 401 into the expanded configuration, and lesser angles can require less force to be applied to the frame 401 to move the frame into the expanded configuration. In still other embodiments, other geometries of interlocking toothed portions can be used.

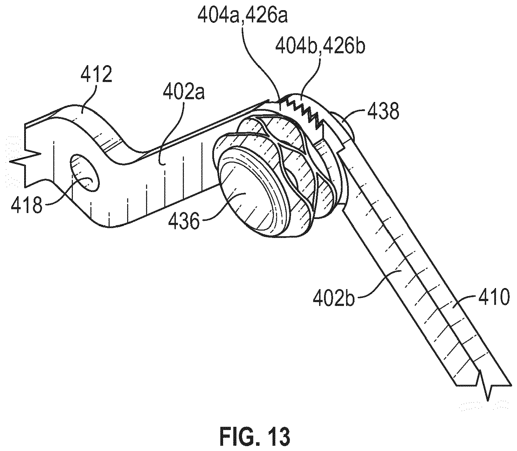

As shown in FIGS. 9-13, the frame 401 can move from the radially compressed configuration (FIG. 9) to the radially expanded configuration (FIG. 13) by pivoting the first and second struts 402a, 402b away from one another about the pivot axis 424 extending through junction 422.

As shown in FIG. 9, when in the radially compressed configuration, the first and second locking features 404a and 404b are in the disengaged position such the first and second toothed portions 426a, 426b, respectively, are rotationally offset from one another. Referring to FIG. 10, as the frame 401 expands (e.g., using the actuators 20 as described above) the first and second struts 402a, 402b pivot away from one another about pivot axis 424 and the first and second locking features 404a, 404b rotate toward one another about the fastener 420. The angled surface 432 of a first tooth 434a of the first toothed portion 426a can slide along the angled surface 432 of a first tooth 434b of the second toothed portion 426b until the first teeth 434a, 434b are engaged. The first surfaces 430 of the first teeth 434a, 434b prevent the struts 402a, 402b from pivoting toward one another and thereby radially collapsing the frame.

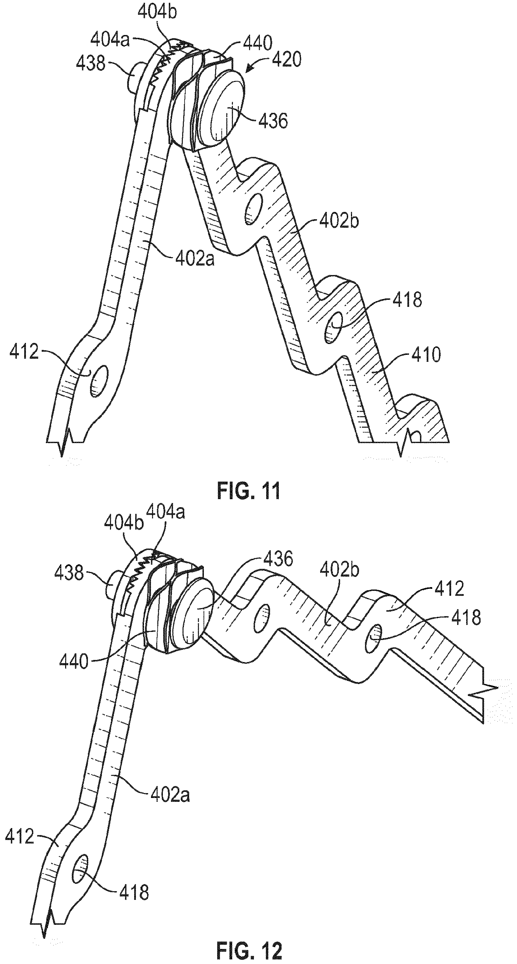

Once the frame 401 is expanded to a diameter in which the first teeth 434a, 434b are engaged, as shown in FIG. 11, the frame 401 is retained in a locked configuration, where the frame 401 can be further radially expanded but cannot be radially collapsed. In this manner, the first and second locking features 404a, 404b comprise a ratchet mechanism that permits pivoting movement of the struts 402a, 402b relative to each other for expanding the frame and resist pivoting movement of the struts 402a, 402b relative to each other to resist radial compression of the frame.

As shown in FIGS. 12-13, the teeth 428 of the first toothed portion 426a can engage successive teeth 428 of the second toothed portion 426b, with each tooth 428 representing a different degree of radial expansion of the prosthetic valve. When all teeth 428 of the first toothed portion 426a are engaged with all teeth 428 of the second toothed portion 426b, the locking features can be said to be in a fully engaged position, as shown in FIG. 13.

In some embodiments, the first and/or second locking features 404a, 404b can comprise one or more rotational stops configured to prevent expansion of the frame beyond a preselected diameter. The rotational stops can be, for example, rectangular blocks or teeth disposed at either or both ends of the locking feature 404.

As mentioned previously, the first struts 402a and second struts 402b can be coupled at one or more junctions 422 using one or more fasteners 420. As best shown in FIG. 10, each fastener 420 can comprise a head portion 436, a shaft 438, and a biasing member 440. At least a portion of the shaft 438 can extend through the apertures 418 in the first and second struts 402a, 402b. In some embodiments, an end cap (not shown) can be disposed over an end portion 442 of the shaft 438. The end cap can have a diameter greater than the diameter of the apertures 418 to prevent the shaft 438 from sliding through the apertures 418 and/or to prevent the shaft from moving axially relative to the first and second struts 402a, 402b.

The biasing member 440 is configured to bias the locking features 404 of the first and second struts 402a, 402b against each other. In the illustrated embodiment, the biasing member 440 is disposed between the head portion 436 and a radially facing outer surface of the first strut 402a. In other embodiments, the biasing member 440 can be disposed between the head portion 436 and a radially facing inner surface of the second strut 402b (e.g., in embodiments wherein the head portion 436 is oriented radially inwardly). In still other embodiments, the biasing member 440 can be disposed between the end cap and the radially facing inner surface of the first or second struts 402a, 402b.

As the frame 401 is expanded and the first and second locking features 404a, 404b move into the engaged position, the biasing member 440 can bias the first and second locking features 404a, 404b against one another to prevent or mitigate slippage between the locking features. As the angled surfaces 432 slide against one another the first and second struts 402a, 402b can move radially away from one another and the biasing member 440 can be compressed against the head portion 436. The compression of the biasing member 440 allows the first and second struts 402a, 402b to move radially away from one another while keeping the first and second locking features 404a, 404b engaged with one another.

As best shown in FIG. 10, in the illustrated embodiment, the biasing member 440 comprises a spring washer, which can have one or more curved layers 444 that can be coupled to each other at one or more coupling points 446. The layers 444 of the washer allow the washer to be compressed when the angled surfaces 432 of opposing teeth slide against each other during expansion of the frame and then expand the washer when the high point of an angled surface 432 of one tooth passes over the high point of an angled surface 432 of an opposing tooth to engage the low point of the next successive tooth to maintain contact between opposing toothed portions. The spring washer can be made of any of various suitable metals or polymeric materials. The biasing member 440 can comprise a central lumen 448 through which the shaft 438 extends. In other embodiments, the biasing member can have various other configurations, such as a solid elastomeric washer or metal coil.

The prosthetic valve 400 including frame 401 can be expanded in the following exemplary manner. Generally, a distal end portion of the delivery apparatus 300 (along with prosthetic valve 400) can be advanced through the vasculature of a patient to a selected implantation site (e.g., the native aortic annulus) as previously described. The prosthetic valve 400 can then be deployed at the implantation site as previously described and locked in the expanded configuration using the first and second locking features 404a, 404b.

In a particular example, the actuators (e.g., actuators 20) of prosthetic valve 400 can include push-pull mechanisms 32 comprising inner and outer members 34, 36, as described above with reference to FIG. 1. Once the prosthetic valve is at the selected implantation site, a user can actuate the actuator assemblies 306 of the delivery apparatus 300 (e.g., using the second knob 312) to transmit pushing and/or pulling forces from the handle of the delivery apparatus to the push-pull mechanisms 32 of the prosthetic valve.

For example, a distal end portion of a first actuation member 308 can engage or abut a corresponding outer member 36, and a distal end portion of the second actuation member 309 (FIGS. 1 and 17) can be coupled to a corresponding inner member 34. In this way, the delivery apparatus 300 can apply a distally directed force to the outer members 36 and/or apply a proximally directed force to the inner members 34 to move the prosthetic valve from the radially compressed configuration to the radially expanded configuration. As the prosthetic valve expands, the struts 402a, 402b pivot relative to one another such that the locking features 404a, 404b become rotationally aligned and therefore engage one another. The locking features 404a, 404b can continue to move relative to one another in a first direction, allowing further expansion of the prosthetic valve and can be restrained from moving relative to one another in a second, opposing direction, thereby preventing the prosthetic valve from being radially compressed. In alternative embodiments, the actuator assemblies of the delivery apparatus 300 can be transfer rotational forces from the handle 302 to actuators 20 of the prosthetic valve, with the actuators 20 converting the rotational forces into axial directed forces that expand the prosthetic valve.

FIGS. 14-25 illustrate an exemplary embodiment of a prosthetic valve 500 comprising a frame 502 and one or more expansion and locking mechanisms 550. The frame 502 comprises a plurality of pivotably connected struts 504 defining an inflow end 506 (which is the distal end of the frame in the delivery configuration for the illustrated embodiment) and an outflow end 508 (which is the proximal end of the frame in the delivery configuration for the illustrated embodiment). The struts 504 are pivotably connected to each other at a plurality of junctions that permit pivoting of the struts relative to each other when the frame 502 is radially compressed and expanded, as described above in connection with prosthetic valves 10 and 100.

The prosthetic valve 500 can include a valvular structure (e.g., valvular structure 18) and inner and/or outer skirts, as previously described, although these components are omitted for purposes of illustration. The one or more expansion and locking mechanisms 550 can be used in lieu of or in addition to actuators 20 and/or locking features 404 described above. The expansion and locking mechanisms 550 can be used to both radially expand and lock the frame 502 of prosthetic valve 500 in a radially expanded state.

FIG. 14 shows three expansion and locking mechanisms 550 mounted to the frame 502 with the frame 502 shown in the radially expanded configuration. Though the illustrated embodiment shows three expansion and locking mechanisms 550 spaced apart from each other about the circumference of the frame, it should be noted that a prosthetic valve can comprise any number of expansion and locking mechanisms 550. For example, in some embodiments, a prosthetic valve can comprise a single expansion and locking mechanism, or two expansion and locking mechanisms, or four expansion and locking mechanisms, etc. The expansion and locking mechanisms 550 can be placed at any position about the circumference of the frame 502. For example, in some embodiments such as the illustrated embodiment, the expansion and locking mechanisms 550 are equally spaced from one another about the circumference of the frame 502. In other embodiments, it can be advantageous to have two or more expansion and locking mechanisms situated adjacent to one another.

Each expansion and locking mechanism 550 can include an outer member in the form of a sleeve 552 having an inner lumen, cavity, or bore 554 (FIG. 17) and an inner member 556 extending at least partially into the cavity 554. The sleeve 552 in the illustrated embodiment comprises an inner wall 586, an outer wall 588 (FIG. 17), and two side walls 590, each of which extends radially between a longitudinal edge of the inner wall 586 and an opposing longitudinal edge of the outer wall 588. The inner wall 586, the outer wall 588, and the two side walls 590 define the cavity 554, which is sized and shaped to receive the inner member 556.

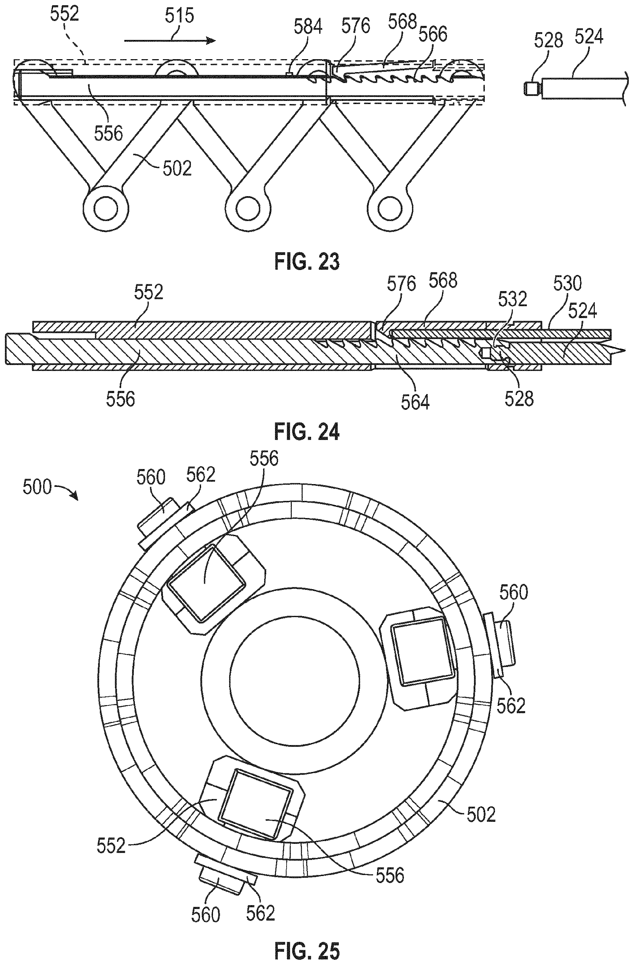

The sleeve 552 in the illustrated embodiment has a rectangular shape in cross-section and the inner member 556 has a rectangular shape in cross-section corresponding to the shape of the bore 554. In other embodiments, the sleeve 552 and/or the inner member 556 can have a square cross-sectional profile. As shown in FIG. 25, the rectangular and/or square cross-sections can advantageously minimize the distance that the expansion and locking members extend into the lumen of the frame 502, which can reduce the overall crimp profile of the valve 500. However, in other embodiments, the sleeve and the inner member can have any of various corresponding shapes in cross-section, for example, circular, ovular, triangular, rectangular, square, or combinations thereof.

As best shown in FIG. 14, a distal end portion 558 of the inner member 556 can be coupled to the frame 502 at a first location via a fastener 560 that is affixed to and extends radially from the distal end portion 558 of the inner member 556. The fastener 560 can be for example, a rivet or pin. As shown, in some embodiments, the fastener 560 can extend through corresponding apertures at a junction of two overlapping struts 504 of the frame 502 and can serve as a pivot pin around which the two struts 504 can pivot relative to each other and the inner member 556. In some embodiments, an end cap or nut 562 (see e.g., FIG. 25) can be disposed over an end portion of the fastener 560. The nut 562 can have a diameter greater than the diameter of the apertures to retain the fastener 560 within the apertures. In alternative embodiments, the inner member 556 need not comprise a fastener 560 and can be coupled to the frame 502 via other means of attachment such as welding, adhesives, etc.

The sleeve 552 can be coupled to the frame 502 at a second location, axially spaced from the first location. For example, in the illustrated embodiment, the inner member 556 is secured to the frame 502 near the distal or inflow end 506 of the frame and the sleeve 552 is secured to the frame 502 closer to or at the proximal or outflow end 508 of the frame, such as via a fastener 561 (e.g., a rivet or pint). The fastener 561 is affixed to and extends radially from the sleeve 552 through corresponding apertures at a junction of two overlapping struts 504 and can serve as a pivot pin around which the two struts 504 can pivot relative to each other and the sleeve 552. A nut 562 can be mounted on each fastener 561 to retain the fastener within the corresponding apertures. As discussed above in connection with the actuators 20 of the prosthetic valve 10 of FIG. 1, the expansion and locking mechanism 550 can be pivotably coupled to the frame 502 at any two axially spaced, circumferentially aligned locations on the frame.

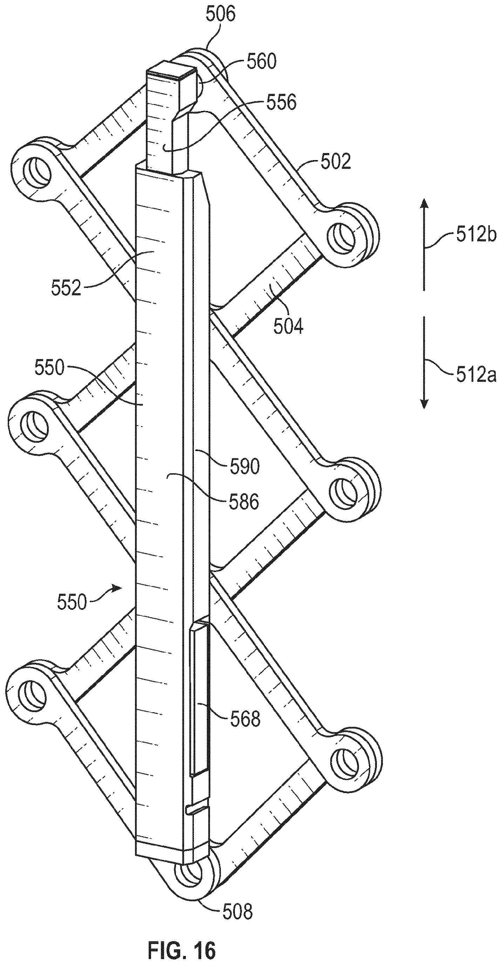

As shown in FIG. 16, the inner member 556 can be axially movable relative to the sleeve 552 in a proximal direction, as shown by arrow 512a, and in a distal direction, as shown by arrow 512b. As such, because the inner member 556 and the sleeve 552 are secured to the frame at axially spaced locations, moving the inner member 556 and the sleeve 552 axially with respect to one another in a telescoping manner can cause radial expansion or compression of the frame 502. For example, moving the inner member 556 proximally toward the outflow end 508 of the frame, as shown by arrow 512a, while holding the sleeve 552 in a fixed position and/or moving the sleeve 552 distally toward the inflow end 506 of the frame can cause the frame 502 to foreshorten axially and expand radially. Conversely, moving the inner member 556 distally in the direction of arrow 512b and/or moving the sleeve 552 proximally causes the frame 502 to elongate axially and compress radially.

Referring now to FIG. 17, the expansion and locking mechanism 550 can comprise a rachet mechanism or rachet assembly, wherein the inner member 556 comprises a linear rack 564 having a plurality of teeth 566 and the sleeve 552 comprises a pawl 568 configured to engage the teeth 566 of the inner member 556. This configuration, wherein the pawl 568 and teeth 566 engage one another within the sleeve 552 can help mitigate the risk of damage to the soft components of the valve and/or soft tissue of the patient, because the interlocking and/or ratcheting components of the locking mechanism 550 are internal to the mechanism.

The pawl 568 and the teeth 566 are configured such that when the pawl 568 is engaged with the rack 564, the inner member 556 and the sleeve 552 can move relative to one another in a first axial direction, but are prevented from moving relative to one another in a second, opposite axial direction. For example, in the illustrated embodiment, when the pawl 568 is engaged with the rack 564, the inner member 556 can move axially in a proximal direction (e.g., up in the orientation shown in FIG. 17) but cannot move axially in a distal direction (e.g., down in the orientation shown in FIG. 17). This ensures that when the pawl 568 is engaged with the rack 564, the frame 502 can be radially expanded but cannot be radially compressed.

Once the prosthetic valve has been implanted within a selected implantation site within a patient, the patient's native anatomy (e.g., the native aortic annulus) may exert radial forces against the prosthetic valve that would tend to compress the frame 502. However, the engagement between the pawl 568 and the rack 564 prevents such forces from compressing the frame 502, thereby ensuring that the frame remains locked in the desired radially expanded state.

The inner member 556 can comprise an elongated member extending at least partially through the sleeve 552. In the illustrated embodiment, the sleeve 552 can comprise an opening 570 (FIG. 15) at a distal end portion 572 thereof such that the inner member 556 can extend through the sleeve and beyond the distal end portion 572 of the sleeve, as shown in FIG. 16. In some embodiments, the inner member 556 can be housed entirely within the sleeve 552 and the sleeve 552 can be closed at its distal end. In some embodiments, substantially the entire length of the inner member 556 can comprise teeth 566. In other embodiments, a portion of the inner member 556 near the outflow end 508 of the frame 502 can comprise teeth 566.

The pawl 568 can comprise an elongated body 574 terminating in a locking tooth 576 that can engage the teeth 566 of the linear rack 564. As shown in FIGS. 18-19, the tooth 576 can have a shape that is complimentary to the shape of the teeth 566, such that the tooth 576 allows sliding movement of the inner member 556 in one direction relative to the pawl 568 (upward in the illustrated embodiment, as shown by arrow 519) and resists sliding movement of the inner member 556 in the opposite direction (downward in the illustrated embodiment) when the tooth 576 is in engagement with one of the teeth 566 of the linear rack 564.

Referring again to FIG. 17, the body 574 of the pawl 568 can be biased inwardly such that the tooth 576 of the pawl 568 is resiliently retained in a position engaging one of the teeth 566 of the inner member 556 (which can be referred to as the engaged position of the pawl 568). In the illustrated embodiment, the body 574 is configured as a leaf spring. In some embodiments, the body 574 can be integrally formed with the sleeve 552, in other embodiments, the body 574 can be separately formed and subsequently coupled to the sleeve 552. The biased configuration of the body 574 ensures that under normal operation, the tooth 576 of the pawl 568 stays engaged with the teeth 566 of the rack 564.

The inner member 556 can comprise a toothless portion 578 extending from a proximal edge 580 of the inner member 556 to the plurality of teeth 566. The toothless portion 578 can be a flat portion of the inner member 556, as shown. The toothless portion 578 is configured to allow bi-directional axial movement (in the distal and proximal directions) of the inner member 556 relative to the sleeve 552. This allows the frame 502 to expand and/or contract prior to the engagement of the pawl 568 with the plurality of teeth 566. The length L1 of the toothless portion 578 can be selected to provide a reversibility range in which the prosthetic valve can be freely expanded and compressed without locking.

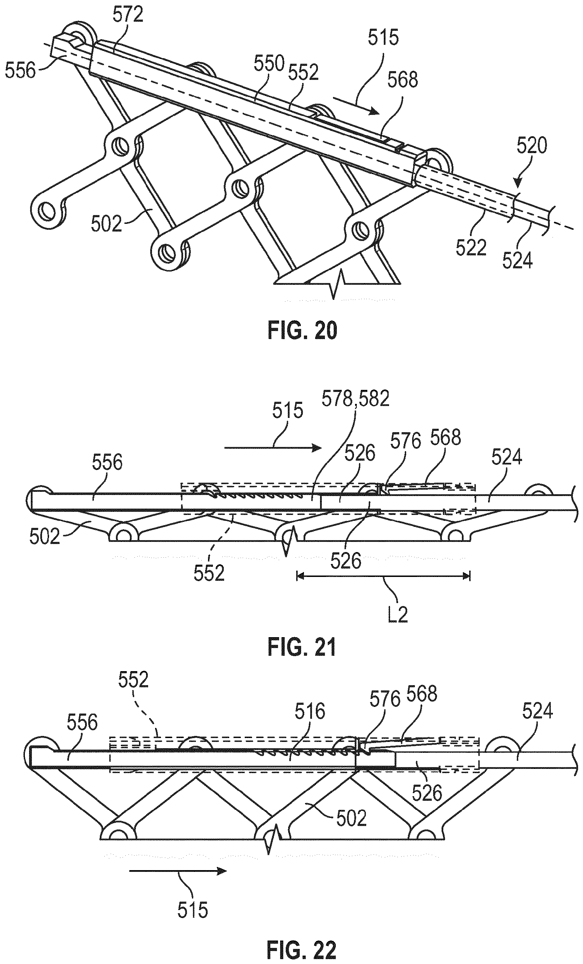

As best shown in FIG. 21, during delivery of the prosthetic valve 500, the inner member 556 can be coupled to an actuation member 524 of a delivery apparatus, as further described below. When so coupled to the actuation member 524, the toothless portion 578 of the inner member 556 and a distal end portion 526 of the actuation member 524 define a reversibility range having a length L2 that is greater than L1.

A prosthetic valve 500 including one or more expansion and locking mechanisms 550 can be expanded in the following exemplary manner. Generally, the prosthetic valve 500 is placed in a radially compressed state and releasably coupled to a distal end portion of a delivery apparatus, such as delivery apparatus 300 (FIG. 6), and then advanced through the vasculature of a patient to a selected implantation site (e.g., the native aortic annulus). The prosthetic valve 500 can then be deployed at the implantation site and expanded and locked in the expanded configuration using the expansion and locking mechanisms 550.

Each expansion and locking mechanism 550 can be releasably coupled to a respective actuation assembly 520 of a delivery apparatus, similar to actuation assemblies 306 of delivery apparatus 300. Referring now to FIG. 20, in a particular example, a distal end portion of a first actuation member 522 of the delivery apparatus can engage or abut the sleeve 552 of the expansion and locking mechanism 550 and a distal end portion 526 (FIG. 21) of a second actuation member 524 can be releasably coupled to a proximal end portion 582 of the inner member 556. The second actuation member 524 can extend co-axially through the first actuation member 522. The proximal end portions of the first and second actuation members can be operatively connected to a handle of the delivery apparatus. The delivery apparatus in this embodiment can include the same features described above for the delivery apparatus 300.

The distal end portion 526 of the second actuation member 524 can comprise an engagement member 528 (FIG. 23) configured to be releasably coupled to the inner member 556. For example, in some embodiments, the engagement member 528 can comprise an external threaded surface and the inner member 556 can comprise a bore 532 having a correspondingly threaded surface configured to threadably engage the engagement member 528. The correspondingly threaded surfaces can releasably secure the inner member 556 and the second actuation member 524 to one another.

In other embodiments, the engagement member 528 can have other configurations that permit the actuation member 524 to be releasably coupled to the inner member 556. For example, the engagement member 528 can be a magnet, and the inner member 556 can include a bore having a correspondingly magnetic material into which the engagement member can extend. The delivery apparatus can be used to apply a distally directed force to the sleeve 552 via the first actuation member 522 and/or a proximally directed force (as shown by arrow 515) to the inner member 556 via the second actuation member 524 to move the sleeve 552 and the inner member 556 axially relative to one another in a telescoping manner to cause the frame to radially expand. The distal end of the first actuation member 522 (also referred to as a support tube) can abut the sleeve 552.

Referring now to FIG. 21, when the frame 502 is in the radially compressed configuration, the inner member 556 can move relative to the sleeve 552 in the proximal and/or distal directions. As the inner member 556 moves, the tooth 576 of the pawl 568 can slide along the distal end portion 526 of the second actuation member 524 and/or the toothless portion 578 of the inner member 556 until it engages the plurality of teeth 566, as shown in FIG. 22. The engagement of the pawl 568 with the plurality of teeth 566 allows for continued radial expansion of the frame but prevents radial compression of the frame.

As shown in FIG. 23, the frame 502 can continue to be expanded by moving the inner member 556 proximally (as shown by arrow 515) until a selected prosthetic valve diameter is achieved. The selected diameter can correspond to a selected position of the tooth 576 of the pawl 568 in which it engages any tooth of the plurality of teeth 566. For example, in the illustrated embodiment, as shown in FIG. 23, the selected diameter corresponds with the position of the tooth 576 of the pawl 568 in which it engages the third tooth 566 from the distal end of the plurality of teeth 566. In other embodiments, the selected diameter can correspond to the position of the tooth 576 in which it engages a distal-most tooth 566 of the plurality of teeth 566. In some embodiments, an optional stopper 584 can be provided on the inner member 556 distal to the teeth 566. The stopper 584 can be positioned to engage the pawl 568 or another portion of the sleeve 552 to prevent further proximal movement of the inner member 556 relative to the sleeve 552 to prevent over expansion of the prosthetic valve. Once the selected diameter is reached, the first and second actuation members 522, 524 can be uncoupled from the expansion and locking mechanism 550 and removed from the patient's body.

Referring to FIG. 24, in some embodiments, the expansion and locking mechanism 550 can receive a retaining member 530. The retaining member 530 can be configured to selectively retain the pawl 568 in the disengaged position, for example, the retaining member 530 can prevent the pawl 568 from contacting the plurality of teeth 566.

In the illustrated embodiment, the retaining member 530 can be an elongated member sized to be inserted within the sleeve 552 and between the pawl 568 and the teeth 566 of the inner member 556. When the retaining member 530 is inserted within the sleeve 552, the retaining member 530 prevents the pawl 568 from moving toward the inner member 556 and thereby prevents the engagement of the tooth 276 of the pawl 568 with the plurality of teeth 566. Accordingly, the inner member 556 can move relative to the sleeve 552 in the proximal and/or distal directions, allowing for unrestricted radial expansion and radial compression of the prosthetic valve 500. The retaining member 530 can be removed from the expansion and locking mechanism 550 by, for example, moving the retaining member in a proximal direction until the retaining member 530 is no longer disposed between the pawl 568 and the inner member 556. Once the retaining member 530 is removed, the pawl 568 can engage the plurality of teeth 566 of the inner member 556 and lock the prosthetic valve 500 at a selected diameter.

In particular embodiments, the retaining member 530 is used to unlock the prosthetic valve 500 from an expanded configuration during assembly of the prosthetic valve and/or during a loading procedure. Typically, the components of the prosthetic valve 500 (the frame 502, the leaflets and other soft components) are assembled while the frame is in a locked, expanded configuration in which the tooth 576 of the pawl engages a tooth 566 of the rack 564. Following assembly, the retaining member 530 can be inserted into the expansion and locking mechanism 550 to push the tooth 576 of the pawl 568 out of engagement with the tooth 566 and prevent engagement of the tooth 576 with any of the other teeth 566 of the rack 564. This allows the inner member 556 to freely slide relative to sleeve 552, permitting radial compression of the prosthetic valve for subsequent loading of the prosthetic valve into or on the delivery apparatus.

In some embodiments, the prosthetic valve 500 can be provided to the end user (e.g., in a sterile package) in a locked, expanded configuration, and the end user can use the retaining member 530 to unlock the expansion and locking mechanism 550, radially compress the prosthetic valve, and load the prosthetic valve in or on the delivery apparatus (e.g., place the radially compressed prosthetic valve within a sheath of the delivery apparatus).

In some embodiments, the retaining member 530 can be a component of the delivery apparatus and can disposed between the inner member 556 and the pawl 568 during the implantation procedure. For example, each actuation assembly 520 of the delivery apparatus can include a respective retaining member 530 that extends from a corresponding expansion and locking mechanism 550 to a handle of the delivery apparatus. After delivering the prosthetic valve to a location at or adjacent the implantation site, the user can freely adjust the diameter of the prosthetic valve, including radially expanding and compressing the prosthetic valve. Once a selected expanded diameter for the prosthetic valve is achieved, the user can remove each retaining member 530 from its corresponding expansion and locking mechanism 550 in order to lock the prosthetic valve at the selected expanded diameter.

In some implementations, the retaining member 530 can be used to selectively disengage the pawl 568 from the plurality of teeth 566 after the locking mechanism 550 has been engaged to lock the prosthetic valve 500 in an expanded diameter during an implantation procedure. For example, the retaining member 530 can be advanced distally into the sleeve 552 to disengage the pawl 568 from the plurality of teeth 566 to permit radial compression of the frame if repositioning or recapture and removal of the prosthetic valve is desired.

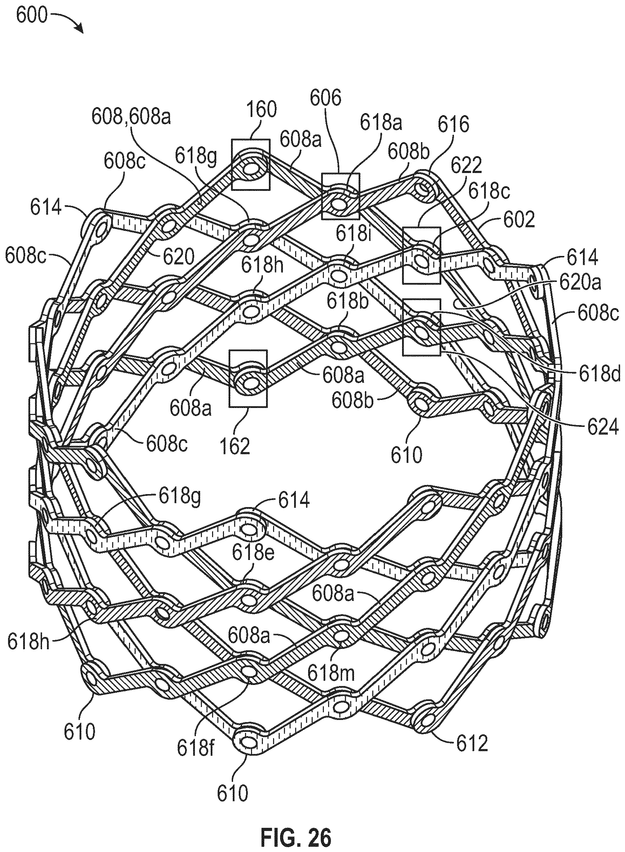

FIGS. 26-28 illustrate another exemplary embodiment of a prosthetic valve 600 comprising frame 602. The prosthetic valve 600 can include a valvular structure (e.g., valvular structure 18) and inner and/or outer skirts, as previously described, although these components are omitted for purposes of illustration.

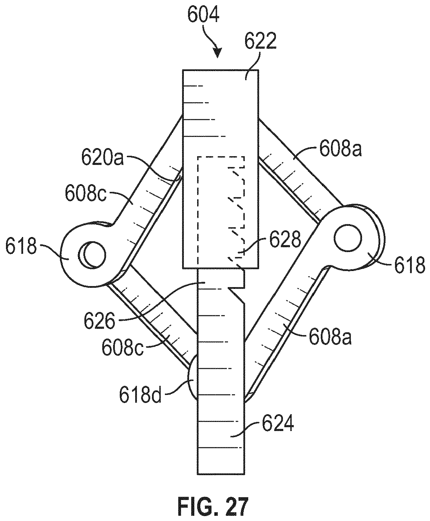

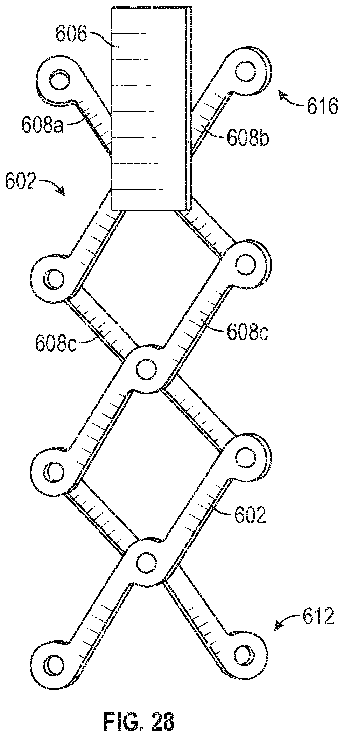

The frame 602 can be coupled to one or more expansion mechanisms (such as one or more actuator assemblies 150 shown in FIG. 2), one or more locking mechanisms 604 (FIG. 27), and/or one or more commissure attachment posts 606 (FIG. 28). In some embodiments, such as the embodiment shown in FIG. 1, the expansion mechanism, locking mechanism, and commissure attachment post can all comprise a single structural element (e.g., actuator 20). However, in the embodiment of FIGS. 26-28, each component (e.g., the expansion mechanism, the locking mechanism, and the commissure attachment post) can be separate structural elements that can be coupled to the frame 602 at spaced apart locations. This configuration can advantageously allow each component to be attached to a selected region of the frame best suited for the particular component and can allow a different number of each component to be used. For example, in some embodiments it may be advantageous to provide three commissure attachment posts (corresponding to three leaflets), two expansion mechanisms, and two locking mechanisms.

The frame 602 can include a plurality of interconnected struts 608 arranged in a lattice-type pattern and forming a plurality of distal apices 610 at the inflow end 612 of the frame 602 and a plurality of proximal apices 614 at the outflow end 616 of the frame. Each strut 608 can be coupled to one or more other struts 608 at a plurality of junctions 618 forming a plurality of cells 620. Each distal and proximal apex 610, 614 is also a junction 618.

Referring to FIG. 26, the struts 608 are arranged in different sets of struts, namely, a first set of first struts 608a, a second set of second struts 608b, and a third set of third struts 608c. For purposes of illustration, the first, second, and third struts 608a, 608b, 608c include different fill patterns in FIG. 26. In alternative embodiments, the frame can be formed from a greater or fewer number of sets of struts.

In the illustrated embodiment, each strut of a particular set is pivotably coupled to another strut of the same set at a distal apex 610, another strut of the same set at a proximal apex 614, and another strut of the same set at a junction 618 between the distal and proximal apices 610, 614 (desirably at a middle junction 618m at the midsections of the two overlapping struts equidistant from the distal and proximal apices). Thus, for example, each first strut 608a of the first set of struts is pivotably coupled to another first strut 608a of the first set at a distal apex 610, another first strut 608a of the first set at a proximal apex 614, and another first strut 608a of the first set at a middle junction 618m at the midsections of the two overlapping first struts wherein the middle junction 618m is equidistant from the distal and proximal apices.

Except where each strut 608a, 608b, 608c is pivotably coupled to a strut of the same set at a distal apex 610, a proximal apex 614, and a middle junction 618m, the strut can be pivotably coupled to a strut of a different set at junctions between the distal apex 610 and the middle junction 618m and at junctions between the proximal apex 614 and the middle junction 618m. For example, a first strut 608a can be pivotably coupled to a second strut 608b at a junction 618a, which is the junction along those struts closest to the proximal apices 614 at the outflow end 616. A first strut 608a can be pivotably coupled to a second strut 608b at a junction 618b, which is the junction along those struts closest to the distal apices 610 at the inflow end 612.

In alternative embodiments, struts of one set can be pivotably coupled to struts of another set at the distal apices 610, the proximal apices 614, and/or the middle junctions 618m.

The frame 602 can be coupled to one or more expansion mechanisms, such as one or more actuator assemblies 150 described above in connection with FIG. 2. For each actuator assembly 150, a pair of a stopper 160 and a sleeve 162 can be affixed to the frame 602 to releasably couple the actuator assembly to the frame. The stopper 160 and the sleeve 162 of a pair are affixed to axially spaced junctions of the frame. The optimum attachment locations for coupling the actuator assembly 150 to the frame are the opposite ends of the frame, as this helps distribute the expansion forces of the actuator assembly along the entire length of the frame. As such, the stopper 160 and the sleeve 162 of each pair desirably are affixed to junctions at a proximal apex 614 and a distal apex 610, respectively. For example, as shown schematically in FIG. 26, the stopper 160 is affixed to a junction at a proximal apex 614 formed by two first struts 608a and the sleeve 162 is affixed to a junction at a distal apex 610 formed by two first struts 608a. As described above in connection with FIG. 2, when an actuator member 152 is connected to the sleeve 162, the actuator assembly 150 can be used to apply a distally directed force to the stopper 160 and/or a proximally directed force to the sleeve 162 thereby causing the frame 602 (FIG. 26) to foreshorten axially and expand radially. The pusher member 156 applies a distally directed force to the proximal end of the frame 602 and the actuator member 152 applies a proximally directed force to the distal end of the frame 602. Applying these expansion forces to the opposite ends of the frame promotes distribution of the expansion forces along the entire length of the frame, thereby providing consistent and even expansion of the frame along its length. Once the frame 602 is expanded to a selected diameter, the actuator assembly 150 can be disengaged from the frame as previously described.

Referring to FIGS. 26-27, the prosthetic valve 600 can further comprise one or more locking mechanisms 604 coupled to the frame 602. Each locking mechanism 604 can comprise an outer member 622 such as in the form of a sleeve or housing and an inner member 624 comprising a linear rack 626 having a plurality of teeth 628. Though not pictured in the illustrated embodiment, the sleeve 622 can comprise a pawl, such as pawl 518 described above. The pawl and the teeth 628 are configured such that when the pawl is engaged with the rack 626, the inner member 624 and the sleeve 622 can move relative to one another in a first axial direction, but are prevented from moving relative to one another in a second, opposite axial direction. For example, when the pawl is engaged with the rack 626, the inner member 624 can move axially in a proximal direction (e.g., up in the orientation shown in FIG. 27) but cannot move axially in a distal direction (e.g., down in the orientation shown in FIG. 27). This ensures that while the pawl is engaged with the rack 626, the frame 602 can be radially expanded but cannot be radially compressed. In other words, the frame 602 is locked in the expanded configuration.

Each locking mechanism 604 can be coupled to a proximal junction and a distal junction of the same cell 620. For example, the sleeve 622 can be coupled to a proximal junction 618c (FIG. 26), and the inner member 624 can be coupled to a distal junction 618d of cell 620a. In certain embodiments, it is desirable that each of the proximal and distal junctions are formed by two struts 608 of different sets of struts. This configuration can advantageously prevent or mitigate buckling of the frame 602 under stress. For example, in the illustrated embodiment, the proximal junction 618c (FIG. 26) is formed by a first strut 608a and a third strut 608c, and the distal junction 618d is formed by a first strut 608a and a third strut 608c.

In other embodiments, referring to FIG. 26, the locking mechanism 604 can be connected to, for example, a proximal junction 618e (formed by a first strut 608a and a second strut 608b) and a distal junction 618f (formed by a first strut 608a and a second strut 608b). Alternatively, the locking mechanism 604 can be connected to a proximal junction 618g (formed by a second strut 608b and a third strut 608c) and a distal junction 618h (formed by a second strut 608b and a third strut 608c).

Moreover, a locking mechanism 604 can be connected to a pair of junctions along the same axial path on the frame as an actuator assembly 150, depending on the size and/or configuration of the actuator assembly, or to a pair of junctions that are circumferentially spaced from an actuator assembly. For example, a locking mechanism 604 can be connected to a pair of junctions 618g, 618h that are located along the same axial path as a pair of a stopper 160 and a sleeve 162, or a pair of junctions 618g, 618h that are circumferentially spaced from the closest pair of a stopper 160 and a sleeve 162.

Where more than one locking mechanism 604 is used, each locking mechanism 604 can be connected to the frame at similar junctions formed by struts of the same two sets. For example, each locking mechanism 604 can be connected to the frame at a respective pair of junctions 618c, 618d, or at a respective pair of junctions 618e, 618f, or at a respective pair of junctions 618g, 618h. In alternative embodiments, the locking mechanisms 604 can be connected to different pairs of junctions. For example, one locking mechanism 604 can be connected to the frame at a respective pair of junctions 618c, 618d, another locking mechanism 604 can be connected to the frame at a respective pair of junctions 618e, 618f, and another locking mechanism 604 can be connected to the frame at a respective pair of junctions 618g, 618h. In still other embodiments, some locking mechanisms 604 can be connected to similar junctions while other locking mechanism 604 can be connected to different junctions.

Unlike the embodiment of the expansion and locking mechanism of FIGS. 14-25, the locking mechanism 604 in the illustrated embodiment does not apply any expansion forces to the frame 602. Instead, the one or more actuator assemblies 105 (FIG. 2) are used to expand the frame 602, while one or more locking mechanisms 604 are used to lock the frame in the expanded configuration. As noted above, an actuator assembly 105 can be connected to the frame 602 at a distal apex 610 and at a proximal apex 614 (which are formed by struts of the same set), while a locking mechanism 604 can be connected to a pair of junctions, each of which is formed by struts of different sets. Thus, by separating the locking mechanism from the actuator assembly, these two devices can be connected to the frame at different locations that promote optimal performance of each device.

As mentioned above, the frame 602 can further comprise one or more commissure attachment posts 606 (also referred to as a commissure attachment member). Desirably, the frame includes one commissure attachment post 606 for each commissure of the leaflet assembly (e.g., leaflet assembly 18 of FIG. 1). For example, for a leaflet assembly having three leaflets and thus three commissures, the frame desirably has three commissure attachment posts 606.

FIG. 28 illustrates a portion of the frame 602 comprising a commissure attachment post 606. In the illustrated embodiment, the commissure attachment post 606 is configured as a rectangular member, however, in other embodiments, the commissure attachment post 606 can have any of various shapes. For example, in some particular embodiments, the commissure attachment post can be a cylindrical member. A commissure can be connected to a commissure attachment post 606 using various techniques or mechanisms. In some embodiments, a commissure is formed by a pair of leaflet tabs of adjacent leaflets and the leaflets tabs are connected directly to a post 606, such as by wrapping the leaflet tabs around the post and securing them in place with sutures or other types of fasteners.

In other embodiments, a commissure clamp, such as commissure clamp 26 described above, can be mounted on the commissure attachment post 606. The commissure clamp 26 can be configured to grip adjacent portions of two leaflets at each commissure at a location spaced radially inwardly of the frame 602.

Each commissure attachment post 606 can be coupled to the frame 602 at any convenient location, which can be dictated by a desired position of a commissure within the frame. For example, in the illustrated embodiment, each commissure attachment post 606 is connected to a junction 618a, which is the junction closest to the proximal apices 614 of the frame 602. In other embodiments, each commissure attachment post 606 can be coupled to a junction 618 at a proximal apex 614. The commissure attachment posts 606 can be connected at various other junctions or other locations on the struts (e.g., at locations along the struts between junctions), depending on the design and/or size of the leaflets. As such, it should be understood that the locations of the commissure attachment posts need not be dictated by the positions of the actuator assemblies and/or locking mechanism on the frame. Further, each commissure attachment post can be a relatively short structure compared to the locking mechanisms and actuator assemblies.

Forming each device (the expansion mechanism 150, the locking mechanism 604, and commissure attachment post 606) as a separate device allows each device to be spaced apart from each other about the frame. Accordingly, each device can be positioned at a location selected to allow for optimal performance of the device.

In other embodiments, one or more of the devices can be combined into a single device that performs multiple functions. For example, in some embodiments, the expansion mechanism and locking mechanism can be combined into single device (e.g., expansion and locking mechanism 550) and the commissure attachment post can be a discrete structural element.

In one specific implementation, a combination expansion and locking mechanism (e.g., mechanism 550) (which can be used as a commissure post or separate commissure posts can be provided) can connected to a junction 618a and a junction 618b, or alternatively, to a junction 618a, a junction 618b, and a junction 618i (located axially between junctions 618a and 618b).

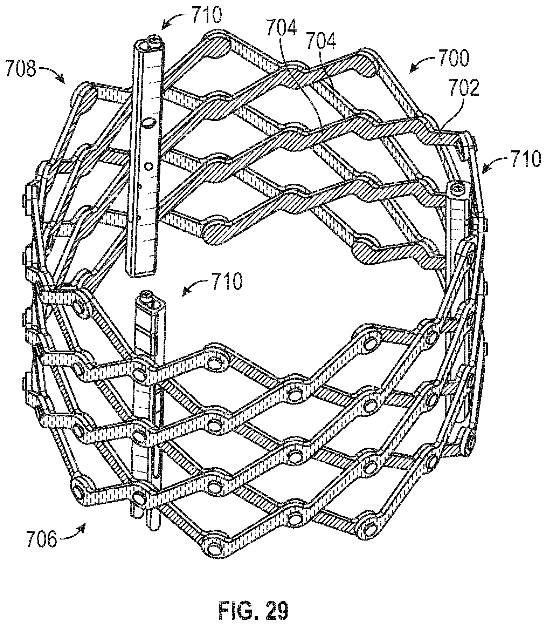

FIGS. 29-46 illustrate an exemplary embodiment of a prosthetic valve 700 comprising a frame 702 and one or more expansion and locking mechanisms 710. The frame 702 comprises a plurality of pivotably connected struts 704 defining an inflow end 706 (which is the distal end of the frame in the delivery configuration for the illustrated embodiment) and an outflow end 708 (which is the proximal end of the frame in the delivery configuration for the illustrated embodiment). The struts 704 are pivotably connected to each other at a plurality of junctions that permit pivoting of the struts relative to each other when the frame 702 is radially compressed and expanded, as described above in connection with prosthetic valves 10 and 100.

The prosthetic valve 700 can include a valvular structure (e.g., valvular structure 18) and inner and/or outer skirts, as previously described, although these components are omitted for purposes of illustration. The one or more expansion and locking mechanisms 710 can be used in lieu of or in addition to actuators 20, locking features 404, and/or expansion and locking mechanisms 550 described above. The expansion and locking mechanisms 710 can be used to both radially expand the frame 702 and lock the frame in a radially expanded state.

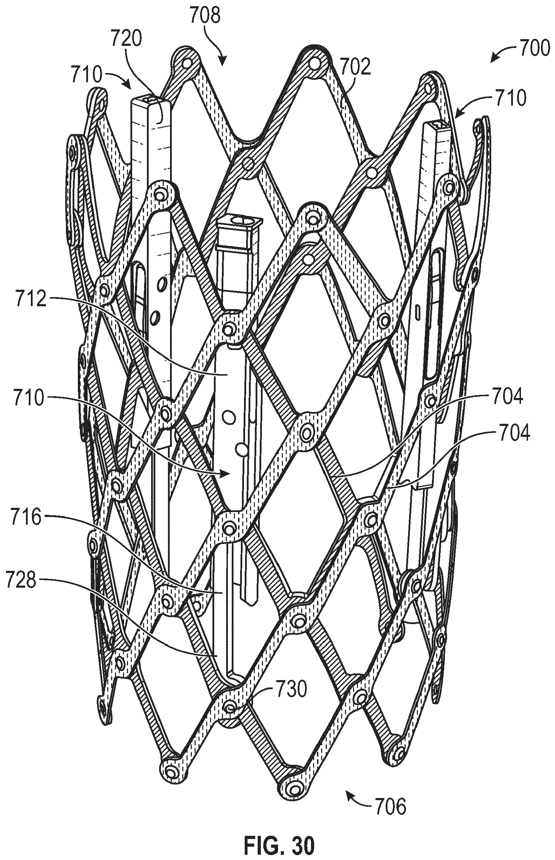

FIGS. 29-30 show three expansion and locking mechanisms 710 mounted to the frame 702. FIG. 29 shows the frame 702 in a radially expanded configuration, and FIG. 30 shows the frame 702 in a partially compressed configuration. Though the illustrated embodiment shows three expansion and locking mechanisms 710 spaced apart from each other about the circumference of the frame, it should be noted that a prosthetic valve can comprise any number of expansion and locking mechanisms 710. For example, in some embodiments, a prosthetic valve can comprise a single expansion and locking mechanism, or two expansion and locking mechanisms, or four expansion and locking mechanisms, etc. The expansion and locking mechanisms 710 can be placed at any position about the circumference of the frame 702 For example, in some embodiments such as the illustrated embodiment, the expansion and locking mechanisms 710 are equally spaced from one another about the circumference of the frame 702. In other embodiments, it can be advantageous to have two or more expansion and locking mechanisms situated adjacent to one another.

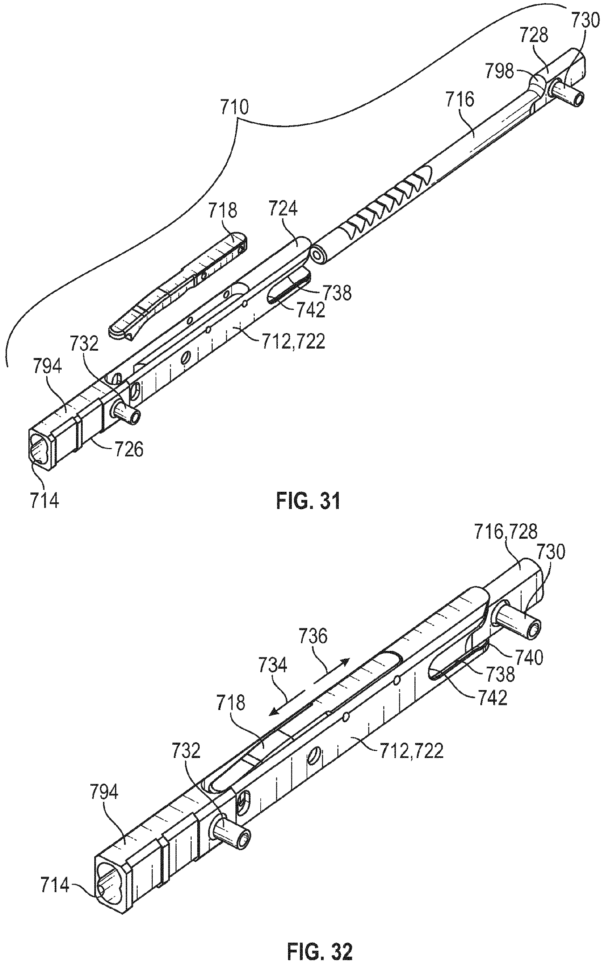

Referring to FIG. 31, each expansion and locking mechanism 710 can include a first or outer member 712 (also referred to as a sleeve) having an inner lumen, cavity, or bore 714, a second or inner member 716 extending at least partially into the cavity 714, and a third or locking member 718 coupled to the outer member 712. The outer member 712 in the illustrated embodiment comprises an inner wall 720 (see FIG. 29), an outer wall 722, and two side walls 724, 726 each of which extends radially between a longitudinal edge of the inner wall 720 and an opposing longitudinal edge of the outer wall 722. The inner wall 720, the outer wall 722, and the two side walls 724, 726 define the cavity 714, which is sized and shaped to receive the inner member 716.

As best shown in FIG. 30, a distal end portion 728 of the inner member 716 can be coupled to the frame 702 at a first location via a fastener 730 (FIG. 31) that is affixed to and extends radially from the distal end portion 728 of the inner member 716. The fastener can be, for example, a rivet or a pin. As shown, in some embodiments, the fastener 730 can extend through corresponding apertures at a junction of two overlapping struts 704 of frame 702 and can serve as a pivot pin around which the two struts 704 can pivot relative to one another and the inner member 716. In some embodiments, an end cap or nut can be disposed over and end portion of the fastener 730, such as nut 562 described above.

The outer member 712 can be coupled to the frame 702 at a second location, axially spaced from the first location. For example, in the illustrated embodiment, the inner member 716 is secured to the frame 702 near the distal or inflow end 706 of the frame and the outer member 712 is secured to the frame 702 closer to or at the proximal or outflow end 708 of the frame, such as via a fastener 732 (e.g., a rivet or pint). The fastener 732 is affixed to and extends radially from the outer member 712 through corresponding apertures at a junction of two overlapping struts 704 and can serve as a pivot pin around which the two struts 704 can pivot relative to each other and the outer member 712. A nut (such as nut 562 described previously) can be mounted on each fastener 732 to retain the fastener within the corresponding apertures. As discussed above in connection with the actuators 20 of the prosthetic valve 10 of FIG. 1, the expansion and locking mechanism 710 can be pivotably coupled to the frame 702 at any two axially spaced, circumferentially aligned locations on the frame.

Referring now to FIG. 32, the inner member 716 can be axially movable relative to the outer member 712 in a proximal direction, as shown by arrow 734, and in a distal direction, as shown by arrow 736. As such, because the inner member 716 and the outer member 712 are secured to the frame at axially spaced locations, moving the inner member 716 and the outer member 712 axially with respect to one another in a telescoping manner can cause radial expansion or compression of the frame 702. For example, moving the inner member 716 proximally toward the outflow end 708 of the frame, as shown by arrow 734, while holding the outer member 712 in a fixed position and/or moving the outer member 712 distally toward the inflow end 706 of the frame can cause the frame 702 to foreshorten axially and expand radially. Conversely, moving the inner member 716 distally in the direction of arrow 736 and/or moving the outer member 712 proximally causes the frame 702 to elongate axially and compress radially.

As shown in FIG. 32, outer member 712 can further comprise a recess 738 in the outer wall 722. The recess 738 can extend through a thickness of the outer wall 722 and can extend to the distal edge 740 of the outer wall. In the illustrated embodiment, the recess is substantially U-shaped, however, in other embodiments the recess can have any of various shapes. The recess 738 can be configured to limit the proximal advancement of the inner member 716 within the outer member 712. For example, as the prosthetic valve 700 expands, the inner member 716 can slide relative to the outer member 712 until the fastener 730 of the inner member 716 enters the recess 738. The inner member 716 can continue moving relative to the outer member 712 until the fastener 730 abuts a proximal edge 742 of the recess 738, restraining further motion of the inner member 716.

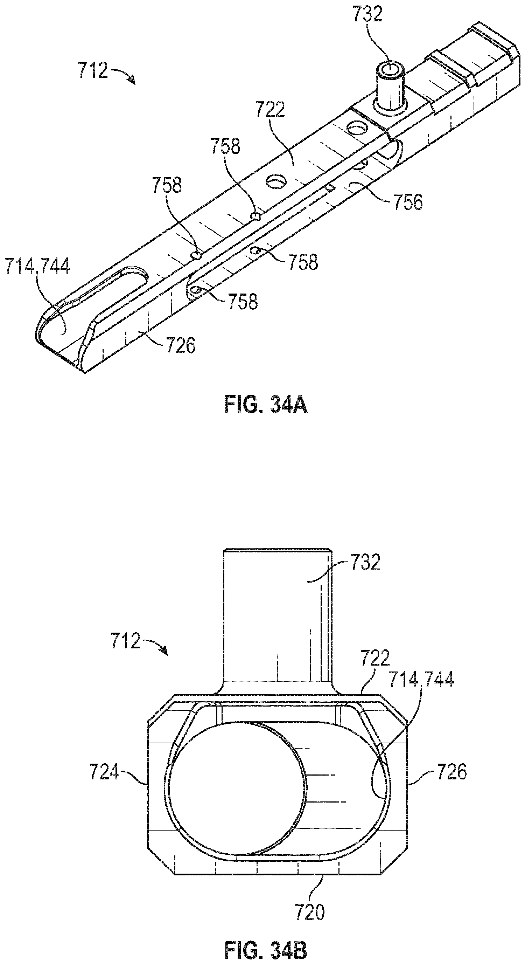

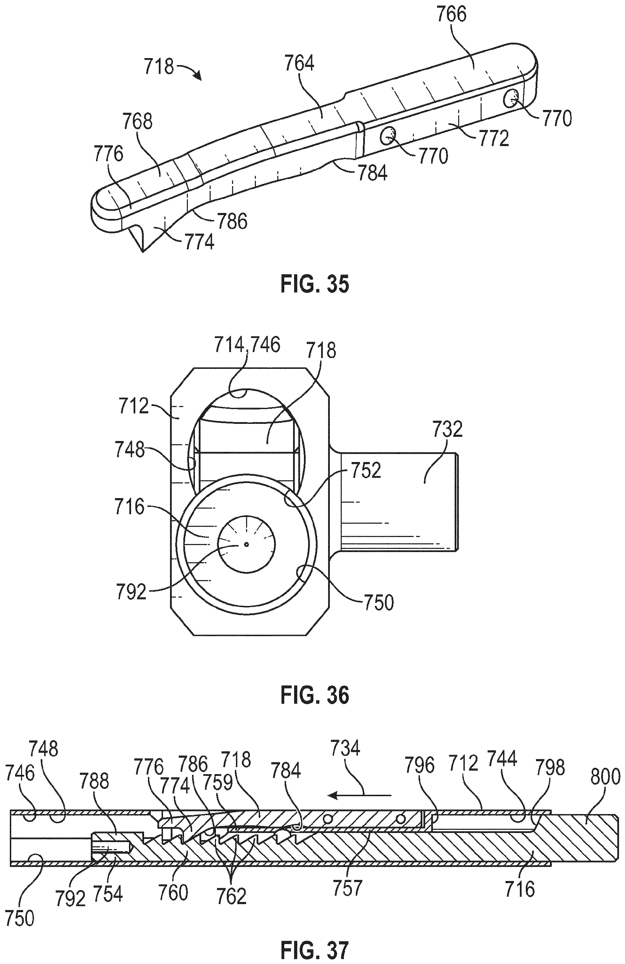

The outer member 712 in the illustrated embodiment has an outer profile that is rectangular shape in cross-section. The inner bore 714 can comprise a first, distal portion 744 (FIGS. 34A and 37) formed in a distal portion of the outer member 712 and a second, proximal portion 746 (FIGS. 36 and 37) formed in a proximal portion of the outer member. As best seen in FIG. 34B, the first portion 744 can have a substantially ovular shape with flat sides in cross section corresponding to the distal end portion 728 of the inner member 716. Referring now to FIG. 36, the second portion 746 of the bore 714 can comprise a first opening 748 and a second opening 750 separated by a neck portion 752. The neck portion 752 can have a thinner width than the first and second openings 748, 750. The first opening 748 can be configured to guide a disengagement member (such as disengagement member 802 described below) into the outer member 712 such that it can actuate the locking member 718, as described in more detail below. The second opening 750 can be configured to guide an actuation member of the delivery apparatus into position such that it can couple a proximal end portion 754 of the inner member 716 to actuate inner member 716, thereby radially expanding and/or collapsing the prosthetic valve 700. The second opening 750 can have a circular shape as shown. The first opening 748 can have a circular shape that intersects with the circular shape of the second opening at the neck portion 752. In other embodiments, the first and second openings can have oval shapes that intersect at the neck portion.

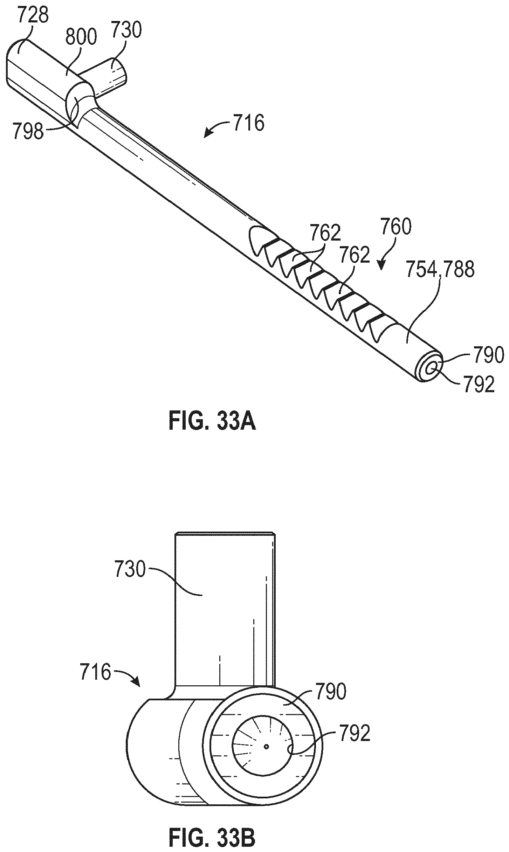

In some embodiments, the proximal end portion 754 of the inner member 716 can have a circular shape in cross-section (see e.g., FIG. 33B). In other embodiments, the proximal end portion 754 of the inner member 716 can have an oval shape in cross-section. In still other embodiments, the cross-section of the proximal end portion 754 of the inner member 716 can have any of various shapes (rectangular, square, triangular, square-oval, etc.) configured to correspond to the shape of the second opening 750 of the proximal portion 746 of the bore 714.

Referring again to FIG. 34A, the outer member 712 can further comprise an opening 756 in one of the side walls 724, 726 configured to receive the locking member 718. The opening 756 can extend through a thickness of the side wall 726. In the illustrated embodiment, the opening 756 is disposed in side wall 726. However, in other embodiments, the opening can be disposed in any of the other walls. The opening 756 can have an elongated oval shape with flat sides corresponding to the shape of the outer perimeter of the locking member 718. In other embodiments, the opening 756 can have any shape corresponding to the shape of the locking member 718.

As best shown in FIG. 37, a ledge portion 757 can extend into at least a portion of the opening 756 to define a ledge on which a portion of the locking member 718 can be disposed. The proximal end 759 of the ledge portion 757 can define a gap into which a portion of the locking member 718 can extend such that the locking member 718 can extend toward and/or contact the inner member 716.

Referring again to FIG. 34A, the outer member 712 can further comprise one or more apertures 758 extending through a thickness of the inner wall 720 and the outer wall 722. In the illustrated embodiment, each wall 720, 722 comprises two apertures 758. However, in other embodiments, the outer member 712 can comprise a greater or fewer number of apertures 758. The apertures 758 can be configured to allow the locking member 718 to be coupled to the outer member, as described in more detail below.

The inner member 716 can comprise an elongated member extending at least partially into the outer member 712. In some embodiments, the inner member 716 can be housed entirely within the outer member 712 and the outer member 712 can be closed at its distal end. As shown in FIG. 33A, the inner member 716 can comprise a linear rack 760 having a plurality of teeth 762. In the illustrated embodiment, the linear rack 760 extends a portion of the length of the inner member 716 adjacent the proximal end portion 754. In other embodiments, the linear rack 760 can extend substantially the entire length of the inner member 716. In still other embodiments, the linear rack 760 can extend a portion of the length of the inner member adjacent the distal end portion 728.

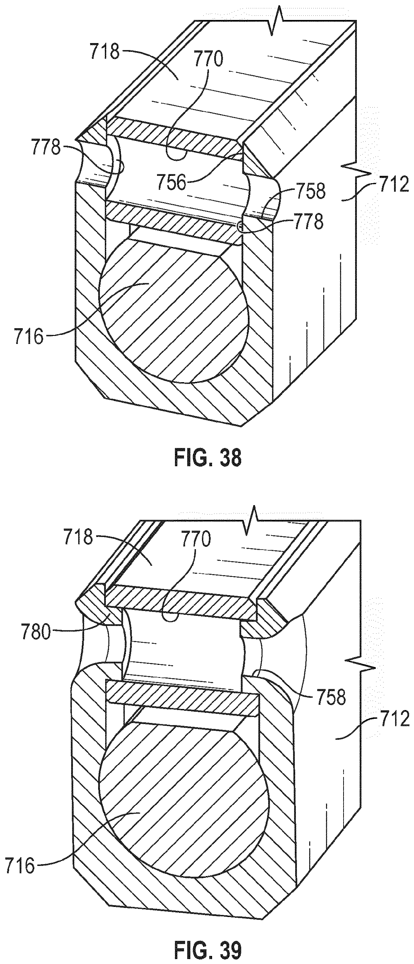

Referring now to FIG. 35, the locking member 718 can comprise an elongated body 764 comprising a first end portion 766 and a second end portion 768. When coupled to the outer member 712, the locking member can be referred to as a pawl 718. The first portion 766 can comprise one or more apertures 770 on first and/or second side portions 772 of the locking member. For example, in the illustrated embodiment, the locking member 718 includes two apertures 770 that extend through a width of the locking member. In other embodiments, the locking member can comprise a greater or fewer number of apertures. In still other embodiments, the apertures need not extend fully through the width of the locking member. For example, the locking member can comprise two pairs of aligned apertures, one on each side 772, that extend through a portion of the width of the locking member. The apertures 770 can be configured to align with the apertures 758 in the outer member 712 when the locking member 718 is disposed in opening 756. The locking member 718 can be coupled to the outer member 712 via the apertures 758, 770, as described below with reference to FIGS. 38-40.

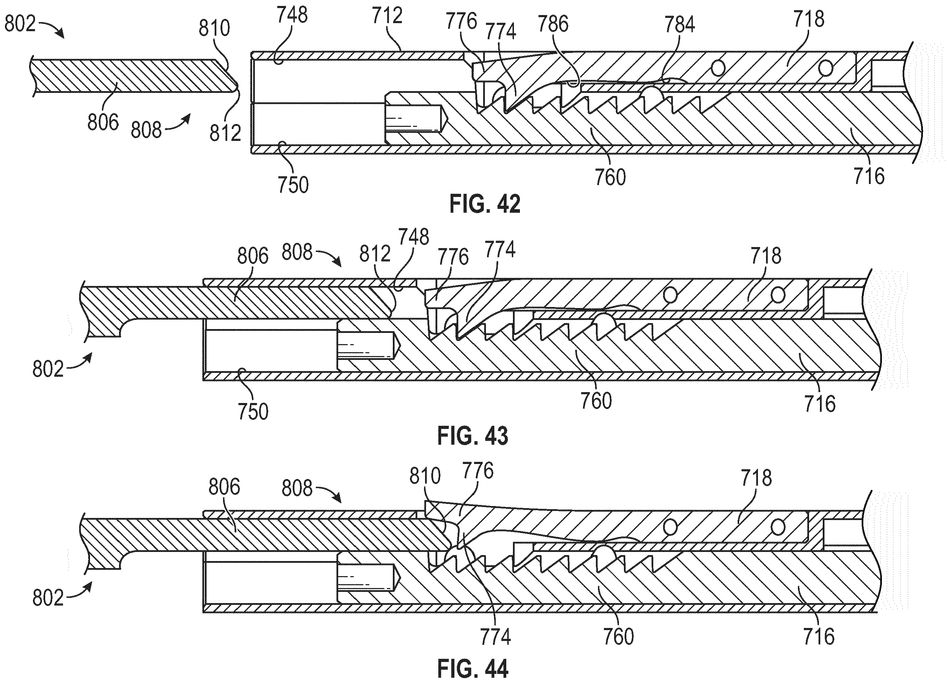

The second end portion 768 of the locking member 718 can comprise a locking tooth 774 that can engage the teeth 762 of rack 760 and a disengagement tooth 776. The disengagement tooth 776 can extend axially from the second end portion 768 and can be configured to engage a disengagement member, such as disengagement member 802, as described in more detail below with reference to FIGS. 43-46. The locking tooth 774 can extend toward the linear rack 760 and can have a shape that is complimentary to the shape of the teeth 762, such that the locking tooth 774 allows sliding movement of the inner member 716 in one direction relative to the locking member 718 (e.g., in the proximal direction) and resists sliding movement of the inner member in the opposite direction (e.g., in the distal direction) when the locking tooth 774 is in engagement with one of the teeth 762 of the linear rack.

Referring to FIGS. 38-40, the locking member 718 can be coupled to the outer member 712 in the following exemplary manner. As shown in FIG. 38, the locking member 718 can be disposed within the opening 756 such that the apertures 770 of the locking member align with the apertures 758 of the outer member 712. The apertures 770 of the locking member 718 can have a diameter greater than the apertures 758 of the outer member 712 such that when the apertures 758, 770 are aligned, each aperture 770 of the locking member 718 defines an annular lip 778 around each aperture 758 of the outer member. In a particular embodiment, the apertures 770 of the locking member 718 can have a diameter of about 0.5 mm and the apertures 758 of the outer member 712 can have a diameter of about 0.3 mm.

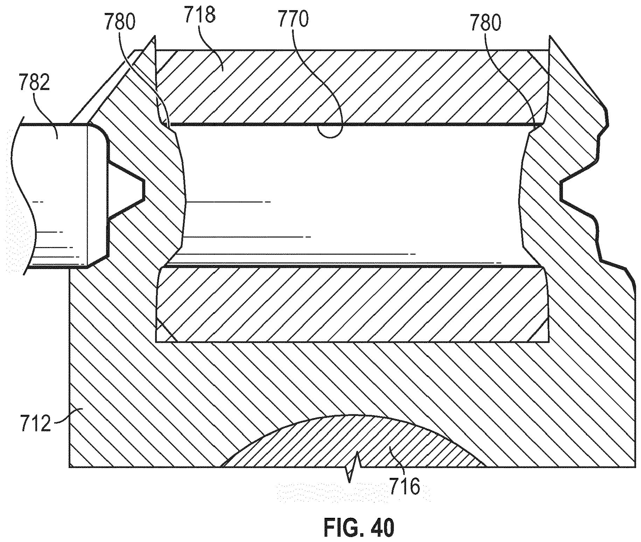

Referring now to FIG. 39, an inwardly-directed force (e.g., toward the locking member 718) can be applied to the outer member 712 thereby deforming the annular lip 778 inwards to form a protrusion 780 that extends into the apertures 770 of the locking member 718. In other embodiments, in lieu of apertures 758 in the outer member 712, the inwardly-directed force can be applied directly to the surface of the inner and outer walls 720, 722 of the outer member 712 thereby deforming the surface radially inwardly to form a dome or hemispherical shaped projection that secures the outer member 712 to the locking member 712, as depicted in FIG. 40.

While FIGS. 38-39 show inner member 716 having a flat edge, in other embodiments, the inner member 716 can have a cylindrical shape.

As shown in the illustrated embodiment, the protrusion can have a substantially cylindrical shape. As shown in FIG. 40, the inwardly-directed force can be applied using a punch member 782. The punch member 782 can be, for example, a cylindrical member comprising a cross-sectional diameter greater than the diameter of apertures 758 but less than the diameter of apertures 770 such that the punch member can extend into the apertures 770 of the locking member in order to deform the annular lip 778 into the aperture 770 thereby securing the locking member 718 to the outer member 712.

This configuration can advantageously simplify manufacturing, for example, by allowing much simpler processing and machining procedures (such as Swiss-type and milling procedures) to be used. Additionally, this configuration avoids small fasteners, which can in some instances be difficult to manufacture and assemble, and additionally avoids welding, which can be inaccurate and impractical at such small sizes. Moreover, the oval (or circular) shapes of the inner member 716 and the openings formed in the outer member 712 are easier to manufacture than components having square or rectangular cross-sectional shapes.

In other embodiments, although less desirable, the locking member 718 can be coupled to the outer member 712 using one or more fasteners extending through the apertures 758, 770. The fasteners can be, for example, rivets or pins.

In alternative embodiments, the locking member 718 can be formed integrally with the outer member 712 such as by cutting the shape of the locking member 718 into a sidewall 724, 726 of the outer member.

Referring to FIGS. 31 and 32, the expansion and locking mechanism 710 can comprise a ratchet mechanism or rachet assembly formed by the inner member 716 and the locking member 718. The locking member 718 can be coupled to the outer member 712 using the methods described above, to form a pawl configured to engage the teeth 762 of the inner member. As mentioned previously with respect to expansion and locking mechanism 550, this configuration, can help mitigate the risk of damage to the soft components of the valve and/or soft tissue of the patient, because the interlocking and/or ratcheting components of the locking mechanism 710 are internal to the mechanism.

Furthermore, as shown in the illustrated embodiment of FIGS. 29-46, the opening 756 and the locking member 718 can be positioned more distally from the outflow end of the frame (e.g., compared to the position of the pawl 568 in the embodiment of FIGS. 14-25). This position advantageously allows the locking member 718 to be distanced from the commissure region of the prosthetic valve 700 such that the locking member 718 does not interfere with the commissures. In some embodiments, the locking member 718 can be located upstream of the adjacent commissure and the inflow edges of the leaflets of the prosthetic valve (e.g., the locking member 718 can be below the commissure and the inflow edges of the leaflets when inflow end of the prosthetic valve is the lower end of the prosthetic valve). This configuration can prevent or mitigate harm to the soft components of the prosthetic valve 700, especially the leaflets.

Additionally, this configuration can advantageously provide sufficient engagement between the locking tooth 774 and the teeth 762 of the linear rack in the locked stated, without the need to form oblique engaging edges, thereby significantly simplifying the manufacturing process.

The pawl 718 and the teeth 762 are configured such that when the pawl 718 is engaged with the rack 760, the inner member 716 and the outer member 712 can move relative to one another in a first axial direction, but are prevented from moving relative to one another in a second, opposite axial direction. For example, in the illustrated embodiment, when the pawl 718 is engaged with the rack 760, the inner member 716 can move axially in a proximal direction (see arrow 734 in FIG. 32) but cannot move axially in a distal direction (see arrow 736 in FIG. 32). This ensures that when the pawl 718 is engaged with the rack 760, the frame 702 can be radially expanded but cannot be radially compressed.

Once the prosthetic valve has been implanted within a selected implantation site within a patient, the patient's native anatomy (e.g., the native aortic annulus) may exert radial forces against the prosthetic valve that would tend to compress the frame 702. However, the engagement between the pawl 718 and the rack 760 prevents such forces from compressing the frame 702, thereby ensuring that the frame remains locked in the desired radially expanded state.

Referring to FIG. 35, the locking member 718 can further comprise first and second cutouts defining a first neck portion 784 and a second neck portion 786. The first and second neck portions 784, 786 can be configured to provide selected elasticity in both the axial and radial directions. That is, the neck portions 784, 786 allow the pawl 718 to retain a locked state under relatively high axial forces, while enabling the disengagement of the locking tooth 774 from the linear rack 760 in response to a relatively low radial force.

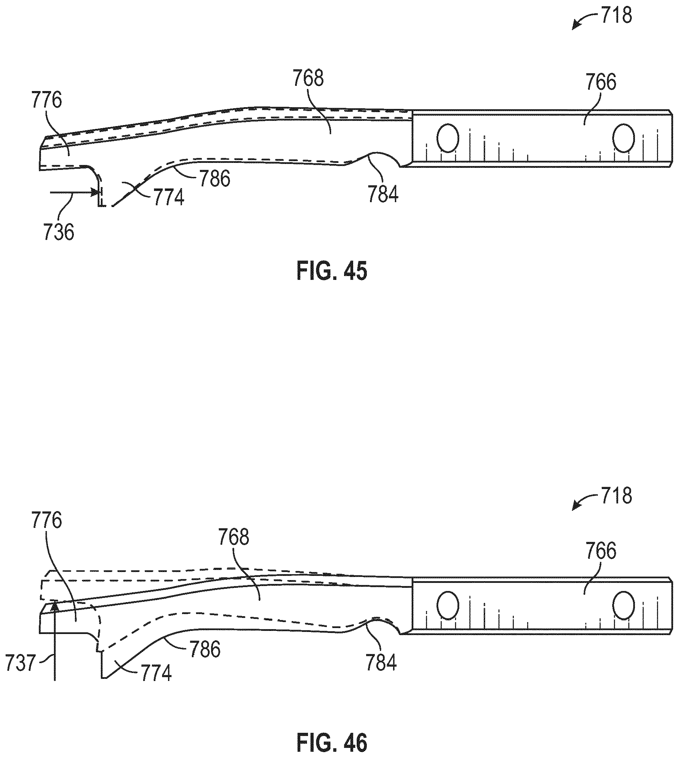

As shown in FIG. 37, the first neck portion 784 can be configured to bias the pawl 718 inwardly such that the locking tooth 774 is resiliently retained in a position engaging one of the teeth 762 of the inner member 716 (which can be referred to as the engaged position of the pawl 718). The biased configuration of the pawl 718 ensures that under normal operation, the locking tooth 774 stays engages with the teeth 762 of the rack 760. As shown in FIG. 46, the first neck portion 784 can be configured to bend easily under relatively low radial forces, for example, when the disengagement member 802 (described below) applies a force to the disengagement tooth 776 in the direction of arrow 737. The degree of indentation of the neck portion 784 can be adjusted such that a greater or lesser force is required to disengage the locking tooth 774 from the plurality of teeth 762.

The second neck portion 786 can be configured to further bias the locking tooth 774 against the linear rack 760. Referring to FIG. 45, an axial force (e.g., in the distal direction as indicated by arrow 736 in the illustrated embodiment) applied to the locking tooth 774 advantageously promotes further compression of the locking member 718 by pressing the locking tooth 774 further against the linear rack 760, allowing the locking member 718 to resist buckling or bending under axial forces exerted on the locking tooth 774.

Referring to FIG. 37, the inner member 716 can comprise a toothless portion 788 extending from a proximal edge 790 of the inner member 716 to the plurality of teeth 762. The toothless portion 788 is configured to allow bi-directional axial movement (in the distal and proximal directions) of the inner member 716 relative to the outer member 712. This allows the frame 702 to expand and/or contract prior to the engagement of the pawl 718 with the plurality of teeth 762. The length of the toothless portion 788 can be selected to provide a reversibility range in which the prosthetic valve can be freely expanded and compressed without locking.

During delivery of the prosthetic valve 700, the inner member 716 can be coupled to an actuation member of the delivery apparatus (such as actuation member 524 described above). When so coupled to the actuation member, the toothless portion 788 of the inner member 716 and a distal end portion of the actuation member define a reversibility range.

A prosthetic valve 700 including one or more expansion and locking mechanisms 710 can be expanded in the following exemplary manner. Generally, the prosthetic valve 700 is placed in a radially compressed state and releasably coupled to a distal end portion of a delivery apparatus, such as delivery apparatus 300 (FIG. 6), and then advanced through the vasculature of a patient to a selected implantation site (e.g., the native aortic annulus). The prosthetic valve 700 can then be deployed at the implantation site and expanded and locked in the expanded configuration using the expansion and locking mechanisms 710.

Each expansion and locking mechanism 710 can be releasably coupled to a respective actuation assembly of a delivery apparatus, such as actuation assemblies 306 of delivery apparatus 300. Each actuation assembly 306 can comprise a first or outer actuation member 308 (see FIG. 6) and a second or inner actuation member 309 (see FIG. 1). The second actuation member 309 can extend co-axially through the first actuation member 308. A distal end portion of the outer actuation member 308 can abut a proximal end portion 794 of the outer member 712.

Referring to FIG. 37, in a particular example, a proximal end portion 754 of the inner member 716 can comprise an engagement portion configured as an inner bore 792 including, for example, a threaded portion. The threaded portion can be configured to couple a correspondingly threaded engagement portion of the inner actuation member 309. The correspondingly threaded portions can releasably secure the inner member 716 and the second actuation member 309 to one another.

In other embodiments, the engagement portion can have other configurations that permit releasably coupling the second actuation member to the inner member 716. For example, the engagement portion of the second actuation member 309 can comprise a magnet and the inner bore 792 of the inner member 716 can comprise a correspondingly magnetic material into which the engagement portion of the second actuation member can extend. The delivery apparatus can be used to apply a distally directed force to the outer member 712 via the outer actuation member 308 and/or a proximally directed force to the inner member 716 via the inner actuation member 309 to move the outer member 712 and the inner member 716 axially relative to one another in a telescoping manner to cause the frame 702 to radially expand.

When the frame 702 is in the radially compressed configuration, the inner member 716 can move relative to the outer member 712 in the proximal and/or distal directions. As the inner member 716 moves, the locking tooth 774 of the pawl 718 can slide along a distal end portion of the outer actuation member 308 and/or the toothless portion 788 of the inner member 716 until it engages the plurality of teeth 762, as shown in FIG. 37. The engagement of the pawl 718 with the plurality of teeth 762 allows for continued radial expansion of the frame but prevents radial compression of the frame.

The frame 702 can continue to be expanded by, for example, moving the inner member 716 proximally (as shown by arrow 734) until a selected prosthetic valve diameter is achieved. The selected diameter can correspond to a selected position of the locking tooth 774 of the pawl 718 in which it engages any tooth of the plurality of teeth 762.

As shown in FIG. 37, the expansion and locking mechanism 710 can have one or more engagement surfaces configured to prevent over-expansion of the prosthetic valve 700. For example, in the illustrated embodiment, an engagement surface 796 can extend into the inner bore 714 of the outer member 712. The engagement surface 796 can be configured to engage a corresponding engagement surface 798 of the inner member 716 to prevent further proximal movement of the inner member 716 relative to the outer member 712 to prevent over expansion of the prosthetic valve 700. As shown in the illustrated embodiment, the locking surface 798 can be disposed on a protrusion 800 extending from a distal end portion 728 of the inner member 716. In other embodiments, the expansion and locking member 710 can comprise a stopper or other mechanism, such as stopper 814 described below.

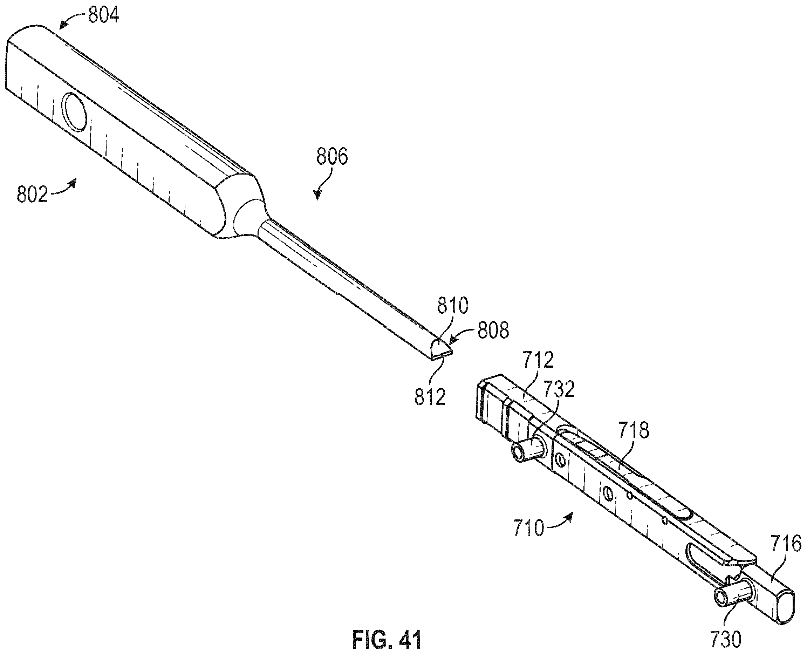

Referring now to FIGS. 41-44, in some embodiments, the expansion and locking mechanism 710 can receive a disengagement member 802. The disengagement member 802 can be configured to selectively disengage the pawl 718 from the linear rack 760 such that the frame 702 can be radially compressed.

The disengagement member 802 can comprise a first end portion 804 and a second end portion 806. The second end portion 806 can be an elongated member sized to be inserted through the first opening 748 in the bore 714 of the outer member 712. A distal end portion of the second end portion can comprise a disengagement portion 808 including an angled surface 810.

As shown in FIGS. 42-44, the disengagement member 802 can be inserted into the outer member 712 via first opening 748. As the disengagement member 802 advances, a distal tip 812 of the disengagement portion 808 can engage the disengagement tooth 776 of the pawl 718. The disengagement tooth 776 can slide along the angled surface 810, bending the pawl 718 and lifting the locking tooth 774 off of the linear rack 760, as shown in FIG. 44. This allows the inner member 716 to move relative to the outer member 712 in the proximal and/or distal directions, allowing for unrestricted radial expansion and radial compression of the prosthetic valve 700. The disengagement member 802 can be removed from the expansion and locking mechanism 710 by, for example, moving the disengagement member 802 in a proximal direction until the disengagement portion 808 no longer engages the disengagement tooth 776. Once the disengagement member 802 is removed, the pawl 718 can bias toward the inner member 716 and engage the plurality of teeth 762 thereby locking the prosthetic valve 700 at a selected diameter.

In some embodiments, the disengagement member 802 can be used to selectively disengage the pawl 718 from the plurality of teeth 762 after the locking tooth 774 has engaged the linear rack 760 to lock the prosthetic valve 700 in an expanded diameter during an implantation procedure. For example, the disengagement member 802 can be advanced distally into the outer member 712 to disengage the pawl 718 from the plurality of teeth 762 to permit radial compression of the frame 702 if repositioning or recapture and removal of the prosthetic valve is desired.

In some embodiments, the disengagement member 802 can be used to perform any of the various functions described with respect to the retaining member 530, described above. That is, in particular embodiments, the disengagement member 802 is used to unlock the prosthetic valve 700 from an expanded configuration during assembly of the prosthetic valve and/or during a loading procedure, permitting radial compression of the prosthetic valve for subsequent loading of the prosthetic valve into or on the delivery apparatus.

In other embodiments, the disengagement member 802 can be a component of the delivery apparatus and can be disposed between the inner member 716 and the pawl 718 during the implantation procedure. In some embodiments, the delivery apparatus can include an actuation assembly 306 or 520 for each expansion and locking mechanism 710, and each actuation assembly 306, 520 can include a disengagement member 802 that can extend through a corresponding outer actuation member 308 or 522 of an actuation assembly 306, 520, respectively. Each disengagement member 802 can extend from the prosthetic valve 700 to the handle of the delivery apparatus (e.g., handle 302). The proximal end portion of each disengagement member 802 can be operatively connected to a knob on the handle, which is operable to move the disengagement members proximally and distally relative to the expansion and locking mechanisms 710.

After delivering the prosthetic valve to a location at or adjacent the implantation site, the user can freely adjust the diameter of the prosthetic valve, including radially expanding and compressing the prosthetic valve. Once a selected expanded diameter for the prosthetic valve is achieved, the user can remove the disengagement member 802 from its corresponding expansion and locking mechanism 710 in order to lock the prosthetic valve at the selected expanded diameter.

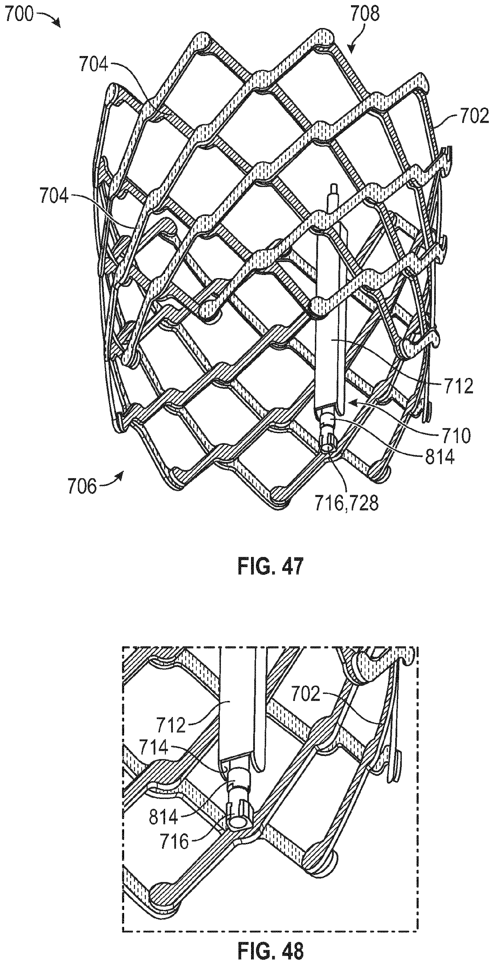

As shown in FIGS. 47-49, and mentioned previously, in some embodiments, in lieu of or in addition to engagement surface 798, the expansion and locking mechanism 710 can comprise a stopper 814. In some such embodiments, the inner member 716 can further lack the protrusion on which engagement surface 798 is disposed. The stopper 814 can be, for example, an annular nut disposed around a distal end portion 728 of the inner member 716. The stopper 814 can be configured to prevent movement of the second member 716 relative to the outer member 712 past a predetermined point.

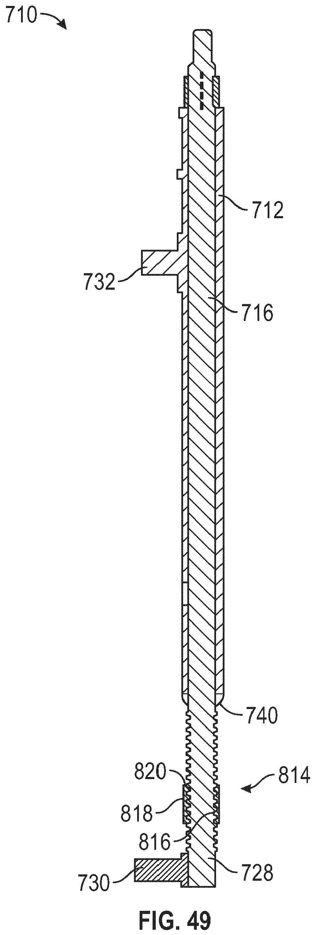

Referring to FIG. 49, the stopper 814 can have an annular inner surface 816 and an annular outer surface 818 defining a shoulder 820 between them. The shoulder 820 can be sized to abut or engage the distal edge 740 of the outer member 712 to prevent further proximal movement of the inner member 716 relative to the outer member 712 to prevent over-expansion of the prosthetic valve 700 past a predetermined diameter.

In some embodiments, the distal end portion 728 of the inner member 716 can comprise a threaded portion extending all or partially along the length of the distal end portion 728. The inner annular surface 816 of the stopper 814 can have a correspondingly threaded surface configured such that the stopper 814 can be axially displaced along the length of the inner member 716. For example, rotation of the stopper 814 in a first direction (e.g., clockwise) can move the stopper 814 distally relative to the inner member 716, and rotation of the stopper 814 in a second direction (e.g., counterclockwise) can move the stopper 814 proximally relative to the inner member 716. The axial position of the stopper 814 along the inner member 716 can determine the maximum diameter to which the prosthetic valve 700 can expand.

Prior to the implantation procedure, the physician can adjust the location of the stopper 814 along the inner member 716 to set a selected maximum diameter for the prosthetic valve 700 sized to accommodate a specific patient's anatomical variability (e.g., selecting the size which best fits the patient's native annulus).

In some embodiments, the inner member 716 can comprise a series of markings or measurement indicia that visually indicate to a physician the position of the stopper 814 that corresponds to a particular maximum prosthetic valve diameter. The physician can set a selected maximum diameter by adjusting the position of the stopper 814 such that it aligns with the indicator corresponding to the desired prosthetic valve diameter.

As the prosthetic valve is expanded (e.g., using actuators 20), the inner member 716 can slide proximally relative to the outer member 712 until the stopper 814 abuts the distal edge 740 of the outer member 712, thereby restraining further motion of the inner member 716 and retaining the prosthetic valve 700 at a predetermined diameter.

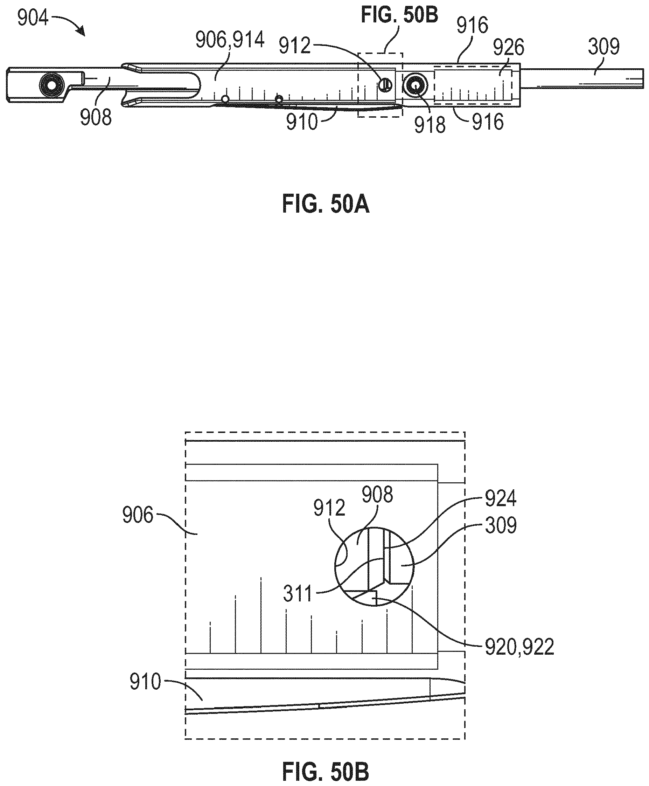

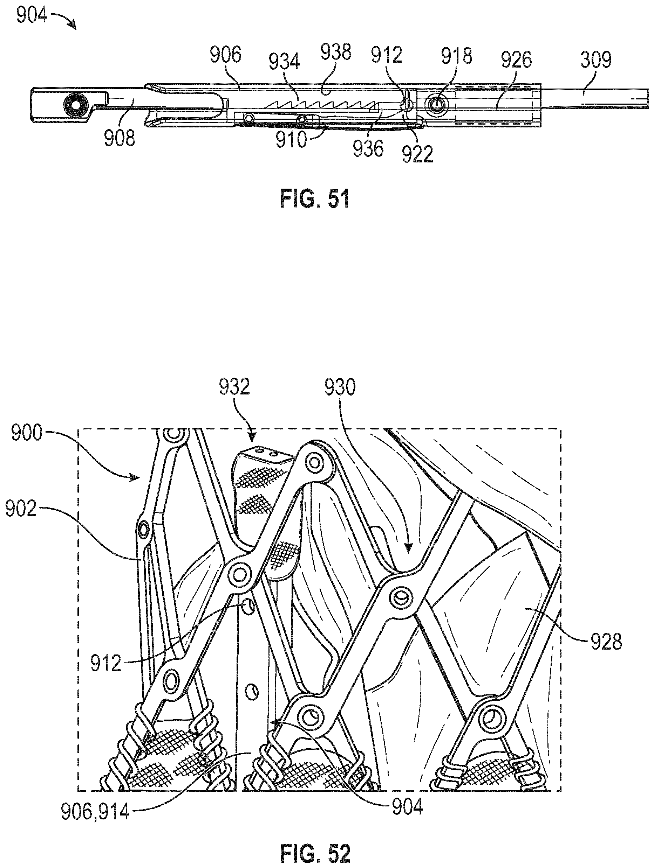

Referring now to FIGS. 50A-52, in some embodiments, a prosthetic valve 900 having frame 902 (FIG. 52) can comprise one or more expansion and locking mechanisms 904. Expansion and locking mechanisms 904 can be similar to expansion and locking mechanisms 710. That is, expansion and locking mechanisms 904 can have an outer member 906, an inner member 908 extending at least partially into a bore 938 of the outer member 906, and a locking member 910, all similar to those of expansion and locking mechanisms 710, except that expansion and locking mechanisms 904 further comprise at least one opening or aperture 912. The aperture 912 can be configured and positioned to allow visual inspection of the attachment region between the expansion and locking mechanism 904 and an actuator of the delivery apparatus, such as second actuator 309 of delivery apparatus 300 (FIG. 6), described above. Although one opening 912 is shown in FIG. 50A, the outer member 906 can include multiple openings 912 that can be axially spaced from each other along the outer member.

As shown in FIG. 50A, in the illustrated embodiment, the aperture 912 extends through a thickness of the outer wall 914 (e.g., the wall facing the frame 902) of the outer member 906 such that the inner components of the expansion and locking mechanism 904 are visible. In other embodiments, the aperture 912 may be positioned on the inner wall (not shown) or one of the side walls 916 of the outer member 906.

In the illustrated embodiment, the aperture 912 is circular, however, in other embodiments, the aperture 912 can have any of various shapes. For example, the aperture can be an oval, a square, a rectangle, a square-oval, a triangle, a hexagon, an octagon, a pentagon, etc. In some embodiments, the aperture can have a shape that is symmetrical along two axes. In other embodiments, the aperture 912 can have a shape that is symmetrical along one axis, or a shape that is asymmetrical.

In the illustrated embodiment, the aperture 912 is positioned distal to the fastener 918 that extends from the outer member 906. As shown in FIG. 50B, in the illustrated embodiment, an apex 920 of the locking tooth 922 is visible through the aperture 912. Referring to FIG. 51, this configuration allows a user to determine whether the locking tooth 922 is engaged with the linear rack 934 or whether the tooth 922 is sliding along the toothless portion 936 of the inner member 908. This allows a user to determine whether the expansion and locking mechanism 904 is in the reversibility range (e.g., whether the inner member 908 can move bi-directionally relative to the outer member). The aperture 912 further allows a user to determine whether the locking tooth 922 has engaged the linear rack 934. Once the locking tooth 922 has engaged the linear rack 934, the inner member 908 can slide relative to the outer member 906 in a first direction, but is restrained from sliding relative to the outer member 906 in a second, opposite direction.

As best seen in FIG. 50B, a proximal edge 924 of the second member 908 and a distal edge 311 of the second actuator 309 can be visible through the aperture 912. This configuration enables a user to determine whether the delivery apparatus 300 is suitably coupled to the expansion and locking mechanism 904. In other words, the aperture 912 allows a user to determine whether the actuator 309 is actually coupled to the inner member 908, or whether the actuator 309 is merely disposed within the lumen of the outer member 906. "Suitably coupled" as used herein, means coupled to the extent that the second actuator 309 can actuate the second member 908 to expand and/or contract the prosthetic valve 900.

Referring again to FIG. 50A, expansion and locking mechanism 904 can further comprise a commissure attachment portion 926. As shown in FIG. 52, when the prosthetic valve 900 is assembled, one or more leaflets 928 of the valvular structure 930 can be coupled to the commissure attachment portion 926 to form a commissure 932. For example, adjacent leaflets 928 can be coupled to the commissure attachment portion 926 using one or more sutures. The sutures can, for example, wrap around the expansion and locking mechanism 904. As shown in the illustrated embodiment, the aperture 912 is positioned such that it is not covered, partially covered, or otherwise obscured by the commissure 932 when the prosthetic valve 900 is assembled. Further details regarding various attachments techniques and mechanisms for attaching commissures to expansion and locking mechanisms are disclosed in U.S. Publication No. 2018/0325665; U.S. Publication No. 2019/0105153; U.S. Application No. 62/869,948; U.S. Application No. 62/813,643; and PCT Application No. PCT/US2019/61392, all of which documents are incorporated herein by reference. Any of the techniques and mechanisms disclosed in the prior documents can be used to connect the commissures 932 to the expansion and locking mechanisms 904.

Once the prosthetic valve 900 is fully assembled, it can be coupled to the delivery apparatus 300 as described previously with respect to prosthetic valve 700. The second actuator 309 can extend into the bore 938 of the outer member 906 and releasably couple the second member 908. Because this coupling takes place within the bore 938 of the outer member 906, it can be difficult to determine whether the second actuator 309 is suitably coupled to the second member 908 rather than simply disposed within the bore. The aperture 912 advantageously allows a user to verify that the second actuator 309 has been coupled to the second member 908. The prosthetic valve 900 can then be radially compressed for delivery to the selected implantation site.

Positioning the aperture 912 as shown in the illustrated embodiment advantageously allows the user to determine whether the prosthetic valve 900 is coupled to the delivery apparatus 300 without compromising the structural integrity and/or strength of the commissure, and without compromising the structural integrity and/or strength of the prosthetic valve. Furthermore, an operator can determine, prior to radial compression and/or implantation of the prosthetic valve, whether the delivery apparatus and the prosthetic valve are suitably coupled.

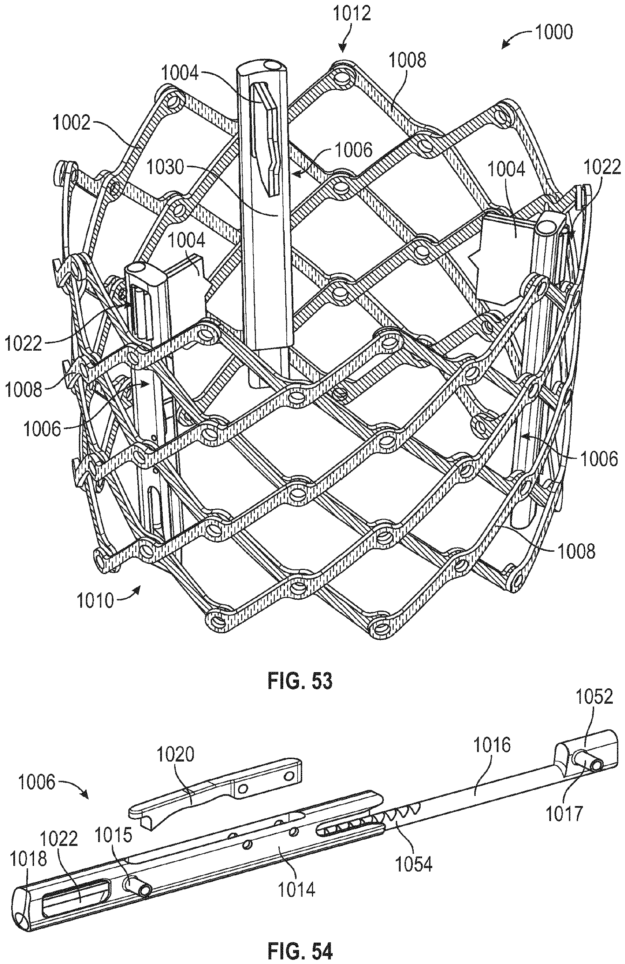

FIGS. 53-63 illustrate an exemplary embodiment of a prosthetic valve 1000 comprising a frame 1002, a valvular structure including one or more leaflets 1004 (portions of which are shown in FIG. 53) disposed within the frame 1002, and one or more expansion and locking mechanisms 1006. The frame 1002 can comprise a plurality of pivotably connected struts 1008 defining an inflow end 1010 (which is the distal end of the frame in the delivery configuration for the illustrated embodiment) and an outflow end 1012 (which is the proximal end of the frame in the delivery configuration for the illustrated embodiment). The struts 1008 are pivotably connected to each other at a plurality of junctions that permit pivoting of the struts relative to each other when the frame 1002 is radially compressed and expanded.

The prosthetic valve 1000 can include inner and/or outer skirts, as previously described, although these components are omitted for purposes of illustration. The one or more expansion and locking mechanisms 1006 can be used in lieu of or in addition to actuators 20, locking features 404, and/or expansion and locking mechanisms 550, 710 described above. The expansion and locking mechanisms 1006 can be used to both radially expand the frame 1002 and lock the frame in a radially expanded state.

FIG. 53 shows three expansion and locking mechanisms 1006 mounted to the frame 1002, which is in a radially expanded configuration. Though the illustrated embodiment shows three expansion and locking mechanisms 1006 spaced apart from each other about the circumference of the frame, it should be noted that a prosthetic valve can comprise any number of expansion and locking mechanisms 1006. For example, in some embodiments, a prosthetic valve can comprise a single expansion and locking mechanism, or two expansion and locking mechanisms, or four expansion and locking mechanisms, etc. The expansion and locking mechanisms 1006 can be placed at any position about the circumference of the frame 1002. For example, in some embodiments such as the illustrated embodiment, the expansion and locking mechanisms 1006 are equally spaced from one another about the circumference of the frame 1002. In other embodiments, it can be advantageous to have two or more expansion and locking mechanisms situated adjacent to one another.

Referring now to FIG. 54, the expansion and locking mechanisms 1006 can be similar to expansion and locking mechanisms 710, 904, described previously. That is, the expansion and locking mechanisms 1006 can comprise a first or outer member 1014, a second or inner member 1016 extending at least partially into a bore 1018 of the outer member 1014, and a locking member 1020, all similar to those of expansion and locking mechanism 710 except that each first member 1014 of expansion and locking mechanisms 1006 furthers comprise one or more commissure openings 1022. The commissure openings 1022 can be configured to accept portions of one or more leaflets 1004 to secure the leaflets 1004 to the frame 1002 via the expansion and locking mechanisms 1006.

Similar to expansion and locking mechanism 710, the outer member 1014 can comprise a first fastener 1015 coupled to the frame 1002 at a first location and the inner member 1016 can comprise a second fastener 1017 coupled to the frame 1002 at a second location spaced apart from the first location. The inner member 1016 can be axially movable relative to the outer member 1014 in a proximal direction (e.g., toward the outflow end 1012 of the frame) and in a distal direction (e.g., toward the inflow end 1010 of the frame). As such, because the inner member 1014 and the outer member 1016 are secured to the frame 1002 at axially spaced locations, moving the inner member 1016 and the outer member 1014 axially with respect to one another in a telescoping manner can cause radial expansion or compression of the frame 1002.

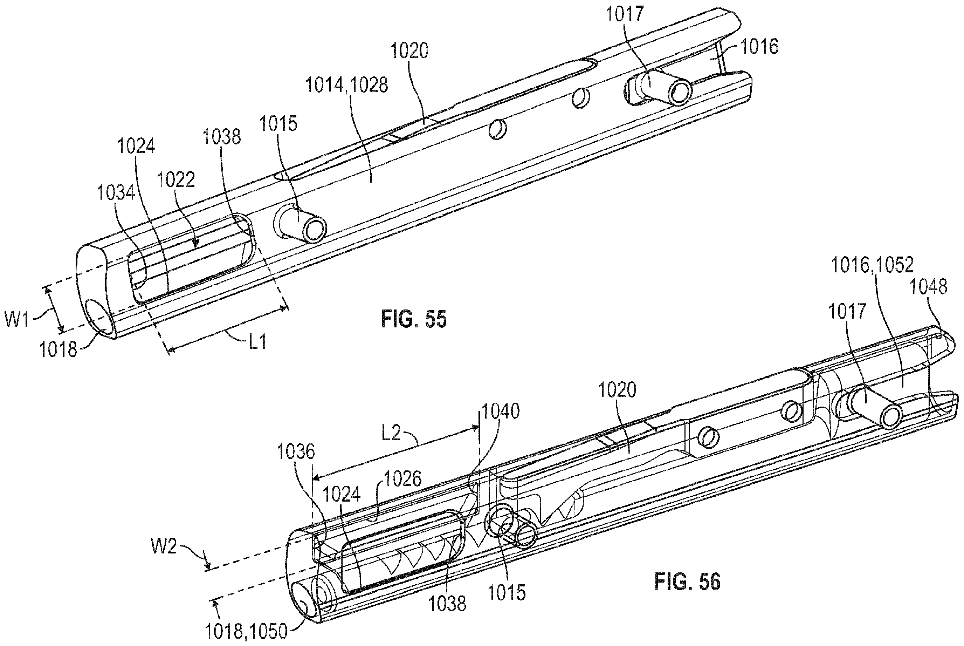

As shown in FIG. 55, each outer member 1014 can include a commissure opening 1022 extending through a thickness of the outer member 1014 and including a first aperture 1024 and a second aperture 1026 (see e.g., FIG. 55). The first aperture 1024 can be disposed in the outer wall 1028, e.g., the wall facing the frame, and the second aperture 1026 can be disposed in the inner wall 1030 (FIG. 53), e.g., the wall facing the valvular structure. As shown in FIG. 53, the commissure opening 1022 can be positioned proximal (e.g., towards the outflow end 1012 of the frame 1002) relative to the fastener 1015 that extends from the outer member 1014.

In the illustrated embodiment, the first aperture 1024 has a rectangular shape with rounded corners. The first aperture 1024 can have a width W₁ and a length L₁. As best seen in FIG. 56, the second aperture 1026 can also have a rectangular shape with rounded corners. The second aperture 1026 can have a width W₂ and a length L₂. In the illustrated embodiment, W₂ is less than W₁ and L₂ is greater than L₁. Accordingly, in the illustrated embodiment, the second aperture 1026 can have a narrow and elongated shape relative to the first aperture 1024. However, in other embodiments, the first and second apertures 1024, 1026 can have any of various shapes configured to accept the leaflets 1004. For example, the apertures 1024, 1026 can be ovular, square, rectangular, triangular, etc.

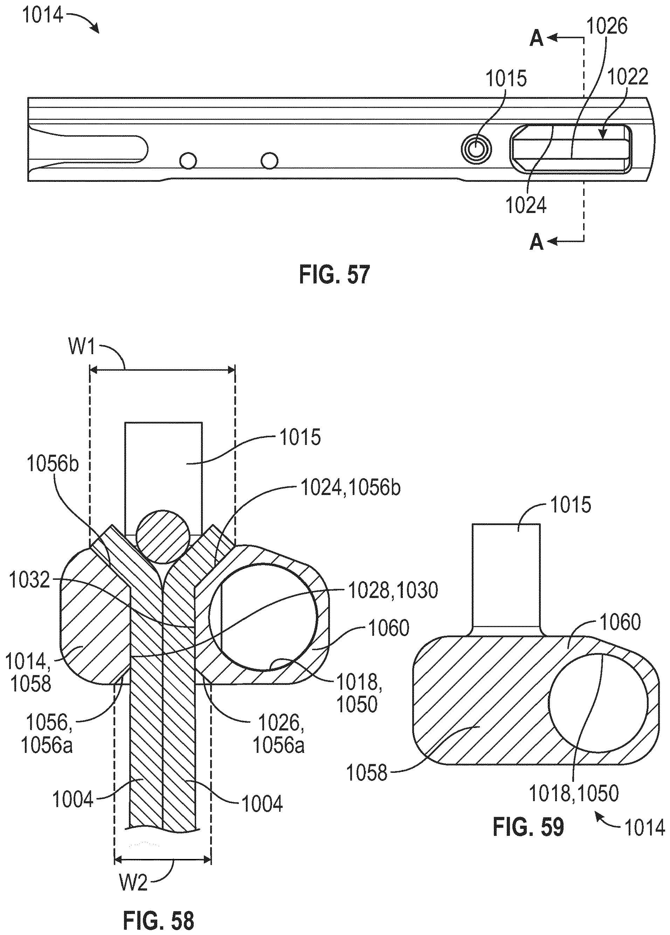

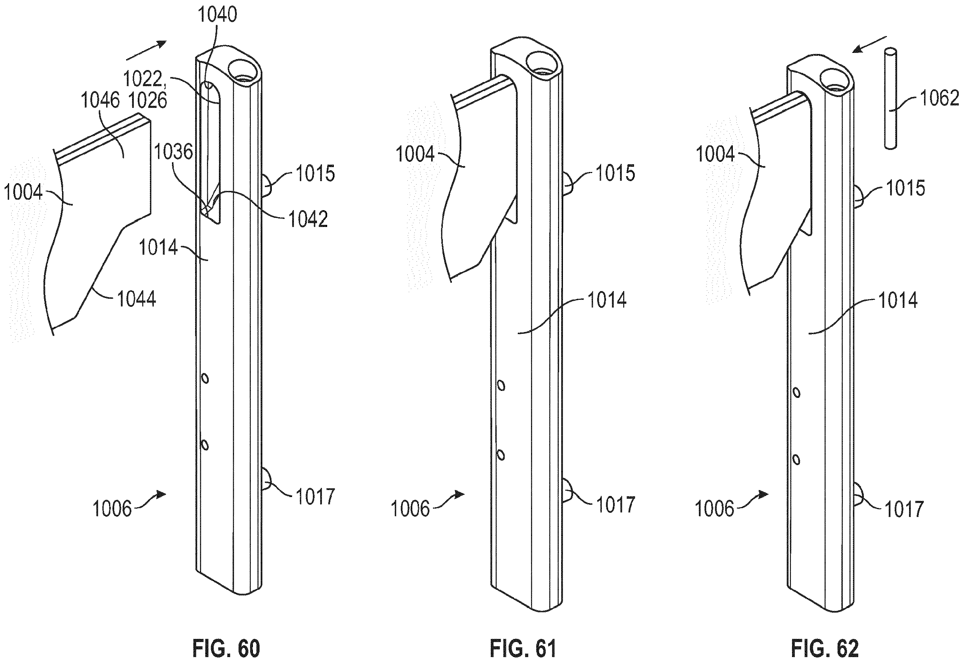

Referring to FIG. 58, the first and second apertures 1024, 1026 can be disposed such that they at least partially overlap along the length of the outer member 1014 such that a channel 1028 is formed between the first and second apertures 1024, 1026 defined by first and second side walls 1030, 1032. The referring to FIG. 56, the outflow edges 1034, 1036 of each aperture 1024, 1026 can be aligned, and the inflow edges 1038, 1040 can be offset from one another (e.g., the inflow edge 1040 of the second aperture 1026 can extend distally past the inflow edge 1038 of the first aperture 1024) such that an angled surface 1042 (FIG. 60) extends between the inflow edge 1036 of the second aperture 1026 and the inflow edge 1038 of the first aperture 1024. The angled surface 1042 can be configured to correspond to the angled cusp edge portion 1044 (FIG. 60) of one or more respective leaflets 1004 and can advantageously facilitate insertion of the tab portions 1046 (FIG. 60) of one or more leaflets 1004 into the commissure opening 1022, as well as advantageously mitigating frictional contact between the outer member 1014 and the leaflets 1004.

Referring to FIG. 56, as mentioned previously, the outer member 1014 can comprise a bore 1018 into which at least a portion of the inner member 1016 can extend. The bore 1018 can extend along the length of the outer member 1014 and can comprise a first or inflow portion 1048 and a second or outflow portion 1050. The inflow portion 1048 can have a substantially oval shape with flat sides in cross section corresponding to the inflow end portion 1052 of the inner member 1016. As shown in FIG. 59, the outflow portion 1050 of the bore 1018 can have a circular shape in cross section corresponding to the outflow end portion 1054 (FIG. 54) of the inner member 1016. In other embodiments, the inflow and/or outflow portions 1048, 1050 of the bore 1018 can have any of various shapes corresponding to the shapes of the inflow and/or outflow ends portions 1052, 1054 of the inner member 1016.

Referring again to FIG. 58, which shows a cross-section of the outer member 1014, each side wall 1030, 1032 of the commissure opening 1022 can comprise one or more chamfered surfaces 1056. In the illustrated embodiment, each sidewall 1030, 1032 comprises a first chamfered surface 1056a angled radially inwardly, a second chamfered surface 1056b angled radially outwardly. The first chamfered surfaces 1056a can be configured to mitigate frictional contact between the leaflets 1004 and the outer member 1014 during systole. The second chamfered surfaces 1056b can be configured to help secure the leaflets 1004 within the commissure opening 1022.

The outer member 1014 can have an asymmetrical shape in cross-section. Referring to FIG. 59, the outer member 1014 can have a first portion 1058 and a second portion 1060. The second portion 1060 can comprise the bore 1018 and can be larger than the first portion 1058. As shown in FIG. 58, in the illustrated embodiment, the commissure opening 1022 is aligned with the fastener 1015 extending from the outer member 1014. However, in other embodiments, the commissure opening 1022 can be offset from the fastener 1015.

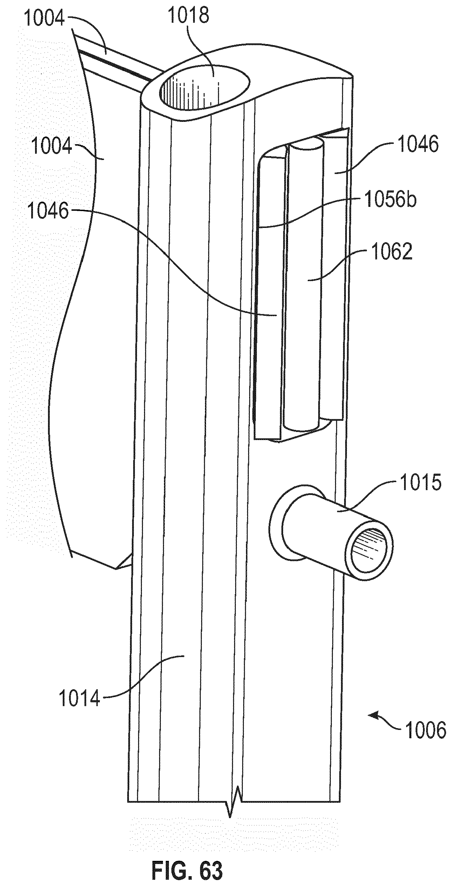

Referring now to FIGS. 60-62, during assembly of the prosthetic valve 1000 two adjacent leaflets 1004 can be coupled to a respective expansion and locking mechanism 1006 in the following exemplary manner. As shown, the tabs 1046 of adjacent leaflets 1004 can be inserted into the second aperture 1026 of the commissure opening 1022 such that they extend through the commissure opening 1022 and out the first aperture 1024. Once the tabs 1046 are disposed within the commissure opening 1022, a wedge 1062 (FIG. 62) can be inserted between the portions of the tabs 1046 that extend through the first aperture 1026. As shown in FIG. 63, the wedge 1062 can press portions of the tabs 1046 against the second chamfered surfaces 1056b. The wedge 1062 can be disposed such that it is aligned with the fastener 1015 of the outer member 1014.

In some embodiments, as shown, the wedge 1062 is an elongated member having a circular shape in cross-section. However, in other embodiments, the wedge can have any of various shapes in cross-section, such as, for example, triangular, ovular, square, rectangular, C-shaped, semi-circular, etc.

The tabs 1046 and wedge 1062 can then be coupled to the expansion and locking mechanism 1006 using, for example, one or more sutures. For example, the sutures can wrap around the expansion and locking mechanism 1006 such that they hold the wedge 1062 in position. Further details regarding various attachments techniques and mechanisms for attaching commissures to expansion and locking mechanisms are disclosed in U.S. Publication No. 2018/0325665; U.S. Publication No. 2019/0105153; U.S. Application No. 62/869,948; U.S. Application No. 62/813,643; and PCT Application No. PCT/US2019/61392, all of which documents are incorporated herein by reference. Any of the techniques and mechanisms disclosed in the prior documents can be used to connect the commissures formed by tabs 1046 to the expansion and locking mechanisms 1006.

Referring again to FIG. 53, in the illustrated embodiment, the expansion and locking mechanisms 1006 are coupled to the frame 1002 such that the commissure openings 1022 extend past the outflow end 1012 of the frame 1002. This configuration advantageously allows the leaflets 1004 to be coupled to the expansion and locking mechanisms 1006 after the expansion and locking mechanisms 1006 have been coupled to the frame 1002. In other embodiments, the expansion and locking mechanisms 1006 can be coupled to the frame 1002 such that the commissure openings 1022 do not extend past the outflow end 1012 of the frame. In such embodiments, the leaflets 1004 may be coupled to the expansion and locking mechanisms 1006 prior to coupling the expansion and locking mechanisms 1006 to the frame 1002.

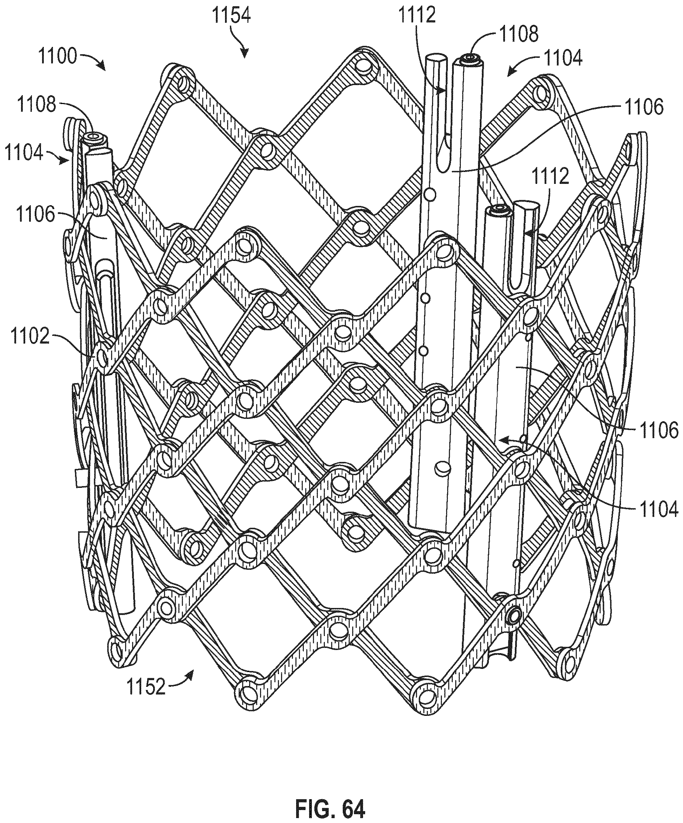

FIGS. 64-74 illustrate another embodiment of a prosthetic valve 1100 having a frame 1102, an inflow end 1152, an outflow end 1154, and a plurality of expansion and locking mechanisms 1104. The expansion and locking mechanisms 1104 can be similar to expansion and locking mechanisms 1006 described above (e.g., including an outer member 1106, inner member 1108, and locking member 1110) except that the commissure opening 1112 can be an "open" commissure opening, as described in more detail below. FIG. 64 shows three expansion and locking mechanisms 1104 mounted to the frame 1102, which is in a radially expanded configuration, though other embodiments can comprise any number of expansion and locking mechanisms 1104.

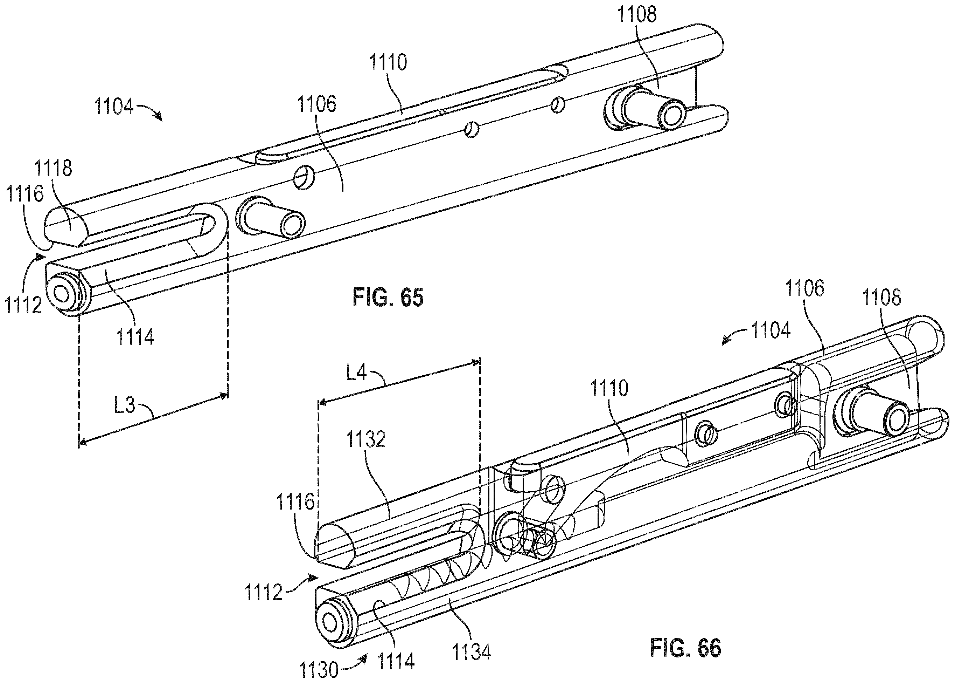

Referring now to FIG. 65, each expansion and locking mechanism 1104 can comprise a commissure opening 1112. The commissure openings 1112 can be similar to commissure openings 1022, except that each aperture 1114, 1116 extends to an outflow edge 1118 of the outer member 1106. Such commissure openings 1112 can be referred to as "open" commissure openings, in contrast to commissure openings 1022 which can be referred to as "closed" commissure openings.

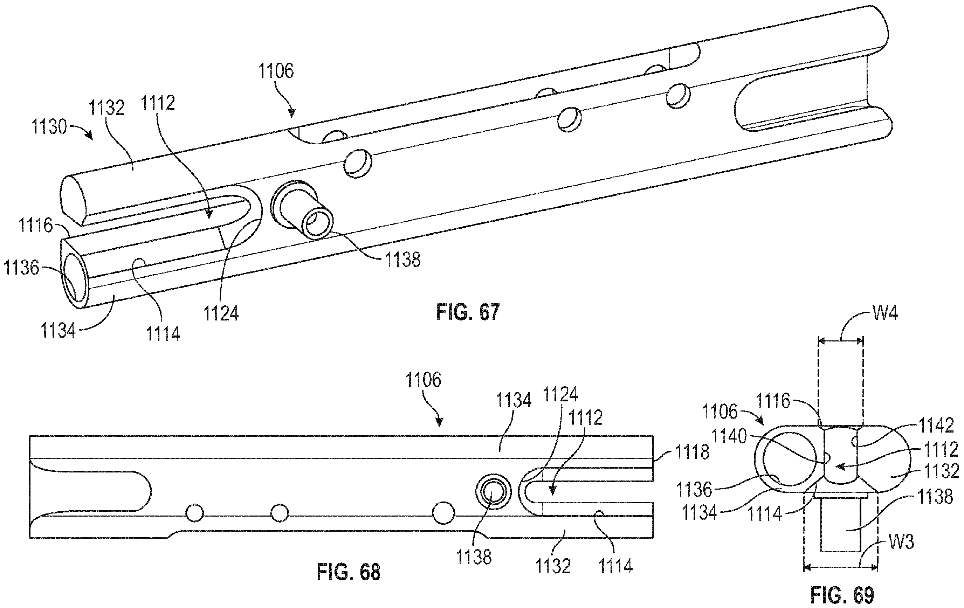

In the illustrated embodiment, each commissure opening 1112 is substantially U-shaped. However, in other embodiments, the commissure opening 1112 can have any of various shapes configured to accept the leaflets 1120. For example, the commissure openings 1112 can be ovular, square, rectangular, triangular, L-shaped, T-shaped, etc. The first aperture 1114 can have a length L₃ and the second aperture 1116 can have a length L₄ greater than L₃. As shown in FIG. 69, the first aperture 1114 can have a width W₃ and the second aperture 1116 can have a width W₄ less than W₃.

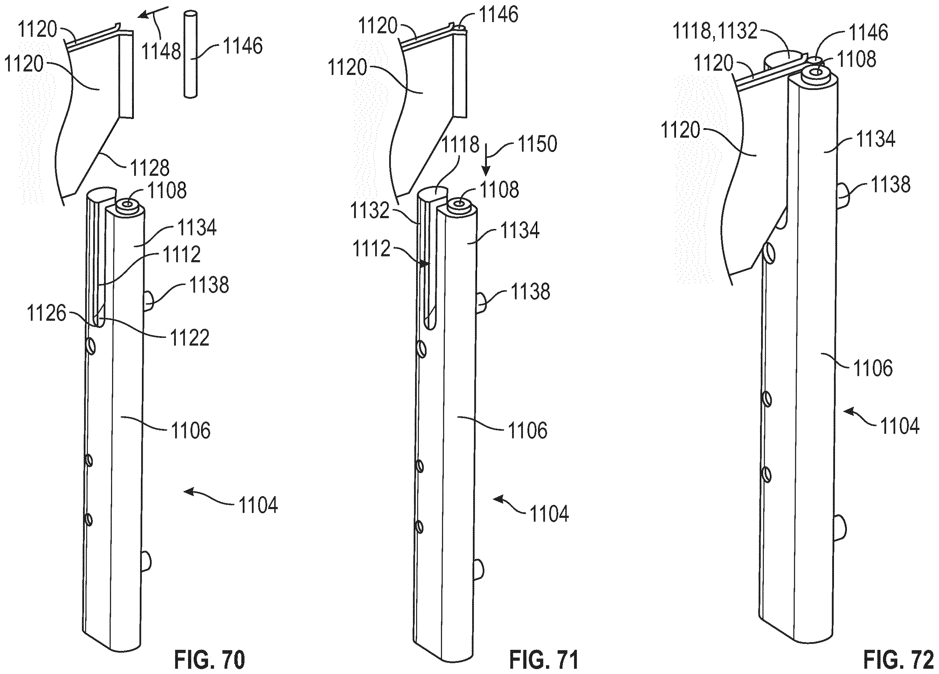

Similarly to commissure openings 1022, commissure openings 1112 can have an angled surface 1122 extending between the inflow edges 1124, 1126 (FIG. 70) of the apertures 1114, 1116. The angled surface 1122 can be configured to correspond to the angled cusp edge portion 1128 (FIG. 70) of one or more respective leaflets 1120 and can advantageously mitigate frictional contact between the expansion and locking mechanism 1104 and the cusp edge portion 1128 of the leaflets 1120.

Referring to FIG. 67, the commissure opening 1112 can separate the outflow end portion 1130 of the outer member 1106 into a first portion 1132 and a second portion 1134. The bore 1136 into which at least a portion of the inner member 1108 can extend can be disposed in the second portion 1134. The outflow end portion of the bore 1136 can have a substantially circular shape in cross-section where it extends through the second portion 1134. In other embodiments, the outflow and/or inflow portions of the bore 1136 can have any of various shapes corresponding to the shapes of the outflow and/or inflow end portions of the inner member 1108.

As shown in FIG. 69, in the illustrated embodiment, the first and second portions 1132 and 1134 can be unequally sized. For example, the second portion 1134 comprising bore 1136 can be larger than the first portion 1132. In the illustrated embodiment, the commissure opening 1112 is aligned with the fastener 1138 extending from the outer member 1106. However, in other embodiments, the commissure opening 1112 can be offset from the fastener 1138.

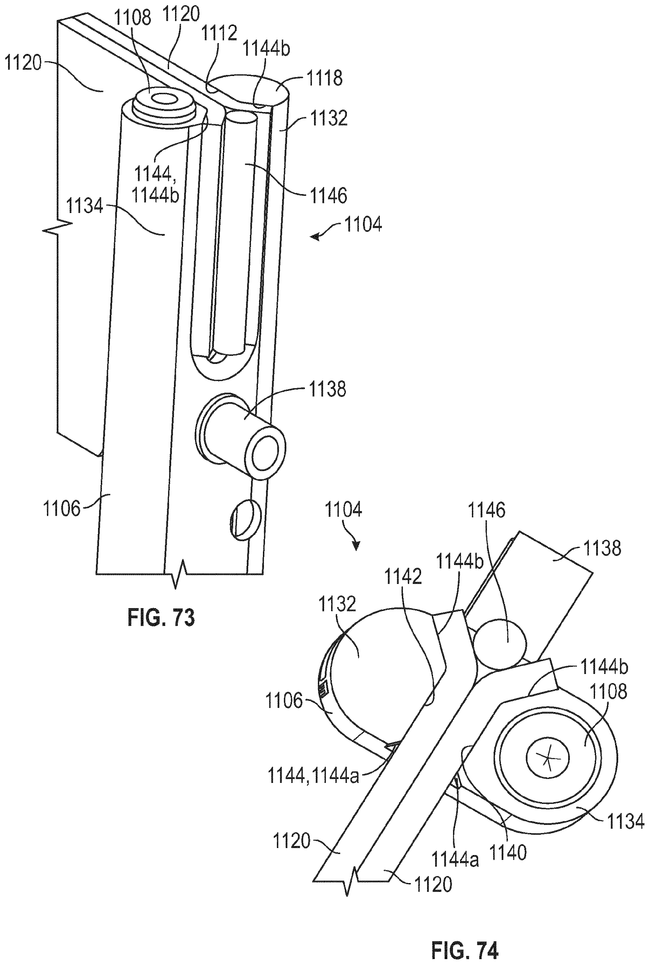

As best seen in FIG. 74, each side wall 1140, 1142 of the commissure opening 1112 can comprise one or more chamfered surfaces 1144. In the illustrated embodiment, each sidewall 1140, 1142 comprises a first chamfered surface 1144a angled radially inwardly, a second chamfered surface 1144b angled radially outwardly. The first chamfered surfaces 1144a can be configured to mitigate frictional contact between the leaflets 1120 and the outer member 1106 during systole. The second chamfered surfaces 114b can be configured to help secure the leaflets 1120 within the commissure opening 1112.

Referring now to FIGS. 70-72, during assembly of the prosthetic valve 1100 two adjacent leaflets 1120 can be coupled to a respective expansion and locking mechanism 1104 in the following exemplary manner. As shown in FIG. 70, a wedge 1146 can be inserted between adjacent leaflets 1120, as shown by arrow 1148, and can be coupled thereto (e.g., using one or more sutures). The leaflets 1120 can then be inserted into the commissure opening 1112 by, for example, sliding them into the commissure opening 1112 through the opening at the outflow end 1118 of the outer member 1106, as shown by arrow 1150 (FIG. 71). The disclosed configuration advantageously allows the commissure assemblies (e.g., the adjacent leaflet 1120 portions and wedge 1146) to be pre-assembled prior to mounting the commissure assembly within the commissure opening 1112, which advantageously simplifies the assembly process.

As best seen in FIGS. 73-74, once the leaflets 1120 are disposed within the commissure opening 1112, the wedge 1146 presses portions of the leaflets 1120 against the second chamfered surfaces 1144b. The wedge 1146 can be disposed such that it is aligned with the fastener 1138 of the outer member 1106. In the illustrated embodiment, the wedge 1146 is an elongated member having a circular shape in cross-section. However, in other embodiments, the wedge can have any of various shapes in cross-section, such as, for example, triangular, ovular, square, rectangular, C-shaped, semi-circular, etc.

The leaflets 1120 and wedge 1146 can then be coupled to the expansion and locking mechanism 1104 using, for example, one or more sutures. For example, the sutures can wrap around the expansion and locking mechanism 1104. Further details regarding various attachments techniques and mechanisms for attaching commissures to expansion and locking mechanisms are disclosed in U.S. Publication No. 2018/0325665; U.S. Publication No. 2019/0105153; U.S. Application No. 62/869,948; U.S. Application No. 62/813,643; and PCT Application No. PCT/US2019/61392, all of which documents are incorporated herein by reference. Any of the techniques and mechanisms disclosed in the prior documents can be used to connect the leaflets 1120 to the expansion and locking mechanisms 1104.

Referring again to FIG. 64, in the illustrated embodiment, the expansion and locking mechanisms 1104 are coupled to the frame 1102 such that the commissure openings 1112 extend past the outflow end 1154 of the frame 1102. This configuration advantageously allows the leaflets 1120 to be coupled to the expansion and locking mechanisms 1104 after the expansion and locking mechanisms 1104 have been coupled to the frame 1102. In other embodiments, the expansion and locking mechanisms 1104 can be coupled to the frame 1102 such that the commissure openings 1112 do not extend past the outflow end 1154 of the frame 1102. In such embodiments, the leaflets 1120 may be coupled to the expansion and locking mechanisms 1104 prior to coupling the expansion and locking mechanisms 1104 to the frame 1102.

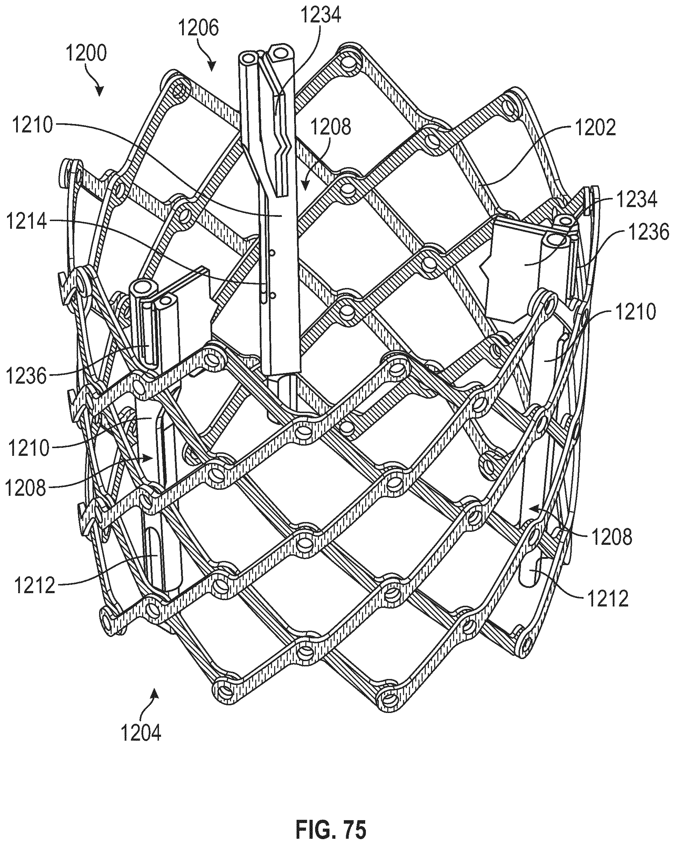

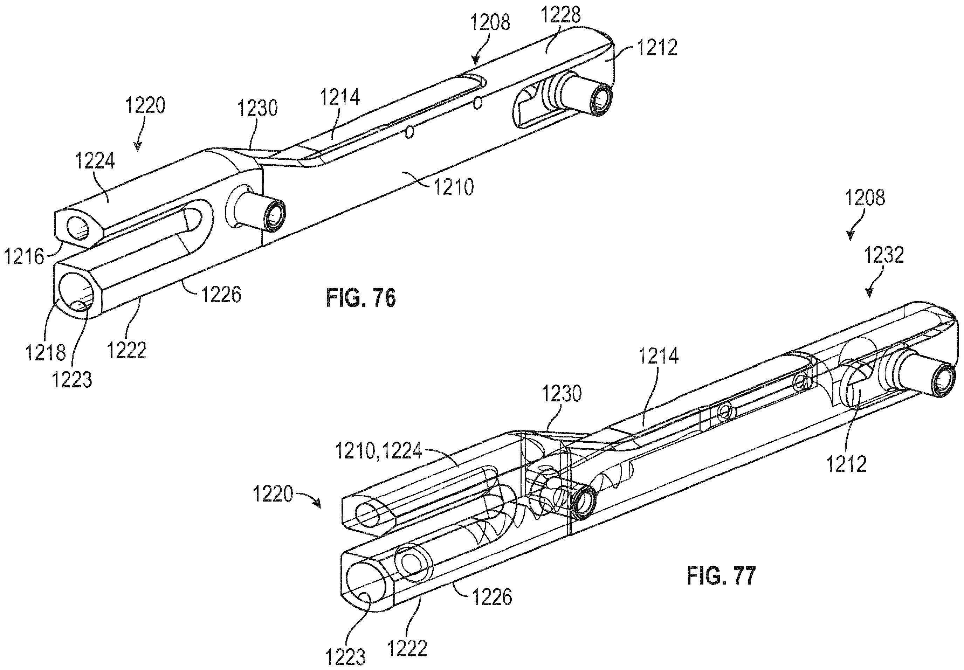

FIGS. 75-80 illustrate another embodiment of a prosthetic valve 1200 having a frame 1202, an inflow end 1204, an outflow end 1206, and a plurality of expansion and locking mechanisms 1208. The expansion and locking mechanisms 1208 can be similar to expansion and locking mechanisms 1104 described above (e.g., including an outer member 1210, inner member 1212, and locking member 1214, as shown in FIG. 76). FIG. 75 shows three expansion and locking mechanisms 1208 mounted to a frame 1202, which is in a radially expanded configuration, though other embodiments can comprise any number of expansion and locking mechanisms 1208.

Referring now to FIG. 76, each expansion and locking mechanism 1208 can comprise an outer member 1210. Similar to outer member 1106, outer member 1210 has a commissure opening 1216 extending to an outflow edge 1218 of the outer member 1210, however, as shown in FIG. 76, outer member 1210 has a non-uniform width along a length of the outer member 1210. The commissure opening 1216 separates the outflow end portion 1220 of the outer member 1210 into a first portion 1222 and a second portion 1224. The first portion 1222 can comprise a first bore 1223, into which the inner member 1212 can at least partially extend. In some embodiments, as illustrated, the second portion 1224 can comprise a second bore 1225,

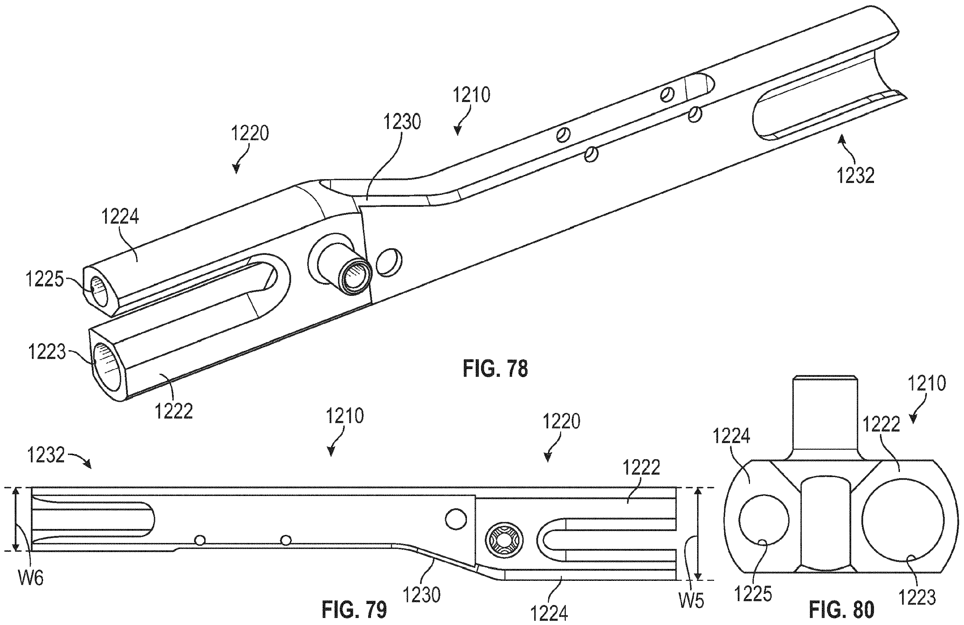

The first side wall 1226 of the outer member 1210 can be substantially straight, and the second side wall 1228 can comprise an angled portion 1230 that extends away from a longitudinal axis of the expansion and locking mechanism 1208. Accordingly, as best shown in FIG. 79, the outflow end portion 1220 of the outer member 1210 can have a first width W₅ and the inflow end portion 1232 can have a second width W₆ narrower than W₅. This configuration advantageously allows the outflow end portion 1220 to be sized to accommodate a commissure assembly (e.g., tabs 1234 of adj acent leaflets and a wedge 1236, as shown in FIG. 75) while allowing the inflow end portion 1232 to be smaller relative to the outflow end portion 1220 such that the inner member 1212, locking member 1214, and inflow end portion 1232 of the outer member 1210 can be sized such as they would be for an expansion and locking mechanism that does not include a commissure opening, such as expansion and locking mechanism 710.

A commissure assembly can be mounted to a respective expansion and locking mechanism 1208 in the same manner as described previously for expansion and locking member 1104 with reference to FIGS. 70-71. Though the illustrated embodiment of FIGS. 75-80 shows an "open" commissure opening (e.g., wherein the commissure opening extends to the outflow edge of the outer member), it should be appreciated that, in other embodiments, the expansion and locking mechanism 1208 can comprise a "closed" commissure opening.

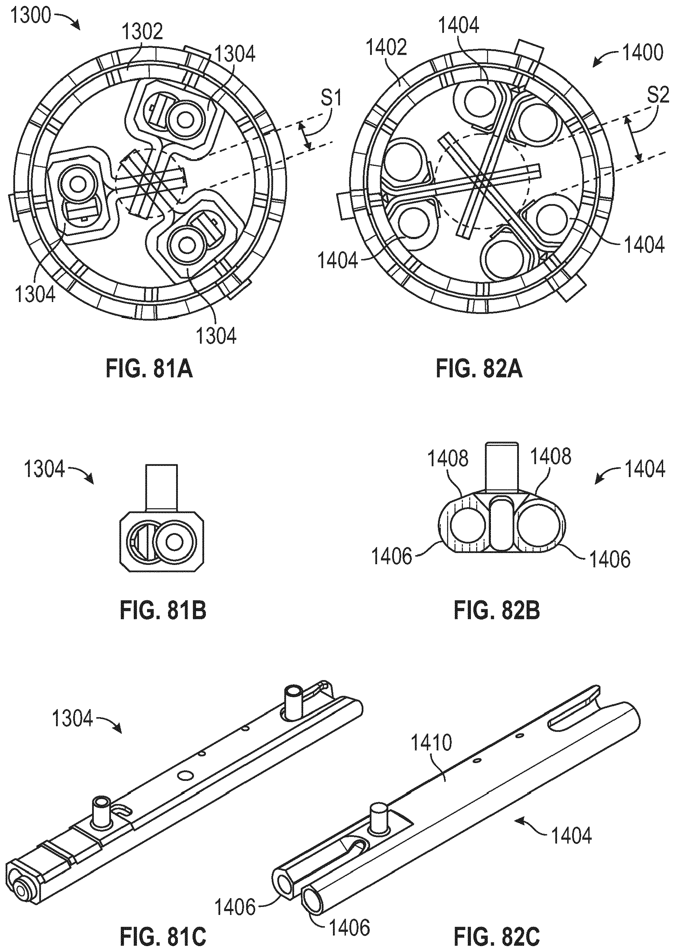

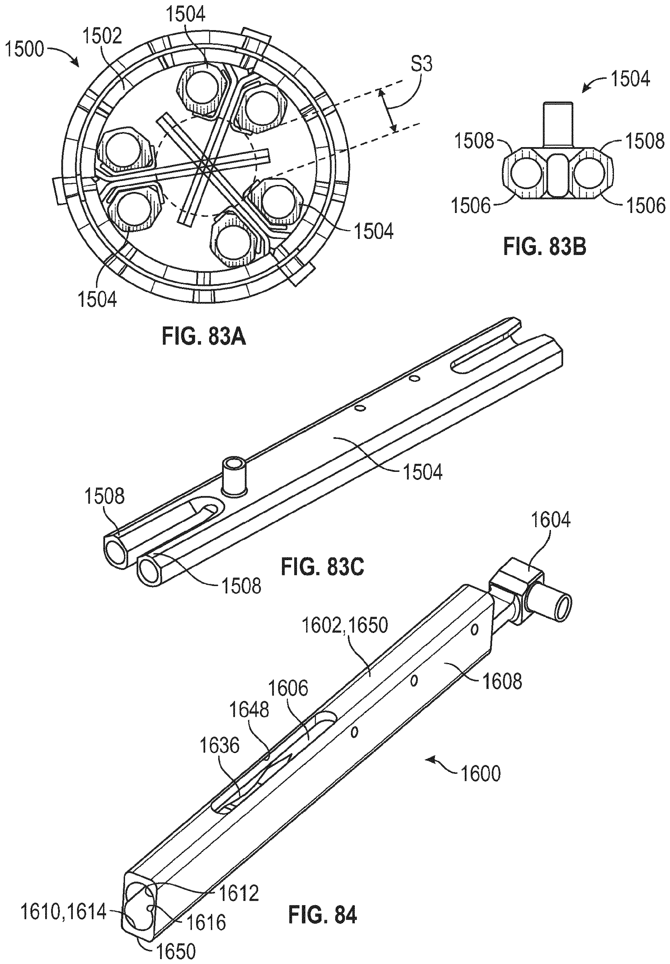

In some or all of the previously described embodiments, the outer profile of the expansion and locking mechanisms (e.g., expansion and locking mechanisms 1006, 1104, 1208) can comprise chamfered or rounded edges. FIGS. 81A-81C illustrate an exemplary prosthetic valve 1300 including frame 1302 and three rectangular expansion and locking mechanisms 1304, similar to expansion and locking mechanisms 710 described previously. FIG. 81A shows the prosthetic valve 1300 in the compressed configuration. As shown, the size and/or shape of the expansion and locking mechanisms 1304 results in relatively narrow spaces S₁ between the expansion and locking mechanisms 1304. FIGS. 82A-82C illustrate an exemplary prosthetic valve 1400 having frame 1402 and including three expansion and locking mechanisms 1404 having rounded inner radial 1406 (FIG. 82B). As shown in FIG. 82A, the rounded edges result in greater spaces S₂ between the expansion and locking mechanisms 1404. The expansion and locking mechanisms 1404 can further comprise rounded radially outer edges 1408 (FIG. 82B) which allow the radially outer surface 1410 (FIG. 82C) of the expansion and locking mechanism 1404 to conform to the contour of the frame 1402. Likewise, FIGS. 83A-83C illustrate an embodiment wherein prosthetic valve 1500 having frame 1502 comprises three expansion and locking mechanisms 1504 having chamfered edges. The chamfered radially inner and radially outer edges 1506, 1508 (FIG. 83B) result in greater spacing S₃ (relative to prosthetic valve 1300 shown in FIGS. 81A-81C) between the expansion and locking mechanisms 1504.

In some embodiments, the spacing S₂ can be greater than the spacing S₃ which can be greater than the spacing S₁. For example, the spacing S₂ can be about 1.26 mm, the spacing S₃ can be about 1.24 mm, and the spacing S₁ can be about 0.75 mm. The greater (relative to Si) spacing provided in prosthetic valves 1400 and 1500 advantageously provides fewer restrictions on the size of the leaflets and/or the size of the expansion and locking mechanisms 1404, 1504.

FIGS. 84-88 illustrate another embodiment of an expansion and locking mechanism for use with a prosthetic valve. Locking mechanism 1600 includes an outer member 1602, an inner member 1604, and a locking member 1606. Referring to FIG. 84, expansion and locking mechanism 1600 can be similar to expansion and locking mechanism 710 described previously, except that the locking member 1606 can be at least partially enclosed by the outer member 1602. Expansion and locking mechanisms 1600 can have any or all of the previously-disclosed features. For example, the outer member 1602 can have a rounded or chamfered outer profile and/or can comprise a U-shaped recess in the outer wall 1608 similar to recess 738, etc.

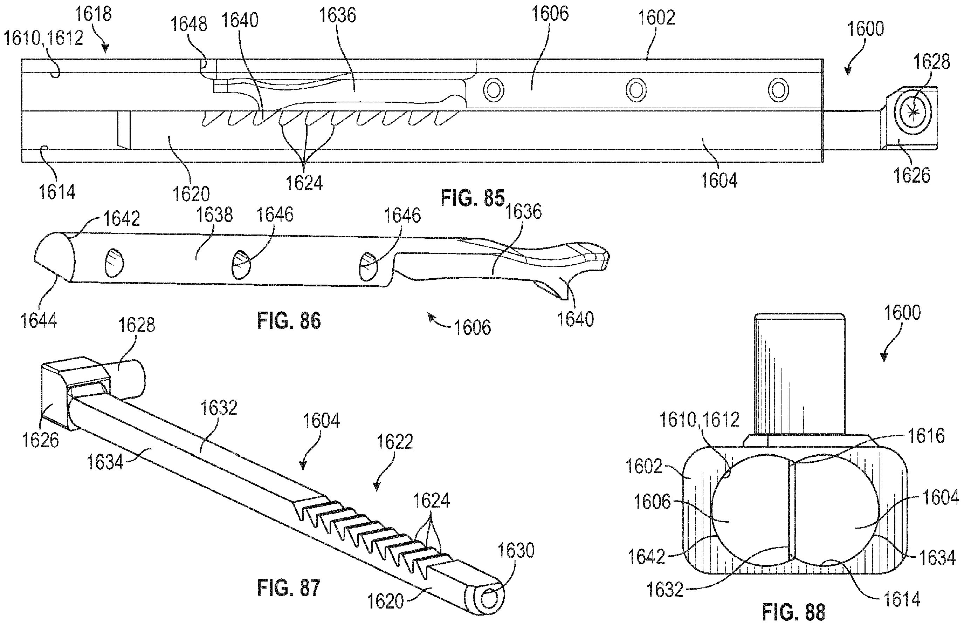

The outer member 1602 can include a cavity or bore 1610. The bore 1610 can have a shape in cross-section similar to the outer profile of the number '8.' That is, the bore 1610 can have a first portion 1612 and a second portion 1614 separated by a neck portion 1616. As best seen in FIG. 88, the neck portion 1616 can have a thinner width than the first and second portions 1612, 1614. As shown in FIG. 85, the locking member 1606 can be disposed in the first portion 1612, and at least a portion of the inner member 1604 can be disposed in the second portion 1614. The second portion 1614 can further be configured to guide an actuation member of the delivery apparatus into position (e.g., through a proximal or outflow end 1618 of the outer member 1602) such that it can couple an outflow end 1620 of the inner member 1604 to actuate the inner member 1604, thereby radially expanding and/or collapsing the prosthetic valve.

Referring to FIG. 87, the inner member 1604 can be an elongated member comprising a linear rack 1622 having a plurality of teeth 1624. The linear rack 1622 can extend a portion of the length of the inner member 1604 adjacent the outflow end portion 1620 and/or the inflow end portion 1626. In other embodiments, the linear rack 1622 can extend substantially the entire length of the inner member 1604. The inflow end portion 1626 of the inner member 1604 can include a fastener 1628 and the outflow end portion 1620 can include an engagement portion 1630 for coupling a delivery apparatus. The inner member 1604 can have a semi-circular or "D" shape in cross-section that extends at least partially along the length of the inner member 1604 such that the inner member comprises a flat surface 1632 (on which the linear rack 1622 is disposed) and a curved surface 1634.

As best seen in FIG. 86, the locking member 1606 can comprise an elongated body including a first end portion 1636 and a second end portion 1638. The first end portion 1636 can be configured as a bendable pawl including a locking tooth 1640 configured to engage the linear rack 1622 of inner member 1604. The locking tooth 1640 can extend toward the linear rack 1622 and can have a shape that is complimentary to the shape of the teeth 1624, such that the locking tooth 1640 allows sliding movement of the inner member 1604 in one direction relative to the locking member 1606 (e.g., to expand the prosthetic valve) and resists sliding movement of the inner member in the opposite direction (e.g., to compress the prosthetic valve) when the locking tooth 1640 is in engagement with one of the teeth 1624 of the linear rack. The second end portion 1638 of the locking member 1606 can have a semi-circular or "D" shape in cross-section such that it comprises a curved surface 1642 and a flat surface 1644. The second end portion 1638 can further comprise one or more apertures 1646 configured such that the locking member 1606 can be coupled to the outer member 1602 via the apertures 1646, such as described with respect to locking member 718 and outer member 712 previously.

As best seen in FIG. 84, the outer member 1602 can comprise an opening 1648 in one of the side walls 1650 aligned with the first end portion 1636 of the locking member 1606. The opening 1648 can extend through a thickness of the side wall 1650 and can have an elongated oval shape. The opening 1648 can be sized such that the first end portion 1636 of the locking member 1606 can deflect into the opening 1648 when, for example, the locking tooth 1640 is engaged with the linear rack 1622.

When the expansion and locking mechanism 1600 is assembled, the locking member 1606 can be disposed in the first portion 1612 of the bore 1610 and the inner member 1604 in the second portion 1614 of the bore such that the flat surface 1644 of the locking member 1606 faces the flat surface 1632 of the inner member 1606. The curved surfaces 1634, 1642 of the inner member 1604 and the locking member 1606 can abut the inner edges of the first and second portions 1612, 1614 of the bore 1610, respectively. In some embodiments, the flat surfaces 1632, 1644 can be spaced apart from one another such that they do not contact one another. In other embodiments, the flat surfaces 1632, 1644 can contact one another. In embodiments wherein the surfaces 1632, 1644 contact one another, each surface can be polished to have low coefficients of friction, such that the surfaces can slide easily along one another, and/or each surface can be coated with one or more lubricious, low-friction layers.

Advantageously, the disclosed configuration ensures that at least a portion of the locking member 1606 is fully retained within the outer member 1602, thereby preventing or mitigating the risk of spontaneous detachment of the locking member 1606 from the expansion and locking mechanism 1600.

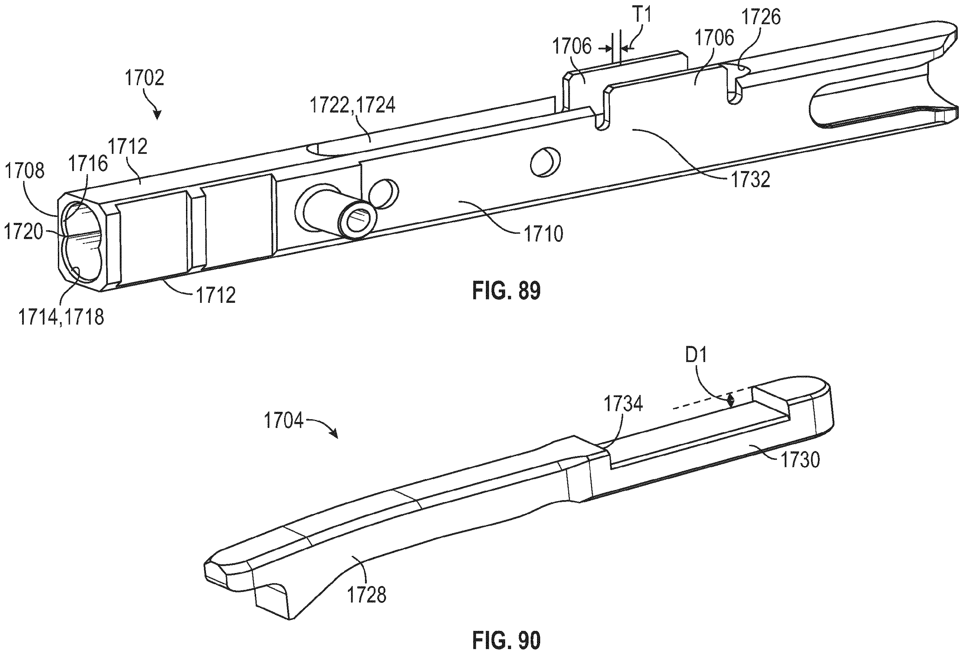

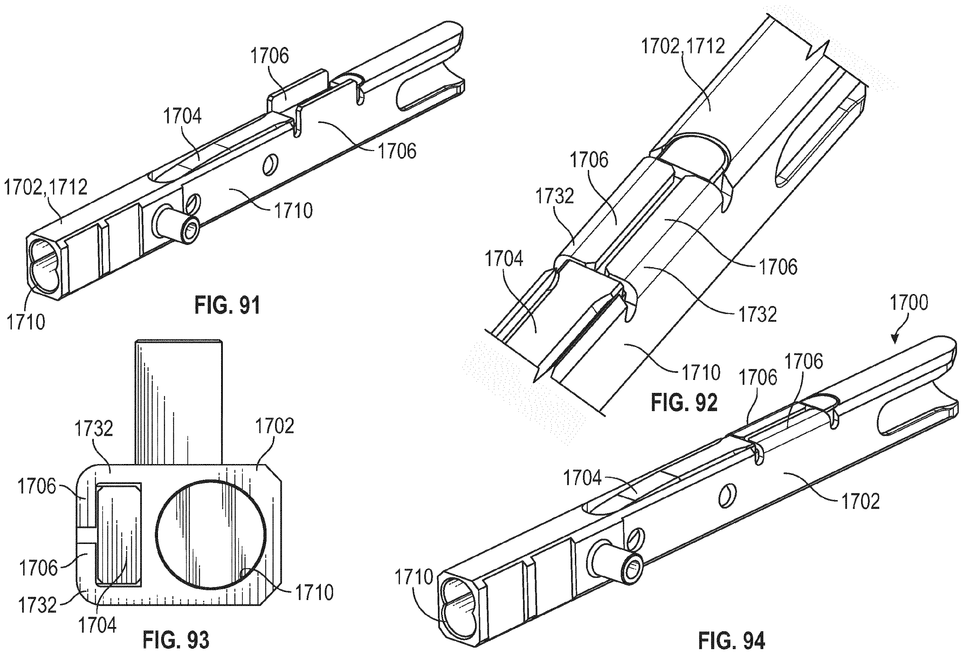

FIGS. 89-94 illustrate another embodiment of an expansion and locking mechanism 1700 (see FIG. 94) including an outer member 1702, an inner member (not shown), and a locking member 1704. Expansion and locking mechanism 1700 can be similar to expansion and locking mechanisms 710 and 1600 described previously, except that the locking member 1704 can be at least partially enclosed by the outer member 1702 via extension portions 1706. Expansion and locking mechanisms 1700 can have any or all of the previously-disclosed features. For example, the outer member 1702 can have a rounded or chamfered outer profile and can comprise a commissure opening, etc.

Referring to FIG. 89, the outer member 1702 comprises an inner wall 1708 (e.g., facing radially inwardly toward the valvular structure of the prosthetic valve when the prosthetic valve is assembled), an outer wall 1710 (e.g., facing radially outwardly toward the frame when the prosthetic valve is assembled), and two side walls 1712. The walls define a cavity or bore 1714 having an '8'-shaped cross-section including a first portion 1716 and a second portion 1718 separated by a neck portion 1720. The outer member 1702 can further comprise an opening 1722 extending through a thickness of a side wall 1712. The opening 1722 can have a first portion 1724 aligned with a first end portion or pawl 1728 (FIG. 90) of the locking member 1704 and a second portion 1726 aligned with a second end portion 1730 (FIG. 90) of the locking member 1704.

The inner and outer walls 1708, 1710 of the outer member 1702 can each include a respective lateral extension 1706. The lateral extensions 1706 can be aligned with the second end portion 1726 of the opening 1722 and can be used to retain the locking member 1704 within the opening 1722. The extensions 1706 can comprise a bendable portion 1732 configured to allow them to be bent such that they extend over the opening 1722, as shown in FIG. 92. In the illustrated embodiment, the lateral extensions 1706 are aligned with one another along the length of the outer member 1702 and each extension has a rectangular shape wherein the length of the extension (e.g. along the longitudinal axis of the outer member) is greater than the width. However, in other embodiments, the lateral extensions 1706 can be offset from one another along the length of the outer member 1702 and can have any of various shapes, for example, square, triangular, ovoid, t-shaped, etc. In some embodiments, the extensions 1706 can have a rectangular shape wherein the width of the extension is greater than the length. In such embodiments, the extensions 1706 can be offset from one another along the length of the outer member 1702 such that the extensions 1760 extend past one another but do not overlap when in the bent position.

Referring to FIG. 90, the locking member 1704 can comprise a recess 1734 disposed in the second end portion 1730 of the locking member 1704. The recess 1734 can be sized such that the lateral extensions 1706 can be disposed within the recess 1734, as shown in FIG. 92. The recess 1734 can have a depth D₁ substantially equivalent to a thickness T₁ (FIG. 89) of the lateral extensions 1706, such that when the lateral extensions 1706 are disposed within the recess 1734, the lateral extensions 1706 do not protrude out of the recess 1734.

Referring to FIGS. 91-94, the locking member 1704 can be coupled to the outer member 1702 in the following exemplary manner. As shown in FIG. 91, the locking member 1704 can be disposed within the opening 1722 such that the pawl 1728 is aligned with the first portion 1724 of the opening 1722 and such that the second portion 1730 is aligned with the second portion 1726. When disposed in such a manner, the lateral extensions 1706 align with the recess 1734 of the locking member 1704.

Referring now to FIG. 92, an inwardly directed force (e.g., toward a longitudinal axis of the expansion and locking mechanism 1700) can be applied to the lateral extensions 1706, thereby deforming the bendable portion 1732 such that the lateral extensions 1706 extend into the recess 1734, thereby securing the locking member 1704 to the outer member 1702.

This configuration can advantageously simplify manufacturing, for example, by allowing much simpler processing and machining procedures (such as Swiss-type and milling procedures) to be used. Additionally, this configuration avoids small fasteners, which can in some instances be difficult to manufacture and assemble, and additionally avoids welding, which can be inaccurate and impractical at such small sizes. Moreover, the recess 1734 in the locking member 1704 and the lateral extensions 1706 are easier to manufacture than components having more complex shapes.

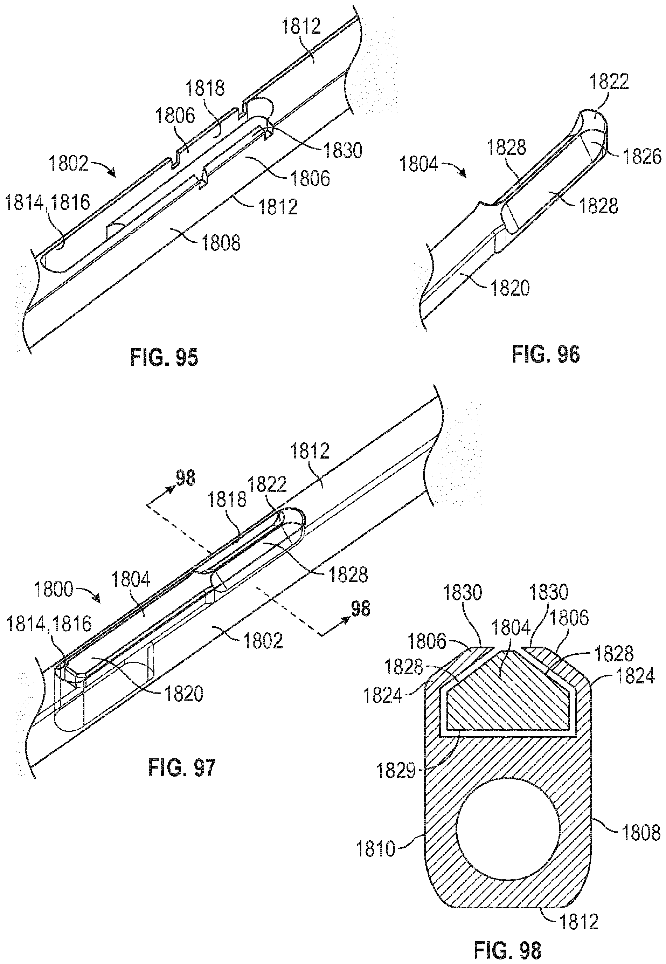

FIGS. 95-98 illustrate another embodiment of an expansion and locking mechanism 1800 (FIG. 97) similar to expansion and locking mechanism 1700 described previously. Expansion and locking mechanism 1800 can comprise an outer member 1802 and a locking member 1804. The expansion and locking mechanism 1800 can further comprise an inner member (such as inner member 1108 or 1604 described previously), although this component is omitted for purposes of illustration. Expansion and locking mechanism 1800 can have any or all of the previously-disclosed features. For example, though not shown in the illustrated embodiment, the outer member 1802 can comprise a commissure opening (such as commissure openings 1022 or 1112) and/or a recess (such as recess 738).

Referring to FIG. 95, the outer member 1802 can include one or more lateral extensions 1806 configured to couple the locking member 1804 to the outer member 1802. The outer member 1802 can comprise an inner wall 1808 (e.g., facing radially inwardly toward the valvular structure of the prosthetic valve when the prosthetic valve is assembled), an outer wall 1810 (FIG. 98) (e.g., facing radially outwardly toward the frame when the prosthetic valve is assembled), and two side walls 1812. The outer member 1802 can further comprise an opening 1814 extending through a thickness of a side wall 1812. The opening 1814 can have a first portion 1816 and a second portion 1818. When the locking member 1804 is disposed within the opening 1814, as shown in FIG. 97, a first end portion 1820 of the locking member 1804 can be aligned with the first portion 1816 of the opening and a second end portion 1822 of the locking member can be aligned with the second portion 1818 of the opening.

The inner and outer walls 1808, 1810 of the outer member 1802 can each include a respective lateral extension 1806. The lateral extensions 1806 can be aligned with the second end portion 1820 of the opening 1814 and can be used to retain the locking member 1804 within the opening 1814. The extensions 1806 can comprise a bendable portion 1824 (FIG. 98) configured to allow them to be bent such that they extend over the opening 1814 at an angle, as shown in FIG. 98. In the illustrated embodiment, the lateral extensions 1806 are aligned with one another along the length of the outer member 1802 and each extension has a rectangular shape wherein the length of the extension 1806 (e.g., along a longitudinal axis of the outer member 1802) is greater than the width. However, in other embodiments, the lateral extensions 1806 can be offset from one another along the length of the outer member 1802 and can have any of various shapes.

FIG. 96 illustrates an embodiment of a locking member 1804 for use with expansion and locking mechanism 1800. As mentioned previously, the locking member 1804 can comprise a first end portion 1820 and a second end portion 1822. The first end portion 1820 can be configured a pawl portion having a locking tooth and a disengagement tooth, similar to locking member 1704 described previously, although these components are omitted for purposes of illustration.

The second end portion 1822 can comprise a recess 1826 including two angled surfaces 1828. The angled surfaces 1828 can be angled toward a longitudinal axis of the locking member 1804 relative to a base surface 1829 of the locking member, such that the locking member 1804 has a substantially triangular shape in cross-section, with the apices of the triangle cut off. When expansion and locking mechanism 1800 is assembled, the lateral extensions 1806 of the outer member 1802 can abut or engage the angled surfaces 1828, thereby retaining the locking member 1804 within the opening 1814 of the outer member 1802. In some embodiments, each lateral extension 1806 can comprise a chamfered edge surface 1830. The chamfered edge surfaces 1830 can be configured such that when the lateral extensions 1806 are disposed against the angled surfaces 1828, the lateral extensions 1806 do not protrude out of the recess 1826 (e.g., do not extend past the side surface 1812 of the outer member 1802).

The locking member 1804 can be coupled to the outer member 1802 in the following exemplary manner. As shown in FIG. 97, the locking member 1804 can be disposed within the opening 1814 such that the first end portion 1820 is aligned with the first portion 1816 and such that the second end portion 1822 is aligned with the second portion 1818 of the opening. When disposed in such a manner, the lateral extensions 1806 align with the angled surfaces 1828 of the locking member 1804.

An inwardly-directed force (e.g., toward a longitudinal axis of the expansion and locking mechanism 1800) can be applied to the lateral extensions 1806, thereby deforming the bendable portion 1824 such that the lateral extensions 1806 abut the angled surfaces 1828, as shown in FIG. 98, thereby securing the locking member 1804 to the outer member 1802. Though FIG. 98 shows the lateral extensions 1806 spaced apart from the angled surfaces 1828, it should be understood that the lateral extensions 1806 can abut and frictionally engage the angled surfaces 1828 to prevent or mitigate movement of the locking member 1804 relative to the outer member 1802.

This configuration can advantageously simplify manufacturing, for example, by allowing much simpler processing and machining procedures (such as Swiss-type and milling procedures) to be used. This configuration further avoids small fasteners, which can in some instances be difficult to manufacture and assemble, and additionally avoids welding, which can be inaccurate and impractical at such small sizes. The recess 1826 in the locking member 1804 and the lateral extensions 1806 are easier to manufacture than components having more complex shapes. Moreover, the angled surfaces 1828 of the recess 1826 create a larger contact area between the lateral extensions 1806 and the locking member 1804, which can prevent or mitigate movement of the locking member 1804 relative to the outer member 1802.

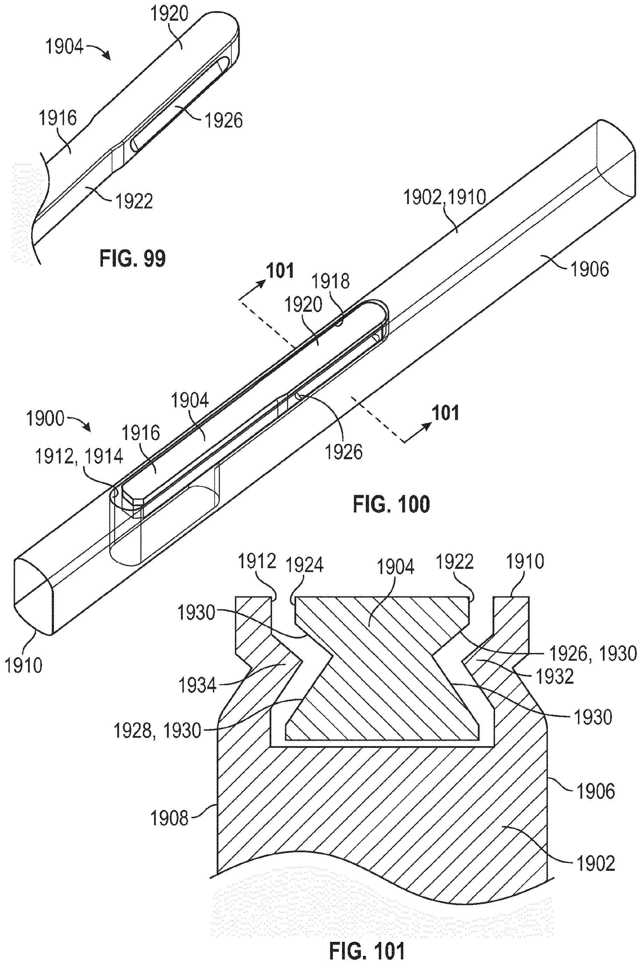

FIGS. 99-101 illustrate another embodiment of an expansion and locking mechanism 1900 (FIG. 100) for use with a prosthetic heart valve. Expansion and locking mechanism 1900 is similar to expansion and locking mechanisms 1700 and 1800, except that the outer member 1902 does not comprise lateral extensions and the locking member 1904 can be coupled to the outer member 1902 via one or more recesses in the locking member 1904. Expansion and locking mechanism 1900 can have any or all of the previously-disclosed features. For example, though not shown in the illustrated embodiment, the outer member 1902 can comprise a commissure opening (such as commissure openings 1022 or 1112) and/or a recess (such as recess 738).

Referring to FIG. 100, the outer member 1902 can comprise an inner wall 1906 (e.g., facing radially inwardly toward the valvular structure of the prosthetic valve when the prosthetic valve is assembled), an outer wall 1908 (FIG. 101) (e.g., facing radially outwardly toward the frame when the prosthetic valve is assembled), and two side walls 1910. The outer member 1902 can further comprise an opening 1912 extending through a thickness of a side wall 1910. The opening 1912 can have a first portion 1914 aligned with a first end portion 1916 of the locking member 1904 and a second portion 1918 aligned with a second end portion 1920 of the locking member 1904.

Referring to FIG. 99, the locking member 1904 can comprise a first end portion 1916 and a second end portion 1920, and can have an inner wall 1922 (e.g., facing the valvular structure of the prosthetic valve) and an outer wall 1924 (FIG. 101) (e.g., facing the frame of the prosthetic valve). The first end portion 1916 can be configured as a pawl having a locking tooth and a disengagement tooth, similar to locking member 1704 described previously, although these components are omitted for purposes of illustration.

The second end portion 1920 can comprise first and second recesses 1926 and 1928 (FIG. 101) disposed in the inner and outer walls 1922, 1924 respectively. Each recesses 1926, 1928 can have an elongated, oval shape extending at least partially along the length of the second end portion 1920. As best seen in FIG. 101, in the illustrated embodiment, each recess 1926, 1928 can comprise two angled surfaces 1930 disposed such that the recess has a V-shape in cross-section, with the opening of the V facing the inner or outer wall 1922, 1924, respectively. However, in other embodiments, the recesses 1926, 1928 can have any of various shapes.

The locking member 1904 can be coupled to the outer member 1902 in the following exemplary manner. As shown in FIG. 100, the locking member 1904 can be disposed within the opening 1912 such that the pawl (not shown) is aligned with the first portion 1914 of the opening 1912 and such that the recesses 1926, 1928 are aligned with the second portion 1918. An inwardly-directed force (e.g., toward a longitudinal axis of the expansion and locking mechanism 1900) can be applied to inner and outer walls 1906, 1908 of the outer member, deforming the inner and outer walls 1906, 1908 into respective recesses 1926, 1928 to form respective protrusions 1932, 1934, as shown in FIG. 101. The protrusions 1932, 1934 couple the locking member 1904 to the outer member 1902.

In the illustrated embodiment, the protrusions 1932, 1934 have a V-shape corresponding to the V-shape of the recesses 1926, 1928. However, in other embodiments, the protrusions can have any of various shapes corresponding to the shape of the recesses 1926, 1928. Though FIG. 101 shows the protrusions 1932, 1934 spaced apart from the recesses 1926, 1928, it should be understood that the lateral extensions 1806 can abut and frictionally engage the angled surfaces 1828 to prevent or mitigate movement of the locking member 1804 relative to the outer member 1802.

This configuration can advantageously simplify manufacturing, for example, by allowing much simpler processing and machining procedures (such as Swiss-type and milling procedures) to be used. This configuration further avoids small fasteners, which can in some instances be difficult to manufacture and assemble, and additionally avoids welding, which can be inaccurate and impractical at such small sizes. The recesses 1926, 1928 in the locking member 1904 are easier to manufacture than components having more complex shapes. Moreover, the angled surfaces 1930 within each recess 1926, 1928 create a larger contact area between the protrusions 1932, 1934 and the locking member 1904, which can prevent or mitigate movement of the locking member 1904 relative to the outer member 1902.

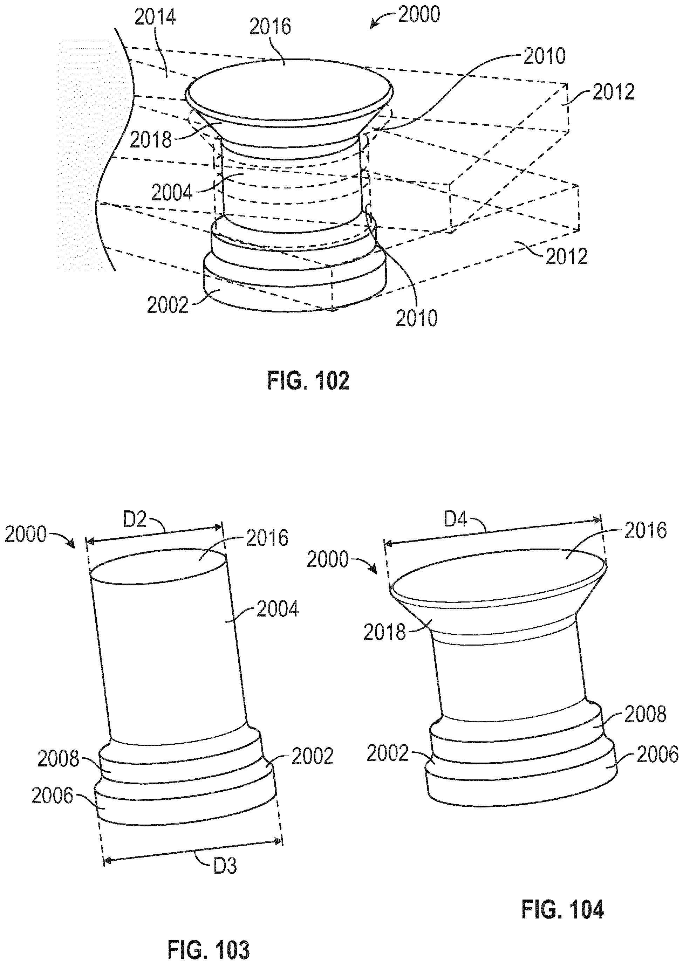

Referring now to FIGS. 102-105, as mentioned previously, the outer and inner members of a respective expansion and locking mechanism can each comprise a respective fastener (e.g., expansion and locking mechanism 710, described previously, includes fasteners 730 and 732). An exemplary fastener 2000 can be similar to the fasteners described previously except that fastener 2000 can be radially riveted to retain the fastener within the openings in the frame, as described in more detail below.

Referring now to FIG. 103, each fastener 2000 can include a base portion 2002 and a body portion 2004. The body portion 2004 can have a diameter D₂ and the base portion 2002 can have a diameter D₃ greater than the diameter D₂ of the body portion. In the illustrated embodiment, the base portion 2002 can have a tiered or stepped configuration including a first step 2006 and a second step 2008 having a diameter smaller than the first step 2006. This configuration can advantageously allow the base portion 2002 to be seated within a correspondingly stepped recess in, for example, an internal surface of the expansion and locking mechanism. In other embodiments, the base portion 2002 can include any number of steps or tiered surfaces corresponding to a recess in the corresponding .

In some embodiments, the fastener 2000 can be formed as a separate component coupled to a radially outer wall of the expansion and locking mechanism. For example, the body portion 2004 can extend through an aperture in the outer wall and the base portion 2002 can abut the radially inner surface of the outer wall. In some embodiments, the radially inner surface of the outer wall can comprise a recess in which the base portion 2002 of the fastener 2000 can be disposed. In other embodiments, the fastener 2000 can be formed integrally with the expansion and locking mechanism and can, for example, extend radially from an outer surface of the radially outer wall.

In the illustrated embodiment, the fastener 2000 is a solid piece of material. Such a configuration provides greater retention strength and improved performance. However, in other embodiments, the fastener can be configured as a hollow tube, see, for example, fasteners 730 and 732.

Referring now to FIG. 102, when an expansion and locking mechanism (not shown) is coupled to the frame, each fastener 2000 can extend through corresponding apertures 2010 at a junction of two overlapping struts 2012 of the frame 2014 and can serve as a pivot pin around which the two struts 2012 can pivot relative to one another and the expansion and locking mechanism.

The fastener 2000 can be secured within the apertures 2010 in the following exemplary manner. Once the fastener 2000 has been disposed within the apertures 2010, a radially outer end surface 2016 of the body portion 2004 can be radially riveted to form a flanged portion 2018, as shown in FIG. 104. The flanged portion 2018 can have a diameter D₄ greater than the diameter D₂ of the body portion 2004 and greater than the diameter of the apertures 2010, such that the fastener 2000 is retained within the apertures 2010 and cannot pass through the apertures, as shown in FIG. 102.

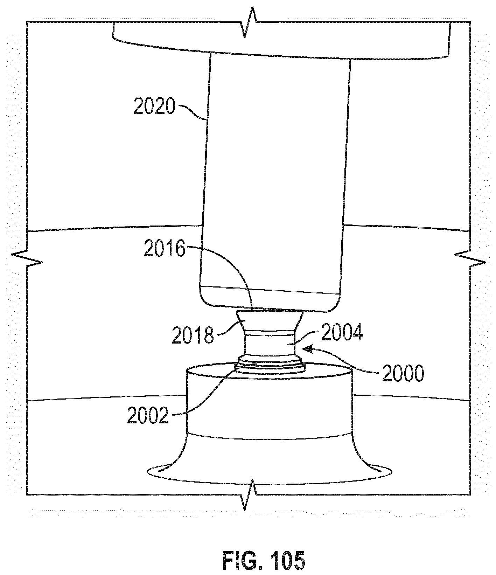

Referring to FIG. 105, radial riveting can be performed using a riveting member 2020 (also referred to as a punch). The riveting member 2020 can rotate around the fastener 2000, applying pressure to the radially outer end surface 2016 in a rosette shaped path (e.g., a hypocycloid path) to gently deform the fastener 2000, thereby forming the flanged portion 2018. The longitudinal axis of the riveting member 2020 is disposed at an angle relative to the riveting surface (e.g., the radially outer end surface 2016 of the fastener 2000). The amount of applied force, the length of the riveting process, and the shape of the riveting member 2020 can each be modified in order to vary the diameter, thickness, and/or shape of the flanged portion 2018.

Radial riveting has various advantages. Namely, radial riveting applies very little lateral force, mitigating the need to clamp or fix the fastener 2000 in place during the riveting process, and applies very little axial force, thereby mitigating the risk of damaging the components surrounding the fastener (such as struts 2012). Moreover, since radial riveting is a cold-forming process, the flanged portion 2018 can be formed without deforming or swelling the remainder of the fastener body 2004. The radial riveting process can further produce a smooth, finished surface on the flanged end portion 2018, mitigating potential damage if the fastener 2000 comes in contact with the sheath of the delivery apparatus during delivery of the prosthetic valve and/or comes in contact with the native anatomy of the implantation site. This configuration can advantageously simplify assembly of a prosthetic valve, for example, by allowing much simpler processing and machining procedures to be used. This configuration further avoids impact punching, such as is performed on hollow tube fasteners having internal bores. Drilling internal bores can be difficult when components are very tiny.

Though the preceding description refers to fasteners 2000 coupled to an expansion and locking mechanism, it should be noted that fasteners such as fastener 2000 can be used at any junction between two struts to pivotably couple the struts together, and that the above-described processes can be used to retain the fasteners 2000 within any such junctions.

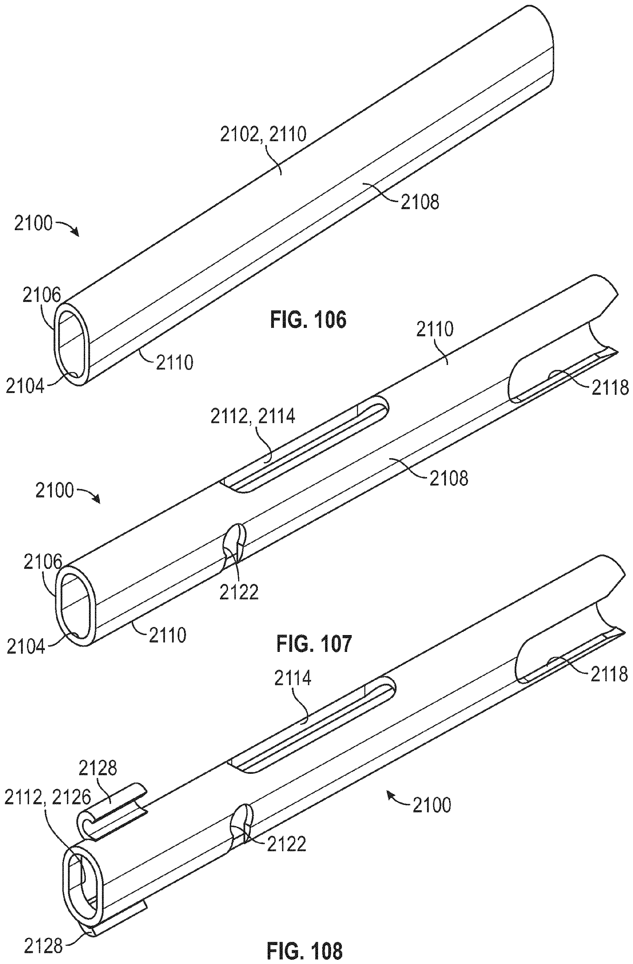

Referring now to FIGS. 106-115, in some embodiments, an outer member 2100 of an expansion and locking mechanism can be manufactured in the following exemplary manner. A tubular member 2102 such as a metallic tube can be cut to a selected length to serve as an outer member 2100 having an internal bore 2104. The tubular member can be squeezed or otherwise deformed such that it forms an elliptical, oval, square oval, or substantially rectangular shape in cross-section, as shown in FIG. 106. The outer member 2100 can generally comprise a radially inner wall 2106, a radially outer wall 2108, and first and second side walls 2110.

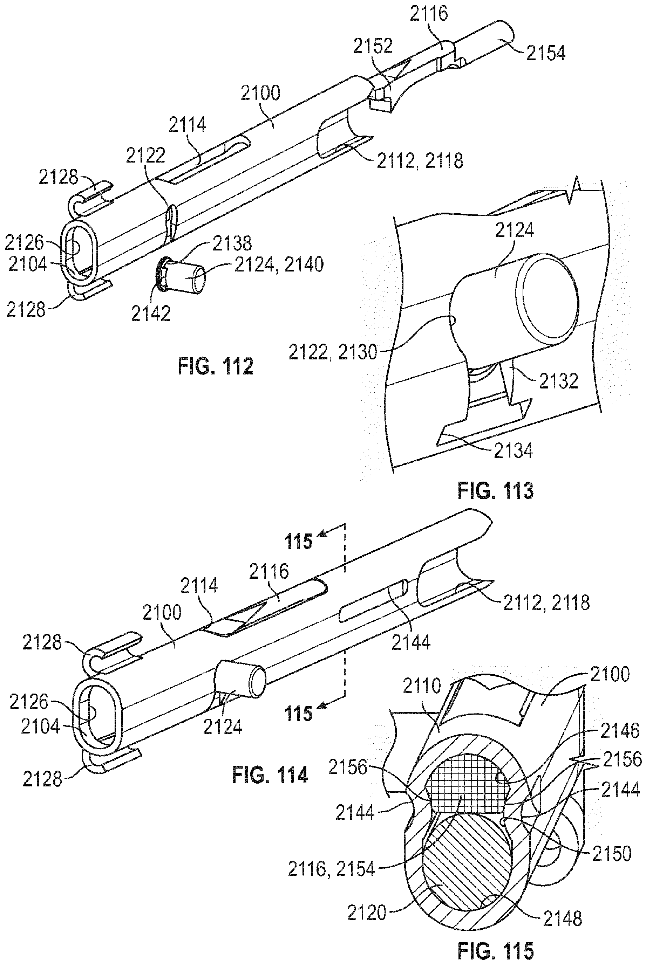

Referring to FIG. 107, one or more cutouts 2112 can be cut into the tube (e.g., via laser cutting). For example, the cutouts 2112 can include an opening 2114 in which the locking arm 2116 (FIG. 112) can be disposed, an inflow end cutout 2118 configured to guide and/or restrain the fastener of the inner member 2120 (FIG. 115), and a fastener opening 2122 in which the fastener 2124 (FIG. 112) of the outer member 2100 can be disposed. In the illustrated embodiment, the locking arm opening 2114 can be disposed in a side wall 2110, and the inflow cutout 2118 and the fastener opening 2122 can be disposed in the radially outer wall 2108. However, in other embodiments, the locking arm opening 2114 can be disposed in any wall.

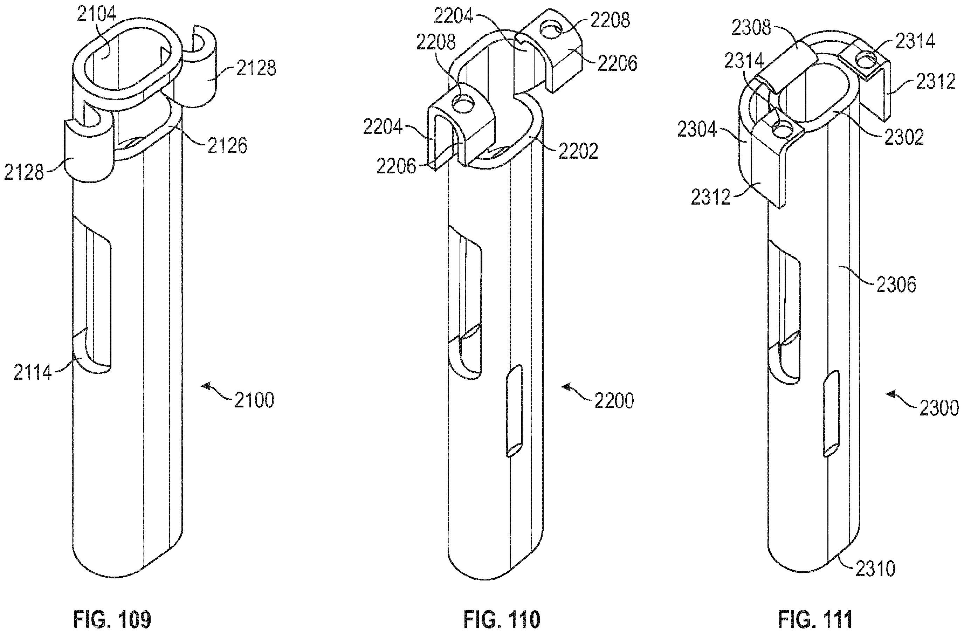

Referring now to FIG. 108, in some embodiments, such as the illustrated embodiment, the outer member 2100 can comprise an additional cutout 2112 configured as a commissure opening 2126. Portions of the outer member 2100 adjacent the commissure opening 2126 can be bent such that they curve away from a longitudinal axis of the outer member 2100 to form one or more commissure post arms 2128. Referring to FIG. 109, in some embodiments, the commissure opening 2126 formed is a "closed" commissure opening (e.g., similar to commissure opening 1022 described previously), however, in other embodiments, the commissure opening can be an "open" commissure opening (e.g., similar to commissure opening 1112 described previously).

FIGS. 110-111 illustrate alternative embodiments of commissure structural arrangements. For example, FIG. 110 illustrates an outer member 2200 including an "open" commissure opening 2202 wherein the opening extends to an outflow edge of the outer member 2200. In some such embodiments, first portions 2204 of the outer member 2200 can be bent away from a longitudinal axis of the outer member, and second portions 2207 may additionally be bent in an axial direction. Post-arm apertures 2209 can be drilled through the second portions 2207. In another example, FIG. 111 illustrates an outer member 2300 wherein a radial cutout 2302 extends almost entirely around a perimeter of the outer member 2300 separating an outflow end portion 2304 from an inflow end portion 2306 and leaving one or more narrow neck portions 2308 connecting the outflow and inflow end portions 2304, 2306. The outflow end portion 2304 can be bent axially toward the inflow end 2310 of the outer member 2300 such that the outflow end portion 2304 surrounds the outer member 2300 connected by at least one neck portion 2308. One or more commissure post arms 2312 can be coupled to the outflow end portion 2304 and can include one or more post arm apertures 2314. The commissure post arms 2312 can be separate components coupled to the outflow end portion 2304 or, alternatively, can be portions of the outflow end portion 2304 configured as commissure post arms 2312.

Referring to FIG. 113, the fastener opening 2122 can comprise a main portion 2130, a guide portion 2132, and an entry portion 2134. The guide portion 2132 can be narrower than the entry portion 2134 and the main portion 2130. As shown in FIG. 112, the fastener 2124 can have a base portion 2138 having a first diameter and a body portion 2140 having a second diameter narrower than the first diameter, and can comprise one or more recessed portions 2142. The recessed portions 2142 can define a reduced diameter of the body portion 2140 along a first axis of the fastener 2124, while retaining the diameter of the body portion 2140 along a second axis of the fastener 2124 perpendicular to the first axis.

The fastener 2124 can be coupled to the outer member 2100 in the following exemplary manner. The base portion 2138 of the fastener 2124 can be inserted into the entry portion 2134 of the opening 2122 such that the recessed portions 2142 are aligned with the edges of the guide portion 2132 (e.g., such that the fastener 2124 is rotationally aligned with the fastener opening 2122). The fastener 2124 can be advanced through the guide portion 2132 by sliding the recessed portions 2142 along the guide portion 2132 until the fastener 2124 is disposed in the main portion 2130. The fastener 2124 can then be rotated until the recessed portions 2142 are rotationally offset from the guide portion 2132. When the fastener 2124 is rotationally offset from the fastener opening 2122, the body portion 2140 of the fastener 2124 is too wide to fit through the guide portion 2132, thereby securing the fastener within the opening 2122. In some embodiments, the guide portion 2132 can be deformed (e.g., by pinching and/or welding) after insertion of the fastener 2124 into the main portion 2130, to further retain the fastener 2124 within the main portion 2130.

Referring now to FIGS. 114-115, in some embodiments, after the locking member 2116 has been disposed within the locking member opening 2114, the outer member 2100 can be deformed to create one or more indentations 2144 configured to help retain the locking member 2116 in place. As shown in FIG. 115, the indentations 2144 can define a first portion 2146 and a second portion 2148 separated by a neck portion 2150 in the inner bore 2104 of the outer member 2100. The locking member 2166 can be disposed in the first portion 2146, and the inner member 2120 can be disposed at least partially within the second portion 2148.

The indentations 2144 can be formed on the radially inner and/or radially outer walls 2106, 2108. In the illustrated embodiment, as shown in FIG. 112, a first portion of the locking member 2116 can comprise a pawl 2152 and a second portion 2154 of the locking member 2116 can have a semi-circular shape in cross-section, with chamfered corner portions 2156 (FIG. 115). As shown in FIG. 115, the first portion 2146 of the inner bore 2104 can have a cross-sectional shape corresponding to that of the second portion 2154 of the locking member 2116. The indentations 2144 can abut the chamfered corner portions 2156, pressing the locking member 2116 against the side wall 2110 and retaining the locking member 2116 in position relative to the outer member 2100. In other embodiments, the second portion 2154 of the locking member 2116 and the first portion 2146 of the inner bore 2104 can have any of various other corresponding shapes in cross-section. The second portion 2148 of the inner bore 2104 can be shaped to accommodate at least a portion of the inner member 2120. In some embodiments, such as the illustrated embodiment, the second portion 2148 can be larger than the first portion 2146, and can have a different shape than the first portion 2146. However, in other embodiments, the first and second portions 2146, 2148 can be similarly shaped and/or sized.

The embodiments illustrated in FIGS. 106-115 can advantageously simplify manufacturing, for example, by allowing much simpler processing and machining procedures (such as laser cutting) to be used. This configuration further avoids small fasteners, which can in some instances be difficult to manufacture and assemble, and additionally allows for a flexible design wherein a manufacturer can select from a variety of opening and commissure opening configurations depending on requirements.

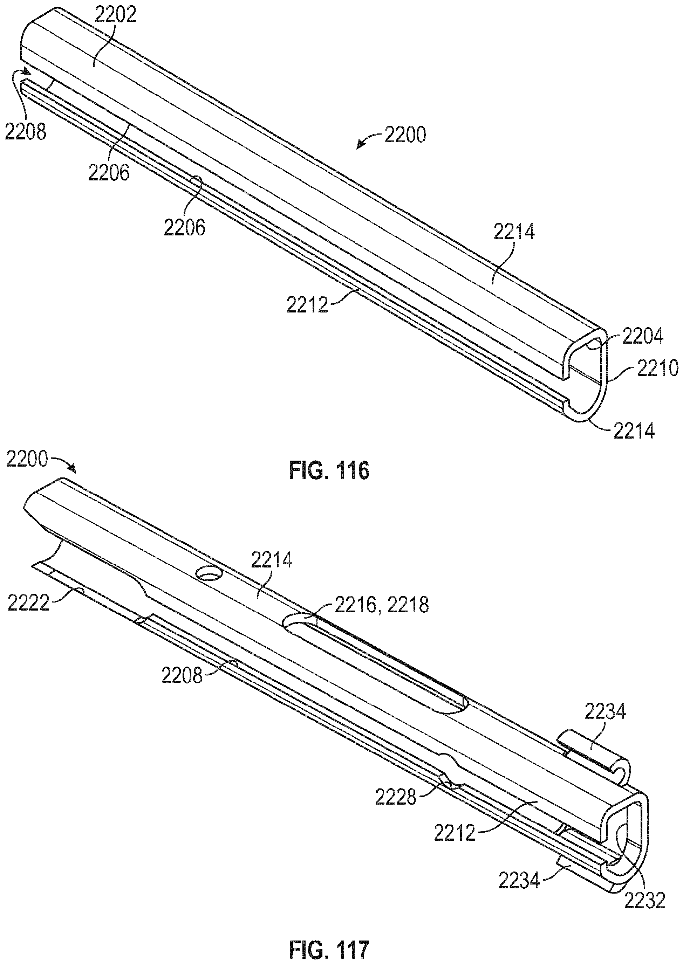

Referring now to FIGS. 116-120, in other embodiments, an outer member 2200 of an expansion and locking mechanism can be manufactured in the following exemplary manner. As shown in FIG. 116, a flat sheet of material 2202 can be cut and bent to form an elongated member having a substantially rectangular shape in cross-section, which can serve as an outer member 2200 having an internal bore 2205. The outer member 2200 can be formed such that the edges 2206 of the sheet 2202 define a slot 2208 between them, the slot 2208 extending parallel to a longitudinal axis of the outer member 2200. The outer member 2200 can generally comprise a radially inner wall 2210, a radially outer wall 2212 including the slot 2208, and first and second side walls 2214.

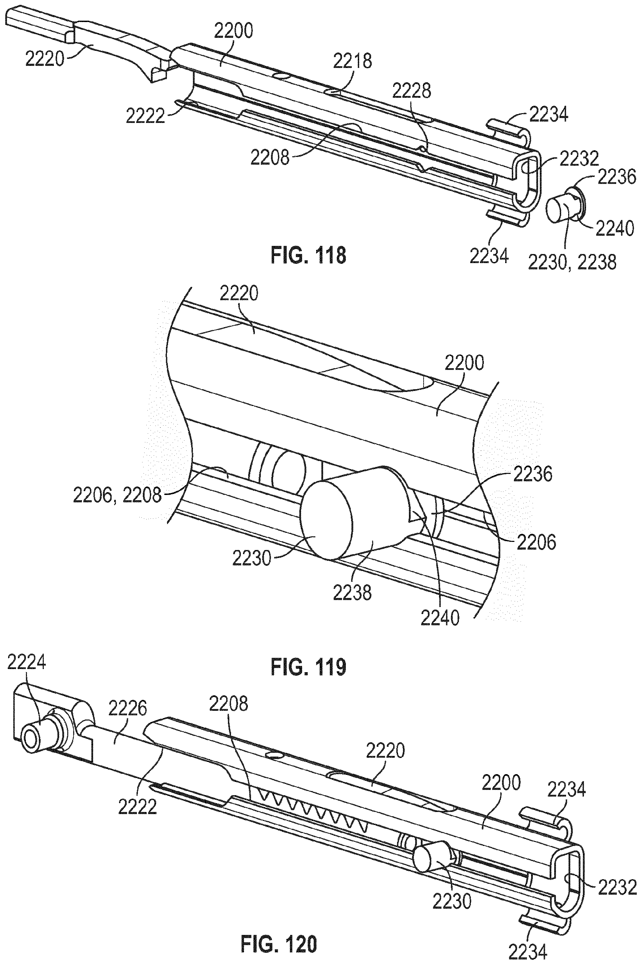

Referring to FIG. 117, one or more cutouts 2216 can be cut into the outer member 2200 (e.g., via laser cutting). For example, the cutouts 2216 can include an opening 2218 in which the locking arm 2220 (FIG. 118) can be disposed, an inflow cutout 2222 configured to guide and/or restrain the fastener 2224 of the inner member 2226 (FIG. 120), and a fastener opening 2228 in which the fastener 2230 (FIG. 118) of the outer member 2200 can be disposed. The fastener opening 2228 can comprise two semi-circular openings separated by the slot 2208. In the illustrated embodiment, the locking arm opening 2218 can be disposed in a side wall 2214, and the inflow cutout 2222 and the fastener opening 2228 can be disposed in the radially outer wall 2212. However, in other embodiments, the locking arm opening 2218 can be disposed in any wall. Commissure openings 2232 and commissure arm posts 2234 can be formed as described above with respect to FIGS. 106-115.

Referring to FIGS. 118-119, a fastener 2230 (similar to fastener 2124 described previously and comprising a base portion 2236, a body portion 2238, and one or more recessed portions 2240) can be coupled to the outer member 2200 in the following exemplary manner. The fastener 2230 can be inserted into the slot 2208 such that the edges 2206 of the slot 2208 are disposed within the recessed portions 2240 (e.g., such that the fastener 2230 is rotationally aligned with the slot 2208). The fastener 2230 can be advanced along the slot 2208 by sliding the fastener 2230 along the edges 2206 of the slot 2208 until the fastener 2230 is disposed in the fastener opening 2228, as shown in FIG. 119. The fastener 2230 can then be rotated until the recessed portions 2240 are perpendicular to the slot 2208 (e.g., such that the fastener 2230 is rotationally offset from the slot 2208). When the fastener is rotationally offset from the slot 2208, the body portion 2238 of the fastener 2230 is too wide to fit through the slot 2208, thereby securing the fastener 2230 within the fastener opening 2228. In some embodiments, the slot 2208 can be deformed (e.g., by pinching and/or welding) after insertion of the fastener 2230 into fastener opening 2228, to further retain the fastener 2230 within the opening 2228.

The locking member 2220 can be coupled to the outer member 2200 in the manner described previously (e.g., using indentations in one or more walls of the outer member 2200). The inner member 2226 can then be disposed at least partially within the bore 2205 of the outer member 2200 to form the expansion and locking mechanism, which can function similarly to expansion and locking mechanisms 710, 1006, 1104, 1208, etc. described above.

The configuration illustrated in FIGS. 116-120 can advantageously simplify manufacturing, for example, by allowing much simpler processing and machining procedures (such as laser cutting) to be used. This configuration further avoids small fasteners, which can in some instances be difficult to manufacture and assemble, and additionally allows for a flexible design wherein a manufacturer can select from a variety of opening and commissure opening configurations depending on requirements.

### General Considerations

For purposes of this description, certain aspects, advantages, and novel features of the embodiments of this disclosure are described herein. The disclosed methods, apparatus, and systems should not be construed as being limiting in any way. Instead, the present disclosure is directed toward all novel and nonobvious features and aspects of the various disclosed embodiments, alone and in various combinations and sub-combinations with one another. The methods, apparatus, and systems are not limited to any specific aspect or feature or combination thereof, nor do the disclosed embodiments require that any one or more specific advantages be present or problems be solved.

Although the operations of some of the disclosed embodiments are described in a particular, sequential order for convenient presentation, it should be understood that this manner of description encompasses rearrangement, unless a particular ordering is required by specific language set forth below. For example, operations described sequentially may in some cases be rearranged or performed concurrently. Moreover, for the sake of simplicity, the attached figures may not show the various ways in which the disclosed methods can be used in conjunction with other methods. Additionally, the description sometimes uses terms like "provide" or "achieve" to describe the disclosed methods. These terms are high-level abstractions of the actual operations that are performed. The actual operations that correspond to these terms may vary depending on the particular implementation and are readily discernible by one of ordinary skill in the art.

All features described herein are independent of one another and, except where structurally impossible, can be used in combination with any other feature described herein. For example, a delivery apparatus 200 as shown in FIG. 5 can be used in combination with prosthetic valve 10. In another embodiment, a locking mechanism as shown in FIG. 1 can be used in combination with the prosthetic valve 100 shown in FIG. 2. Expansion and locking mechanisms 710, 1006, 1104, 1208 can be used with any of the disclosed prosthetic valves.

As used in this application and in the claims, the singular forms "a," "an," and "the" include the plural forms unless the context clearly dictates otherwise. Additionally, the term "includes" means "comprises." Further, the term "coupled" generally means physically, mechanically, chemically, magnetically, and/or electrically coupled or linked and does not exclude the presence of intermediate elements between the coupled or associated items absent specific contrary language.

As used herein, the term "proximal" refers to a position, direction, or portion of a device that is closer to the user and further away from the implantation site. As used herein, the term "distal" refers to a position, direction, or portion of a device that is further away from the user and closer to the implantation site. Thus, for example, proximal motion of a device is motion of the device away from the implantation site and toward the user (e.g., out of the patient's body), while distal motion of the device is motion of the device away from the user and toward the implantation site (e.g., into the patient's body). The terms "longitudinal" and "axial" refer to an axis extending in the proximal and distal directions, unless otherwise expressly defined.

In view of the many possible embodiments to which the principles of the disclosure may be applied, it should be recognized that the illustrated embodiments are only preferred examples and should not be taken as limiting the scope of the disclosure. Rather, the scope of the disclosure is defined by the following claims. We therefore claim all that comes within the scope and spirit of these claims.

In the following the further embodiments are disclosed:
1. An implantable prosthetic device, comprising;
   a frame movable between a radially compressed and a radially expanded configuration, the frame comprising:
      a first strut comprising a first locking feature disposed on a radially facing inner surface of the strut;
      a second strut comprising a second locking feature disposed on a radially facing outer surface of the strut; and
   wherein the first and second locking features engage each other so as to allow pivoting of the first and second struts relative to one another in a first direction upon radial expansion of the frame and resist pivoting of the first and second struts relative to one another in a second direction to resist radial compression of the frame.
2. The implantable prosthetic device of embodiment 1, wherein the first locking feature is disposed at a first end portion of the first strut, and wherein the second locking feature is disposed at a first end portion of the second strut.
3. The implantable prosthetic device of embodiment 1 or 2, wherein the first locking feature comprises a first toothed portion and the second locking feature comprises a second toothed portion.
4. The implantable device of embodiment 3, wherein the first and second struts are pivotably coupled to one another at a junction, and wherein the first and second toothed portions are arrayed circumferentially around at least a portion of the junction.
5. The implantable device of embodiment 3 or 4, wherein the first toothed portion comprises a first set of surfaces extending perpendicularly to the radially facing inner surface of the first strut and a second set of surface extending at an angle less than 90° relative to the radially facing inner surface of the strut, and wherein the second toothed portion comprises a third set of surfaces extending perpendicularly to the radially facing outer surface of the second strut and a fourth set of surfaces extending at an angle less than 90° relative to the radially facing outer surface of the strut.
6. The implantable prosthetic device of any of embodiments 1-5, wherein the first locking feature is formed integrally with the first strut and wherein the second locking feature is formed integrally with the second strut.
7. The implantable prosthetic device of any of embodiments 1-6, wherein the first and second struts are pivotably coupled to one another at a junction by a fastener extending through the junction, and wherein the fastener comprises a biasing member configured to bias the first and second struts against one another.
8. The implantable prosthetic device of embodiment 7, wherein the fastener has a head portion and a shaft, the shaft extending through the first and second struts, and wherein the biasing member is disposed around the shaft at a location between the head portion and the first and second struts.
9. The implantable prosthetic device of any of embodiments 1-7, wherein the first and second locking features are movable from a disengaged position to an engaged position, wherein when in the disengaged position the first and second struts can move relative to one another in the first and second directions.
10. The implantable prosthetic device of embodiment 9, wherein when in the engaged position the first and second locking features are rotationally aligned with one another and wherein when in the disengaged position the first and second locking features are rotationally offset from one another.
11. An implantable prosthetic device, comprising:
   a frame movable between a radially compressed and a radially expanded configuration, the frame comprising
      a first set of struts,
      a second set of struts, each strut of the second set of struts being pivotally connected at one or more junctions to at least one strut of the first set of struts;
   wherein at least one strut of the first set of struts and at least one strut of the second set of struts comprise first and second locking features, respectively, at a junction where the at least one strut of the first set of struts and the at least one strut of the second set of struts are pivotally connected to each other; and
   wherein the first and second locking features engage each other so as to allow pivoting of the struts of the first set relative to the struts of the second set in a first direction upon radial expansion of the frame and resist pivoting of the struts relative to one another in a second direction to resist radial compression of the frame.
12. The implantable device of embodiment 11, wherein the first locking feature is a first toothed portion and wherein the second locking feature is a second, correspondingly toothed portion.
13. The implantable device of embodiments 11 or 12, wherein the first locking feature is disposed on a radially facing inner surface of the at least one strut of the first set of struts and wherein the second locking feature is disposed on a radially facing outer surface of the at least one strut of the second set of struts.
14. The implantable device of any one of embodiments 11-13, wherein the first locking feature is formed integrally with the at least one strut of the first set of struts and wherein the second locking feature is formed integrally with the at least one strut of the second set of struts.
15. The implantable device of any one of embodiments 11-14, wherein each strut comprises a plurality of segments coupled to one another by a plurality of intermediate segments, and wherein the first locking feature is disposed on an intermediate segment of the at least one strut of the first set of struts and the second locking feature is disposed on an intermediate segment of the at least one strut of the second set of struts.
16. The implantable device of any one of embodiments 11-15, wherein each junction comprises a fastener extending through respective struts of the first and second sets of struts.
17. The implantable device of embodiment 16, further comprising a biasing member configured to bias the radially facing inner surface of a respective strut of the first set of struts against the radially facing outer surface of a respective strut of the second set of struts.
18. A method, comprising:
   inserting a distal end of a delivery apparatus into the vasculature of a patient, the delivery apparatus releasably coupled to a prosthetic valve movable between a radially compressed and a radially expanded configuration, the prosthetic valve comprising a frame having a first set of struts pivotably coupled to a second set of struts, each strut having a radially facing inner surface, and a radially facing outer surface;
   advancing the prosthetic valve to a selected implantation site; and
   radially expanding the prosthetic valve such that a first locking feature disposed on a radially facing inner surface of at least one of the first set of struts engages a second locking feature disposed on a radially facing outer surface of at least one of the second set of struts to lock the frame in the radially expanded configuration.
19. The method of embodiment 18, wherein radially expanding the prosthetic valve comprises pivoting the first set of struts away from the second set of struts such that the first and second locking features rotate toward one another and engage one another.
20. The method of any of embodiments 18-19, wherein the first locking feature comprises a first plurality of teeth and the second locking feature comprises a second plurality of teeth and wherein engagement of the first plurality of teeth with the second plurality of teeth prevents radially compression of the prosthetic valve.
21. An implantable prosthetic device, comprising:
   a frame movable between a radially compressed and a radially expanded configuration, the frame comprising an inflow end portion and an outflow end portion;
   at least one expansion and locking mechanism comprising:
      a first member coupled to the frame at a first location, the first member comprising a pawl,
      a second member coupled to the frame at a second location spaced apart from the first location, the second member extending at least partially into the first member and comprising a rack having a plurality of teeth arrayed along a length of the second member;
   wherein engagement of the pawl with the rack allows movement in a first direction to allow radial expansion of the frame and prevents movement in a second direction to prevent radial compression of the frame; and
   wherein the first member comprises a sleeve and the teeth of the second member are housed in the sleeve.
22. The implantable device of embodiment 21, wherein the first and second members have a rectangular or square cross-sectional profile in a plane perpendicular to a length of the expansion and locking mechanism.
23. The implantable prosthetic device of any of embodiments 21-22, wherein the pawl is biased toward the plurality of teeth.
24. The implantable prosthetic device of any of embodiments 21-23, further comprising a retaining member disposed between the pawl and the second member, the retaining member configured to selectively retain the pawl from engaging the rack.
25. The implantable prosthetic device of any of embodiment 21-24 wherein the entirety of the rack is enclosed within the sleeve.
26. An assembly, comprising:
   a prosthetic heart valve comprising
      a frame movable between a radially compressed and a radially expanded configuration, the frame comprising an inflow end portion and an outflow end portion;
      at least one expansion and locking mechanism comprising a first member coupled to the frame at a first location, the first member comprising a pawl, and a second member coupled to the frame at a second location spaced apart from the first location, the second member extending at least partially into the first member and comprising a rack comprising a plurality of teeth, wherein the first member is shaped to enclose the rack;
   a delivery apparatus comprising
      a handle,
      a first actuation member extending from the handle and coupled to the first member, the first actuation member configured to apply a distally directed force to the first member,
      a second actuation member extending from the handle and coupled to the second member, the second actuation member configured to apply a proximally directed force to the second member;
   wherein the prosthetic heart valve is radially expandable from the radially compressed configuration to the radially expanded configuration upon application of the distally directed force and the proximally directed force to the prosthetic heart valve via the first and second actuation members, respectively; and
   wherein expansion of the prosthetic valve causes movement of the first and second members relative to one another such that the pawl engages the teeth of the rack allowing movement of the first and second members in a first direction to allow radial expansion of the frame and preventing movement in a second direction to prevent radial compression of the frame.
27. An implantable prosthetic device, comprising:
   a frame being radially expandable and compressible between a radially compressed state and a radially expanded state, the frame comprising a first set of first struts, a second set of second struts, and a third set of third struts, the frame having a distal end and a proximal end;
   wherein the first struts are pivotably connected to each other at a plurality of distal and proximal apices at the distal and proximal ends of the frame, respectively;
   wherein the second struts are pivotably connected to each other at a plurality of distal and proximal apices at the distal and proximal ends of the frame, respectively;
   wherein the third struts are pivotably connected to each other at a plurality of distal and proximal apices at the distal and proximal ends of the frame, respectively;
   wherein the first struts are pivotably connected to the second and third struts at junctions between the distal and proximal ends of the frame;
   wherein the second struts are pivotably connected to the first and third struts at junctions between the distal and proximal ends of the frame;
   at least one expansion mechanism coupled to the frame at a pair of a distal apex and a proximal apex formed by the first struts;
   at least one locking mechanism coupled to the frame at a pair of axially spaced junctions, each of which is formed by struts of different sets;
   a plurality of commissure posts coupled to the frame at respective junctions; and
   a leaflet assembly comprising a plurality of leaflets arranged to form a plurality of commissures coupled to respective commissure posts.
28. An implantable prosthetic device, comprising:
   a frame movable between a radially compressed and a radially expanded configuration, the frame comprising an inflow end portion and an outflow end portion;
   at least one expansion and locking mechanism comprising:
      a first member coupled to the frame at a first location,
      a second member coupled to the frame at a second location spaced apart from the first location, the second member extending at least partially into the first member and comprising a rack having a plurality of teeth arrayed along a length of the second member, and
      a locking member coupled to the first member via one or more protrusions extending from the second member into the first member, the locking member being biased toward the plurality of teeth; and
   wherein engagement of the locking member with the rack allows movement in a first direction to allow radial expansion of the frame and prevents movement in a second direction to prevent radial compression of the frame.
29. The implantable device of embodiment 28, wherein the locking member comprises one or more apertures extending at least partially through a thickness of the locking member, and wherein the one or more protrusions extend into the apertures.
30. The implantable device of embodiment 29, wherein the one or more protrusions have a substantially cylindrical shape.
31. The implantable device of embodiment 29, wherein the one or more protrusions have a hemispherical shape.
32. The implantable device of any of embodiments 28-31, wherein the locking member comprises a locking tooth extending toward the rack and configured to engage the plurality of teeth.
33. The implantable device of any of embodiments 28-32, wherein the locking member comprises a disengagement tooth extending axially from a proximal edge of the locking member.
34. The implantable device of embodiment 33, further comprising a disengagement member configured to selectively engage the disengagement tooth to retain the locking member from engaging the rack.
35. The implantable device of any of embodiments 32-34, wherein the locking member comprises a cutout defining a neck portion configured to bias the locking tooth radially against the rack.
36. The implantable device of embodiment 35, wherein the neck portion is a first neck portion and wherein the locking member further comprises a second cutout defining a second neck portion configured to bias the locking tooth axially against the rack.
37. The implantable device of any of embodiments 28-36, wherein the first member comprises a first engagement surface and the second member comprises a protrusion having a second engagement surface, and wherein the second engagement surface is configured to selectively abut the first engagement surface to prevent proximal movement of the second member past a predetermined point.
38. The implantable device of any of embodiments 28-36, further comprising a stopper disposed on a distal end portion of the second member, the stopper sized to selectively abut a distal end portion of the first member to prevent proximal movement of the second member past a predetermined point.
39. The implantable device of embodiment 38, wherein the distal end portion of the second member comprises a threaded portion and the stopper comprises a correspondingly threaded portion, and wherein rotation of the stopper in a first direction advances the stopper distally relative to the second member and rotation of the stopper in a second direction advances the stopper proximally relative to the second member.
40. The implantable device of any of embodiments 28-39, wherein the first member has a rectangular cross-sectional profile in a plane perpendicular to a length of the expansion and locking mechanism.
41. The implantable device of any of embodiments 28-40, wherein the first member comprises an inner bore having a first portion and a second portion, each having a first width, the first and second portions being separated by a neck portion having a second width smaller than the first width.
42. The implantable device of embodiment 41, wherein the second member is sized to extend at least partially into the first portion of the inner bore.
43. The implantable device of any of embodiments 28-41, wherein the first member comprises an opening in which the locking member is disposed.
44. The implantable device of embodiment 43, in which the first member comprises a ledge portion extending at least partially into the opening on which a portion of the locking member is disposed.
45. A method of making an expansion and locking mechanism, comprising:
   providing an outer member having an inner wall and an outer wall each comprising one or more first apertures having a first diameter, a first side wall, and a second side wall, the first side wall including an opening;
   disposing a locking member within the opening, the locking member comprising one or more second apertures extending at least partially through a thickness of the locking member, wherein the locking member is disposed in the opening such that each second aperture aligns with a respective first aperture, and wherein each second aperture has a second diameter greater than the first diameter such that a respective lip portion is defined between each pair of first and second apertures; and
   using a punch member to apply force to a respective first aperture such that the lip portion deforms into the respective second aperture thereby securing the locking member to the outer member.
46. The method of embodiment 45, wherein the punch member is a cylindrical member having a third diameter greater than the first diameter and smaller than the second diameter.
47. An implantable prosthetic device, comprising:
   a frame movable between a radially compressed and a radially expanded configuration, the frame comprising an inflow end portion and an outflow end portion;
   at least one expansion and locking mechanism comprising:
      a first member coupled to the frame at a first location, the first member comprising a locking member,
      a second member coupled to the frame at a second location spaced apart from the first location, the second member extending at least partially into the first member and comprising a rack having a plurality of teeth arrayed along a length of the second member, and
      a stopper disposed on an end portion of the second member, the stopper configured to prevent movement of the second member in a first direction past a predetermined point; and
   wherein engagement of the locking member with the rack allows movement in the first direction to allow radial expansion of the frame and prevents movement in a second direction to prevent radial compression of the frame.
48. The implantable prosthetic device of embodiment 47, wherein the stopper comprises an annular nut sized to selectively abut a distal edge of the first member to retain the frame at a predetermined diameter.
49. The implantable prosthetic device of any of embodiments 47-48, wherein the end portion of the second member comprises a threaded portion and the stopper comprises a correspondingly threaded portion, and wherein rotation of the stopper in a first direction advances the stopper in the first direction relative to the second member and rotation of the stopper in a second direction advances the stopper in the second direction relative to the second member.
50. An implantable prosthetic device, comprising:
   a frame movable between a radially compressed and a radially expanded configuration, the frame comprising an inflow end portion and an outflow end portion;
   at least one expansion and locking mechanism comprising:
      a first member coupled to the frame at a first location, the first member comprising an aperture extending through a thickness of the first member,
      a second member coupled to the frame at a second location spaced apart from the first location, the second member extending at least partially into the first member, and
      a locking member coupled to the first member and being biased toward the second member;
   wherein engagement of the locking member with the second member allows movement in a first direction to allow radial expansion of the frame and prevents movement in a second direction to prevent radial compression of the frame; and
   wherein the aperture is positioned such that a proximal edge of the second member can be viewed through the aperture when the prosthetic heart valve is in an assembled configuration.
51. The implantable device of embodiment 50, wherein the first member has a rectangular cross-sectional profile in a plane perpendicular to a length of the expansion and locking mechanism, the first member comprising a first wall and a second wall, the first wall disposed radially outwardly of the second wall.
52. The implantable device of embodiment 51, wherein the aperture extends through a thickness of the first wall.
53. The implantable device of any of embodiments 50-52, wherein the first member comprises a commissure attachment portion and wherein the aperture is disposed distally relative to the commissure attachment portion.
54. The implantable device of any of embodiments 50-53, wherein the aperture is positioned such that an apex of the locking tooth can be viewed through the aperture.
55. The implantable device of any of embodiments 50-54, further comprising a valvular structure including a plurality of leaflets, wherein each pair of adj acent leaflets is coupled to a respective expansion and locking mechanism at a respective commissure attachment portion to form a commissure.
56. The implantable device of embodiment 55, wherein the aperture is positioned such that when the prosthetic device is in the radially expanded position, the commissure does not cover the aperture.
57. The implantable device of any of embodiments 50-56, wherein the second member comprises a rack having a plurality of teeth arrayed along a length of the second member, and wherein the locking member comprises a locking tooth extending toward the rack and configured to engage the plurality of teeth.
58. The implantable device of any of embodiments 50-57, wherein the locking member comprises a disengagement tooth extending axially from a proximal edge of the locking member.
59. The implantable device of embodiment 58, further comprising a disengagement member configured to selectively engage the disengagement tooth to retain the locking member from engaging the rack.
60. The implantable device of any of embodiments 50-59, wherein the first member comprises a first engagement surface and the second member comprises a protrusion having a second engagement surface, and wherein the second engagement surface is configured to selectively abut the first engagement surface to prevent proximal movement of the second member past a predetermined point.
61. The implantable device of any of embodiments 50-60, further comprising a fastener extending radially outward from the first wall of the first member.
62. The implantable device of embodiment 61, wherein the aperture is positioned distally adjacent to the fastener.
63. The implantable device of any of embodiments 50-62, further comprising a fastener extending radially outward from a distal end portion of the second member.
64. The implantable device of any of embodiments 50-63, further comprising a stopper disposed on a distal end portion of the second member, the stopper sized to selectively abut a distal end portion of the first member to prevent proximal movement of the second member past a predetermined point.
65. The implantable device of embodiment 64, wherein the distal end portion of the second member comprises a threaded portion and the stopper comprises a correspondingly threaded portion, and wherein rotation of the stopper in a first direction advances the stopper distally relative to the second member and rotation of the stopper in a second direction advances the stopper proximally relative to the second member.
66. The implantable device of any of embodiments 50-65, wherein the first member comprises an inner bore having a first portion and a second portion, each having a first width, the first and second portions being separated by a neck portion having a second width smaller than the first width.
67. The implantable device of embodiment 66, wherein the second member is sized to extend at least partially into the first portion of the inner bore.
68. The implantable device of any of embodiments 50-67, wherein the first member comprises an opening in which the locking member is disposed.
69. The implantable device of any of embodiments 50-68, wherein the aperture is positioned such that a connection between the second member and an actuator of a delivery apparatus is visible.
70. The implantable device of any of embodiments 50-69, wherein the frame comprises three expansion and locking mechanisms disposed circumferentially around the frame.
71. The implantable device of embodiment 70, wherein the three expansion and locking mechanisms are spaced apart evenly from one another.
72. An implantable prosthetic device, comprising:
   a frame movable between a radially compressed and a radially expanded configuration, the frame comprising an inflow end portion and an outflow end portion;
      at least one expansion and locking mechanism comprising:
      a first member coupled to the frame at a first location, the first member having a first wall and a second wall and comprising an aperture extending through a thickness of the first wall,
      a second member coupled to the frame at a second location spaced apart from the first location, the second member extending at least partially into the first member, and
      a locking member coupled to the first member and being biased toward the second member;
   a valvular structure comprising a plurality of leaflets, wherein each pair of adjacent leaflets is coupled to a respective expansion and locking mechanism at a respective commissure attachment portion to form a commissure; and
   wherein a respective aperture is disposed distally adjacent each commissure.
73. The implantable device of embodiment 72, wherein the valvular structure comprises three leaflets and three commissures.
74. An assembly, comprising:
   an implantable prosthetic device having a frame movable between a radially compressed and a radially expanded configuration, the frame comprising an inflow end portion and an outflow end portion;
      at least one expansion and locking mechanism comprising:
      a first member coupled to the frame at a first location, the first member comprising an aperture extending through a thickness of the first member,
      a second member coupled to the frame at a second location spaced apart from the first location, the second member extending at least partially into the first member, and
      a locking member coupled to the first member and being biased toward the second member;

      a delivery apparatus comprising:
         a handle,
      a first actuation member extending from the handle and coupled to the first member, the first actuation member configured to apply a distally directed force to the first member,
      a second actuation member extending from the handle and coupled to the second member, the second actuation member configured to apply a proximally directed force to the second member; and
   wherein the connection between the second member and the second actuation member is visible through the aperture.
75. The assembly of embodiment 74, wherein the second actuation member extends at least partially into the first member.
76. The assembly of any of embodiments 74-75, wherein a distal end portion of the second actuation member comprises an engagement member.
77. The assembly of embodiment 76, wherein a proximal end portion of the second member comprises a bore into which the engagement member can extend.
78. The assembly of embodiment 77, wherein the engagement member comprises an external threaded surface configured to couple a correspondingly threaded surface of the bore.
79. The assembly of embodiment 77, wherein the engagement member comprises a magnet, and the inner bore comprises a correspondingly magnetic material.
80. The assembly of any of embodiments 74-79, wherein a distal end portion of the first actuation member is configured to abut a proximal end portion of the first member.
81. The assembly of any of embodiments 74-80, wherein the first member has a rectangular cross-sectional profile in a plane perpendicular to a length of the expansion and locking mechanism, the first member comprising a first wall and a second wall, the first wall disposed radially outwardly of the second wall.
82. The assembly of embodiment 81, wherein the aperture extends through a thickness of the first wall.
83. The assembly of any of embodiments 74-82, wherein the first member comprises a commissure attachment portion and wherein the aperture is disposed distally relative to the commissure attachment portion.
84. The assembly of any of embodiments 74-83, the prosthetic device further comprising a valvular structure including a plurality of leaflets, wherein each pair of adjacent leaflets is coupled to a respective expansion and locking mechanism at a respective commissure attachment portion to form a commissure.
85. The assembly of embodiment 84, wherein the aperture is positioned such that when the prosthetic device is in the radially expanded position, the commissure does not cover the aperture.
86. The assembly of any of embodiments 74-85, further comprising a fastener extending radially outward from the first wall of the first member.
87. The implantable device of embodiment 86, wherein the aperture is positioned distally adjacent to the fastener.
88. An implantable prosthetic device, comprising:
   a frame movable between a radially compressed and a radially expanded configuration, the frame comprising an inflow end portion and an outflow end portion;
   at least one expansion and locking mechanism comprising
      a first member coupled to the frame at a first location, the first member comprising a commissure opening extending through a thickness of the first member,
      a second member coupled to the frame at a second location spaced apart from the first location, the second member extending at least partially into the first member, and
      a locking member coupled to the first member, the locking member configured to engage the second member to allow movement in a first direction to allow radial expansion of the frame and prevent movement in a second direction to prevent radial compression of the frame; and
   a valvular structure comprising a plurality of leaflets each including one or more tabs, wherein tabs of adjacent leaflets are disposed within the commissure opening to couple the valvular structure to the frame.
89. The implantable device of embodiment 88, wherein the commissure opening comprises a first aperture and a second aperture forming a channel between them, and wherein the channel has at least one angled surface corresponding to one or more angled edges of the plurality of leaflets.
90. The implantable device of any of embodiments 88-89, wherein the first member comprises a bore extending longitudinally along the length of the first member, and wherein the bore is offset from a longitudinal axis of the first member.
91. The implantable device of any of embodiments 88-90, wherein each expansion and locking mechanism further comprises a wedge disposed between adjacent tabs to help couple the valvular structure to the frame.
92. The implantable device of any of embodiments 88-91, wherein the portion of the first member comprising the commissure opening extends past the outflow end portion of the frame.
93. The implantable device of any of embodiments 88-92, wherein the first member is coupled to the frame via a fastener extending from a surface of the first member.
94. The implantable device of embodiment 93, wherein the commissure opening is closer to the outflow end portion of the frame than the fastener.
95. The implantable device of any of embodiments 88-94, wherein the second member is coupled to the frame via a fastener extending from a surface of the second member.
96. The implantable device of any of embodiments 88-95, wherein the commissure opening extends to an outflow edge of the first member.
97. The implantable device of embodiment 96, wherein the commissure opening defines a first extension and a second extension in the outflow end portion of the first member with the commissure opening between them.
98. The implantable device of embodiment 97, wherein the first member comprises a bore extending along a length of the first member and disposed in the second extension.
99. The implantable device of any of embodiments 88-98, wherein the first member comprises an angled portion such that an outflow end portion of the expansion and locking mechanism has a first width and an inflow end portion has a second width narrower than the first width.
100. The implantable device of any of embodiments 88-99, wherein the first member comprises one or more rounded radially inner edges.
101. The implantable device of any of embodiments 88-100, wherein the first member comprises one or more rounded radially outer edges configured to correspond to a radially inner surface of the frame.
102. The implantable device of any of embodiments 88-101, wherein the first member comprises one or more chamfered radially inner edges.
103. The implantable device of any of embodiments 88-99 or 102, wherein the first member comprises one or more chamfered radially outer edges.
104. A method of assembling a prosthetic valve, comprising:
   providing a frame movable between a radially compressed and a radially expanded configuration, the frame comprising an inflow end portion and an outflow end portion, the frame including one or more expansion and locking mechanisms including an outer member comprising a commissure opening extending through a thickness of the outer member, an inner member, and a locking member;
   inserting tabs of adj acent leaflets of a valvular structure into the commissure opening such that the tabs extend through the outer member;
   inserting a wedge between radially outer portions of the tabs to form a commissure assembly; and
   coupling the commissure assembly to the outer member.
105. The method of embodiment 104, wherein inserting the tabs into the commissure opening comprises inserting the tabs through a radially inner aperture in the outer member and then inserting the tabs at least partially through a radially outer aperture in the outer member.
106. The method of any of embodiments 104-105, wherein coupling the commissure assembly to the outer member comprises using one or more sutures.
107. A method of assembling a prosthetic valve, comprising:
   providing a frame movable between a radially compressed and a radially expanded configuration, the frame comprising an inflow end portion and an outflow end portion, the frame including one or more expansion and locking mechanisms including an outer member comprising a commissure opening extending through a thickness of the outer member and extending to an outflow edge of the outer member to form an outflow aperture, an inner member, and a locking member;
   inserting a wedge between adjacent tabs of adjacent leaflets of a valvular structure and coupling the wedge to the tabs to form a commissure assembly;
   inserting the commissure assembly into the commissure opening by sliding the commissure assembly through the outflow aperture; and
   coupling the commissure assembly to the outer member.
108. The method of embodiment 107, wherein coupling the wedge to the tabs includes using one or more sutures.
109. The method of any of embodiments 107-108, wherein coupling the commissure assembly to the outer member includes using one or more sutures.
110. An implantable prosthetic device, comprising:
   a frame movable between a radially compressed and a radially expanded configuration, the frame comprising an inflow end portion and an outflow end portion;
   at least one expansion and locking mechanism comprising:
      a first member coupled to the frame at a first location, the first member comprising an inner bore extending the length of the first member, the bore having a first portion and a second portion separated by a neck portion,
      a second member coupled to the frame at a second location spaced apart from the first location, the second member extending at least partially into the first portion of the bore, and
      a locking member comprising a pawl portion and a body portion, the locking member disposed within the second portion of the bore; and
   wherein engagement of the locking member with the inner member allows movement in a first direction to allow radial expansion of the frame and prevents movement in a second direction to prevent radial compression of the frame.
111. The implantable device of embodiment 110, wherein at least a portion of the second member has a semi-circular shape in cross-section comprising a curved surface and a flat surface.
112. The implantable device of any of embodiments 110-111, wherein the body portion of the locking member has a semi-circular shape in cross-section comprising a curved surface and a flat surface.
113. The implantable device of any of embodiments 111-112, wherein the flat surfaces of the second member and locking member are oriented toward one another.
114. The implantable device of any of embodiments 111-113, wherein the flat surfaces of the second member and locking member are spaced apart from one another.
115. The implantable device of any of embodiments 111-113, wherein the flat surfaces of the second member and locking member contact one another.
116. The implantable device of any of embodiments 111-115, wherein the flat surfaces of the second member and locking member are coated with a lubricious coating.
117. The implantable device of any of embodiments 110-116, wherein a side wall of the first member comprises an opening aligned with the pawl portion of the locking member such that the pawl portion can selectively deflect into the opening.
118. The implantable device of any of embodiments 110-116, wherein the body portion of the locking member comprises one or more apertures and wherein the locking member is coupled to the first member via one or more protrusions extending from the first member into the apertures.
119. An implantable prosthetic device, comprising:
   a frame movable between a radially compressed and a radially expanded configuration, the frame comprising an inflow end portion and an outflow end portion;
   at least one expansion and locking mechanism comprising:
      a first member coupled to the frame at a first location,
      a second member coupled to the frame at a second location spaced apart from the first location, the second member extending at least partially into the first member, and
      a locking member coupled to the first member via one or more lateral extensions extending from the first member into a recess of the locking member; and
   wherein engagement of the locking member with the inner member allows movement in a first direction to allow radial expansion of the frame and prevents movement in a second direction to prevent radial compression of the frame.
120. The implantable device of embodiment 119, wherein the second member comprises a rack having a plurality of teeth arrayed along a length of the second member, and wherein the locking member comprises a locking tooth extending toward the rack and configured to engage the plurality of teeth.
121. The implantable device of any of embodiments 119-120, wherein the lateral extensions have a rectangular shape.
122. The implantable device of embodiment 121, wherein the lateral extensions have a length along a longitudinal axis of the outer member greater than a width of the lateral extensions.
123. The implantable device of embodiment 121, wherein the lateral extensions have a length along a longitudinal axis of the outer member less than a width of the lateral extensions.
124. The implantable device of any of embodiments 121-123, wherein the recess has a rectangular shape corresponding to the rectangular shapes of the lateral extensions.
125. The implantable device of any of embodiments 119-124, wherein the recess has a depth corresponding to a thickness of the lateral extensions.
126. The implantable device of any of embodiments 119-125, wherein the lateral extensions are aligned with one another along a length of the first member.
127. The implantable device of any of embodiments 119-126, wherein the lateral extensions are offset from one another along a length of the first member.
128. A method of making an expansion and locking mechanism, comprising:
   providing an outer member having an inner wall, an outer wall, a first side wall, and a second side wall, the first side wall including an opening and the inner wall and outer wall each comprising one or more bendable lateral extensions aligned with a first portion of the opening;
   disposing a locking member within the opening, the locking member comprising a pawl portion and a body portion including a recess, the locking member being disposed within the opening such that the recess is aligned with the lateral extensions; and
   applying a force to each lateral extension such that the lateral extension deforms into the recess, thereby securing the locking member to the outer member.
129. The method of embodiment 128, wherein the lateral extensions have a rectangular shape.
130. The method of embodiment 129, wherein the lateral extensions have a length along a longitudinal axis of the outer member greater than a width of the lateral extensions.
131. The method of embodiment 129, wherein the lateral extensions have a length along a longitudinal axis of the outer member less than a width of the lateral extensions.
132. The method of any of embodiments 128-131, wherein when the lateral extensions are in an undeformed position they extend perpendicular to the first side wall.
133. The method of any of embodiments 128-132, wherein when the lateral extensions are in a deformed position they extend parallel to the first side wall.
134. The method of any of embodiments 128-133, wherein the recess has a rectangular shape corresponding to the rectangular shapes of the lateral extensions.
135. The implantable device of any of embodiments 128-134, wherein the recess has a depth corresponding to a thickness of the lateral extensions.
136. The implantable device of any of embodiments 128-135, wherein the lateral extensions are aligned with one another along a length of the first member.
137. The implantable device of any of embodiments 128-136, wherein the lateral extensions are offset from one another along a length of the first member.
138. An implantable prosthetic device, comprising:
   a frame movable between a radially compressed and a radially expanded configuration, the frame comprising an inflow end portion and an outflow end portion;
   at least one expansion and locking mechanism comprising
      a first member coupled to the frame at a first location, the first member comprising an opening including one or more lateral extensions,
      a second member coupled to the frame at a second location spaced apart from the first location, the second member extending at least partially into the first member, and
      a locking member comprising a pawl portion and a body portion, the body portion including first and second angled surfaces, the locking member being coupled to the first member via the one or more lateral extensions engaging the angled surfaces; and
   wherein the locking member is configured to engage the second member to allow movement in a first direction to allow radial expansion of the frame and prevent movement in a second direction to prevent radial compression of the frame.
139. The implantable device of embodiment 138, wherein the body portion of the locking member has a triangular shape with chamfered corners in cross-section.
140. The implantable device of any of embodiments 138-139, wherein the angled surfaces are disposed at an angle relative to an inner wall of the locking member.
141. The implantable device of embodiment 140, wherein the inner wall is disposed nearer to the longitudinal axis of the outer member than the angled surfaces.
142. The implantable device of any of embodiments 140-141, wherein the angle is a 45 degree angle.
143. The implantable device of any of embodiments 138-142, wherein the second member comprises a rack having a plurality of teeth arrayed along a length of the second member, and wherein the locking member comprises a locking tooth extending toward the rack and configured to engage the plurality of teeth.
144. The implantable device of any of embodiments 138-143, wherein the lateral extensions have a rectangular shape.
145. The implantable device of any of embodiments 138-144, wherein each lateral extension comprises a chamfered edge portion.
146. The implantable device of embodiment 145, wherein the chamfered edge portions are configured such that when the lateral extensions are engaged with the angled surfaces the chamfered edge portions do not extend past the angled surfaces.
147. A method of making an expansion and locking mechanism, comprising:
   providing a first member having an inner wall, an outer wall, a first side wall comprising an opening, and a second side wall, the inner and outer walls each comprising a lateral extension;
   disposing a locking member within the opening, the locking member comprising a body portion including first and second angled surfaces, wherein the locking member is disposed within the opening such that each angled surface aligns with a respective lateral extension; and
   applying force to the lateral extensions such that the lateral extensions deform to engage the angled surfaces thereby securing the locking member to the first member.
148. The method of embodiment 147, wherein the lateral extensions have a rectangular shape.
149. The method of any of embodiments 147-148, wherein each lateral extension comprises a chamfered edge portion.
150. The method of embodiment 149, wherein the chamfered edge portions are configured such that when the lateral extensions are engaged with the angled surfaces the chamfered edge portions do not extend past the angled surfaces.
151. The method of any of embodiments 147-150, wherein the force applied is directed toward a longitudinal axis of the first member.
152. An implantable prosthetic device, comprising:
   a frame movable between a radially compressed and a radially expanded configuration, the frame comprising an inflow end portion and an outflow end portion;
   at least one expansion and locking mechanism comprising
      a first member coupled to the frame at a first location,
      a second member coupled to the frame at a second location spaced apart from the first location, the second member extending at least partially into the first member, and
      a locking member comprising a pawl portion and a body portion, the body portion including first and second elongated recesses, the locking member being coupled to the first member via first and second protrusions extending from the first member into the first and second recesses; and
   wherein the locking member is configured to engage the second member to allow movement in a first direction to allow radial expansion of the frame and prevent movement in a second direction to prevent radial compression of the frame.
153. The implantable device of embodiment 152, wherein the first and second elongated recesses each have a V-shape in cross-section.
154. The implantable device of embodiment 153, wherein the opening of the opening of each V-shape is oriented toward the side walls of the first member.
155. The implantable device of any of embodiments 153-154, wherein each protrusion has a V-shape that corresponds to the V-shape of a respective recess.
156. The implantable device of any of embodiments 152-155, wherein the second member comprises a rack having a plurality of teeth arrayed along a length of the second member, and wherein the locking member comprises a locking tooth extending toward the rack and configured to engage the plurality of teeth.
157. A method of making an expansion and locking mechanism, comprising:
   providing a first member having an inner wall, an outer wall, a first side wall including an opening, and a second side wall;
   disposing a locking member within the opening, the locking member including an outer wall and an inner wall, the outer and inner walls each comprising an elongated recess; and
   applying a force to the inner and outer walls of the first member such that the inner and outer walls deform to form protrusions that extend into respective recesses thereby securing the locking member to the first member.
158. The method of embodiment 157, wherein the force applied is directed inwardly toward a longitudinal axis of the first member.
159. The method of any of embodiments 157-158, wherein the elongated recesses each have a V-shape in cross-section.
160. The method of embodiment 159, wherein the opening of the opening of each V-shape is oriented toward the side walls of the first member.
161. The method of any of embodiments 159-160, wherein each protrusion has a V-shape that corresponds to the V-shape of a respective recess.
162. An implantable prosthetic device, comprising:
   a frame movable between a radially compressed and radially expanded configuration, the frame comprising an inflow end portion and an outflow end potion;
   at least one expansion and locking mechanism comprising
      a first member coupled to the frame at a first location via a first fastener, the first fastener comprising a body portion and a flanged end portion,
      a second member coupled to the frame at a second location via a second fastener, the second fastener comprising a body portion and a flanged end portion,
      a locking member coupled to the first member; and
   wherein the body portions of the first and second fasteners extend through one or more apertures in the frame, and wherein the flanged end portions are sized to retain the first and second fasteners within the apertures.
163. The implantable device of embodiment 162, wherein each flanged end portion is formed by radial riveting.
164. The implantable device of any of embodiments 162-163, wherein each fastener is a solid piece of material.
165. The implantable device of any of embodiments 162-164, wherein each fastener further comprises a base portion.
166. The implantable device of any of embodiments 162-165, wherein the first fastener is formed integrally with the first member.
167. The implantable drive of any of embodiments 162-166, wherein the body portion of the first fastener extends through an aperture in a wall of the first member.
168. A method of making a prosthetic valve, comprising:
   providing a frame movable between a radially compressed and a radially expanded configuration, the frame comprising a plurality of struts each including one or more apertures;
   disposing an expansion and locking mechanism comprising a first member having a first fastener such that the first fastener extends through one or more apertures at a first location; and
   radially riveting the first fastener to form a first flanged end portion configured to retain the first fastener within its respective apertures, thereby coupling the expansion and locking mechanism to the frame.
169. The method of embodiment 168, wherein the expansion and locking mechanism further comprises a second member having a second fastener, and wherein the method further comprises disposing the expansion and locking mechanism such that the second fastener extends through one or more apertures at a second location spaced apart from the first location along a longitudinal axis of the frame, and radially riveting the second fastener to form a second flanged end portion configured to retain the second fastener within its respective apertures.
170. An expansion and locking mechanism, comprising:
   an outer member comprising a first wall and a second wall, the first wall comprising an opening extending through the first wall, the opening comprising a main portion, a guide portion, and an entry portion; and
   a fastener having a base portion and a body portion, the body portion having one or more recesses configured such that when the recesses are aligned with the guide portion the fastener can slide along the guide portion and into the main opening and when the recesses are offset from the guide portion the fastener is retained within the main portion.
171. The expansion and locking mechanism of embodiment 170, wherein the recesses are configured such that the portion of the body portion on which the recesses are disposed has a non-circular shape in cross-section.
172. The expansion and locking mechanism of any of embodiments 170-171, wherein the main portion of the opening has a first width greater than a second width of the guide portion.
173. The expansion and locking mechanism of any of embodiments 170-172, wherein the entry portion has a width corresponding to the width of the base portion of the fastener.
174. A method of making an expansion and locking mechanism, comprising:
   deforming a tubular member including an inner bore extending along the length of the tubular member such that the tubular member forms an oval shape in cross-section having first, second, third, and fourth walls;
   cutting a fastener opening and inflow end cutout in the first wall and a locking member opening in the second wall;
   disposing a locking member including a first end portion comprising a pawl and a second end portion within the locking member opening;
   disposing a fastener within the fastener opening; and
   disposing an inner member at least partially within the inner bore of the tubular member.
175. The method of embodiment 174, further comprising deforming the first and third walls to form elongated indentations configured to retain the locking member within the locking member opening.
176. The method of any of embodiments 174-175, wherein the fastener opening comprises a main portion having a first width and a guide portion having a second width narrower than the first width.
177. The method of embodiment 176, wherein disposing the fastener within the fastener opening comprises aligning one or more recesses in the fastener with the guide portion, sliding the fastener through the guide portion into the main portion, and rotating the fastener within the main portion such that the recesses are offset from the guide portion.
178. The method of any of embodiments 174-177, further comprising deforming the guide portion once the fastener is disposed within the main portion.
179. The method of any of embodiments 174-178, further comprising:
   cutting a commissure opening in the third wall of the tubular member to form two deflectable portions; and
   bending the deflectable portions toward the first wall to form first and second commissure posts.
180. A method of making an expansion and locking mechanism, comprising:
   deforming a sheet of material having first and second edges to form an elongated member having a substantially rectangular shape with rounded edges in cross-section, the elongated member comprising an inner bore extending along the length of the elongated member and a slot extending along the length of the elongated member defined by the first and second edges;
   cutting a fastener opening and inflow end cutout in a first wall of the elongated member and a locking member opening in a second wall;
   disposing a locking member including a first end portion comprising a pawl and a second end portion within the locking member opening;
   disposing a fastener within the fastener opening; and
   disposing an inner member at least partially within the inner bore of the elongated member.
181. The method of embodiment 180, wherein the fastener opening and the inflow end cutout incorporate at least a portion of the slot.
182. The method of any of embodiments 180-181, further comprising deforming the first and third walls to form elongated indentations configured to retain the locking member within the locking member opening.
183. The method of any of embodiments 180-182, wherein the fastener opening comprises a main portion having a first width and a guide portion having a second width narrower than the first width.
184. The method of embodiment 183, wherein disposing the fastener within the fastener opening comprises aligning one or more recesses in the fastener with the guide portion, sliding the fastener through the guide portion into the main portion, and rotating the fastener within the main portion such that the recesses are offset from the guide portion.
185. The method of embodiment 184, further comprising:
   cutting a commissure opening in the third wall of the tubular member to form two deflectable portions; and
   bending the deflectable portions toward the first wall to form first and second commissure posts.

## Claims

1. An implantable prosthetic device (500), comprising:
a frame (502) movable between a radially compressed and a radially expanded configuration, the frame (502) comprising an inflow end portion and an outflow end portion;
at least one expansion and locking mechanism (550) comprising:
a first member (552) coupled to the frame (502) at a first location, the first member comprising a pawl (568),
a second member (556) coupled to the frame (502) at a second location spaced apart from the first location, the second member (556) extending at least partially into the first member (552) and comprising a rack (564) having a plurality of teeth (566) arrayed along a length of the second member (556);
wherein engagement of the pawl (568) with the rack (564) allows movement in a first direction to allow radial expansion of the frame and prevents movement in a second direction to prevent radial compression of the frame (502); and
wherein the first member (552) comprises a sleeve (552) and the teeth (566) of the second member (556) are housed in the sleeve (552).

2. The implantable prosthetic device (500) of claim 1, wherein the first and second members (552, 556) have a rectangular or square cross-sectional profile in a plane perpendicular to a length of the expansion and locking mechanism (550).

3. The implantable prosthetic device (500) of any of claims 1 to 2, wherein the pawl (568) is biased toward the plurality of teeth (566).

4. The implantable prosthetic device (500) of any of claims 1 to 3, further comprising a retaining member disposed between the pawl (568) and the second member (556), the retaining member configured to selectively retain the pawl (568) from engaging the rack (564).

5. The implantable prosthetic device (500) of any of claims 1 to 4, wherein the entirety of the rack (564) is enclosed within the sleeve (552).

6. The implantable prosthetic device (500) of any previous claims, wherein the second member (556) is housed entirely within the sleeve (552) and the sleeve (552) is closable at its distal end.

7. The implantable prosthetic device (500) of any of claims 1 to 6, wherein substantially the entire length of the inner member (556) comprises teeth (566).

8. The implantable prosthetic device (500) of any of claims 1 to 6, wherein a portion of the inner member (556) near the outflow end (508) of the frame (502) comprises teeth (566).

9. The implantable prosthetic device (500) of any previous claims, wherein the pawl (568) comprises an elongated body (574) terminating in a locking tooth (576) that engages the teeth (566) of the linear rack (564).

10. The implantable prosthetic device (500) of claim 9, wherein the tooth (576) has a shape that is complimentary to the shape of the teeth (566), such that the tooth (576) allows sliding movement of the inner member (556) in one direction relative to the pawl (568) and resists sliding movement of the inner member (566) in the opposite direction when the tooth (576) is in engagement with one of the teeth (566) of the linear rack (564).

11. The implantable prosthetic device (500) of any of claims 9 or 10, wherein the body (574) of the pawl (568) is biased inwardly such that the tooth (576) of the pawl (568) is resiliently retained in a position engaging one of the teeth (566) of the inner member (556).

12. The implantable prosthetic device (500) of claims 9 to 11, wherein the body (574) is configured as a leaf spring.

13. The implantable prosthetic device (500) of claims 9 to 12, wherein the body (574) is integrally formed with the sleeve (552).

14. The implantable prosthetic device (500) of claims 9 to 12, wherein the body (574) is separately formed and subsequently coupled to the sleeve (552).

15. The implantable prosthetic device (500) of any previous claims, wherein the second member (556) comprises a toothless portion (578) extending from a proximal edge (580) of the second member (556) to the plurality of teeth (566).
